# EUROPEAN PATENT SPECIFICATION

(11) **EP 1 824 881 B1**
(45) Date of publication and mention of the grant of the patent: **15.06.2011**
(21) Application number: 05818361.7
(22) Date of filing: 08.12.2005
(51) Int. Cl.: C07K 14/71, G01N 33/74, A61K 38/17

(54) **TGR3-LIKE PROTEIN RECEPTOR**
REZEPTOR FÜR DAS TGR3-LIKE PROTEIN
RECEPTEUR DE PROTEINES DE TYPE TGR3

(30) Priority: 08.12.2004 GB 0426960
(43) Date of publication of application: 29.08.2007
(73) Proprietor: ARES TRADING S.A., 1170 Aubonne (CH)
(72) Inventor: MICHALOVICH, David Inpharmatica Limited, London W1T 2NU (GB); MITTER, Richard James, Inpharmatica Limited, London W1T 2NU (GB); FAGAN, Richard Joseph, Inpharmatica Limited, London W1T 2NU (GB); POWER, Christine, F-01710 Thoiry (FR)
(74) Representative: Goodfellow, Hugh Robin
(86) International application number: PCT/GB2005/004728
(87) International publication number: WO 2006/061628

(56) References cited:
- DATABASE Geneseq [Online] 8 February 2001 (2001-02-08), "Human ORFX ORF290 polynucleotide sequence SEQ ID NO:579." XP002371190 retrieved from EBI accession no. GSN:AAC74735 Database accession no. AAC74735 & WO 00/58473 A (CURAGEN CORPORATION; SHIMKETS, RICHARD, A; LEACH, MARTIN) 5 October 2000 (2000-10-05)
- DATABASE Geneseq [Online] 8 February 2001 (2001-02-08), "Human ORFX ORF290 polypeptide sequence SEQ ID NO:580." XP002371191 retrieved from EBI accession no. GSN:AAB40526 Database accession no. AAB40526 & WO 00/58473 A (CURAGEN CORPORATION; SHIMKETS, RICHARD, A; LEACH, MARTIN) 5 October 2000 (2000-10-05)
- DATABASE EMBL [Online] 16 May 2001 (2001-05-16), "602732441F1 NIH_MGC_43 Homo sapiens cDNA clone IMAGE:4875922 5', mRNA sequence." XP002371192 retrieved from EBI accession no. EM_PRO:BG752983 Database accession no. BG752983
- JOVINE LUCA ET AL: "A duplicated motif controls assembly of zona pellucida domain proteins" PROCEEDINGS OF THE NATIONAL ACADEMY OF SCIENCES OF THE UNITED STATES OF AMERICA, vol. 101, no. 16, 20 April 2004 (2004-04-20), pages 5922-5927, XP002371186 ISSN: 0027-8424 cited in the application
- YONEZAWA N ET AL: "Identification of the carboxyl termini of porcine zona pellucida glycoproteins ZPB and ZPC" BIOCHEMICAL AND BIOPHYSICAL RESEARCH COMMUNICATIONS, ACADEMIC PRESS INC. ORLANDO, FL, US, vol. 307, no. 4, 8 August 2003 (2003-08-08), pages 877-882, XP004439218 ISSN: 0006-291X cited in the application

### Sequence Listing

<110> ARES TRADING S.A.
<120> TGR3-like Protein Receptor
<130> P039409WO
<150> GB 0426960.1
   <151> 2004-12-08
<160> 47
<170> SeqWin99, version 1.02
<210> 1
   <211> 879
   <212> DNA
   <213> Homo sapiens
<220>
   <221> INSP215 full nucleotide sequence
<400> 1
   atgctgggca ccgtgctgct gctggccctg ctcccaggga tcaccacctt acccagcggg 60
   ccacctgctc ccccgttccc cgcggcgccc ggcccctggc tgcgcagacc cctcttcagc 120
   ctgaagctgt ccgacacaga ggacgtcttt cctcgccgcg cggggccgct cgaggtcccg 180
   gccgacagcc gcgtgttcgt gcaggcggcc ttggcccgtc cctccccgcg ctggggcctg 240
   gccctgcacc gctgctcagt gacgccgtcc tcacgcccgg ccccggggcc cgccctggct 300
   ctgctgcgtg agggctgccc cgccgacacc tctgtcgcct tcccgccacc gccgccgccg 360
   agcccgggtg ccgcccgccc cgcgcgtttc agcttccgcc tgcgcccggt cttcaacgcc 420
   tcggtgcagt tcctgcactg ccagctgagc cgctgccgcc gcctccgggg agtccgccgg 480
   gcgcctgcgc ctctgacgcc gccgccgccg ccgccgccat cgcggtgtct gcctcaggac 540
   gaggcgtgcg ccgacactgg cagtggcagc gccgagggcc tggctgctga cggcccccac 600
   ctgcacacgc tgacgcagcc tatcgtggtc accgtgccgc ggccgccccc caggccgccc 660
   aagagtgtcc ccggccgtgc agtgcgccct gagcctcccg cgccggcccc cgcggccctg 720
   gaacccgcgc cggtggtggc gctggtgttg gcagccttcg tgctgggcgc cgcgctggcc 780
   gccgggctgg gtctcgtctg tgcgcactca gcgccccacg cccctggccc gcccgcgaga 840
   gcctcgccca gcggtcccca gcccaggagg tcccagtga 879
<210> 2
   <211> 292
   <212> PRT
   <213> Homo sapiens
<220>
   <221> INSP215 full protein sequence
<400> 2
   Met Leu Gly Thr Val Leu Leu Leu Ala Leu Leu Pro Gly Ile Thr Thr
   1 5 10 15
   Leu Pro Ser Gly Pro Pro Ala Pro Pro Phe Pro Ala Ala Pro Gly Pro
   20 25 30
   Trp Leu Arg Arg Pro Leu Phe Ser Leu Lys Leu Ser Asp Thr Glu Asp
   35 40 45
   Val Phe Pro Arg Arg Ala Gly Pro Leu Glu Val Pro Ala Asp Ser Arg
       50                  55                  60
   Val Phe Val Gln Ala Ala Leu Ala Arg Pro Ser Pro Arg Trp Gly Leu
   65                  70                  75                  80
   Ala Leu His Arg Cys Ser Val Thr Pro Ser Ser Arg Pro Ala Pro Gly
                   85                  90                  95
   Pro Ala Leu Ala Leu Leu Arg Glu Gly Cys Pro Ala Asp Thr Ser Val
               100                 105                 110
   Ala Phe Pro Pro Pro Pro Pro Pro Ser Pro Gly Ala Ala Arg Pro Ala
           115                 120                 125
   Arg Phe Ser Phe Arg Leu Arg Pro Val Phe Asn Ala Ser Val Gln Phe
       130                 135                 140
   Leu His Cys Gln Leu Ser Arg Cys Arg Arg Leu Arg Gly Val Arg Arg
   145                 150                 155                 160
   Ala Pro Ala Pro Leu Thr Pro Pro Pro Pro Pro Pro Pro Ser Arg Cys
                   165                 170                 175
   Leu Pro Gln Asp Glu Ala Cys Ala Asp Thr Gly Ser Gly Ser Ala Glu
               180                 185                 190
   Gly Leu Ala Ala Asp Gly Pro His Leu His Thr Leu Thr Gln Pro Ile
           195                 200                 205
   Val Val Thr Val Pro Arg Pro Pro Pro Arg Pro Pro Lys Ser Val Pro
       210                 215                 220
   Gly Arg Ala Val Arg Pro Glu Pro Pro Ala Pro Ala Pro Ala Ala Leu
   225                 230                 235                 240
   Glu Pro Ala Pro Val Val Ala Leu Val Leu Ala Ala Phe Val Leu Gly
                   245                 250                 255
   Ala Ala Leu Ala Ala Gly Leu Gly Leu Val Cys Ala His Ser Ala Pro
               260                 265                 270
   His Ala Pro Gly Pro Pro Ala Arg Ala Ser Pro Ser Gly Pro Gln Pro
           275                 280                 285
   Arg Arg Ser Gln
       290
<210> 3
   <211> 831
   <212> DNA
   <213> Homo sapiens
<220>
   <221> INSP215 mature nucleotide sequence
<400> 3
   ttacccagcg ggccacctgc tcccccgttc cccgcggcgc ccggcccctg gctgcgcaga    60
   cccctcttca gcctgaagct gtccgacaca gaggacgtct ttcctcgccg cgcggggccg   120
   ctcgaggtcc cggccgacag ccgcgtgttc gtgcaggcgg ccttggcccg tccctccccg   180
   cgctggggcc tggccctgca ccgctgctca gtgacgccgt cctcacgccc ggccccgggg   240
   cccgccctgg ctctgctgcg tgagggctgc cccgccgaca cctctgtcgc cttcccgcca   300
   ccgccgccgc cgagcccggg tgccgcccgc cccgcgcgtt tcagcttccg cctgcgcccg   360
   gtcttcaacg cctcggtgca gttcctgcac tgccagctga gccgctgccg ccgcctccgg   420
   ggagtccgcc gggcgcctgc gcctctgacg ccgccgccgc cgccgccgcc atcgcggtgt   480
   ctgcctcagg acgaggcgtg cgccgacact ggcagtggca gcgccgaggg cctggctgct   540
   gacggccccc acctgcacac gctgacgcag cctatcgtgg tcaccgtgcc gcggccgccc   600
   cccaggccgc ccaagagtgt ccccggccgt gcagtgcgcc ctgagcctcc cgcgccggcc   660
   cccgcggccc tggaacccgc gccggtggtg gcgctggtgt tggcagcctt cgtgctgggc   720
   gccgcgctgg ccgccgggct gggtctcgtc tgtgcgcact cagcgcccca cgcccctggc   780
   ccgcccgcga gagcctcgcc cagcggtccc cagcccagga ggtcccagtg a            831
<210> 4
   <211> 276
   <212> PRT
   <213> Homo sapiens
<220>
   <221> INSP215 mature protein sequence
<400> 4
   Leu Pro Ser Gly Pro Pro Ala Pro Pro Phe Pro Ala Ala Pro Gly Pro
   1               5                   10                  15
   Trp Leu Arg Arg Pro Leu Phe Ser Leu Lys Leu Ser Asp Thr Glu Asp
               20                  25                  30
   Val Phe Pro Arg Arg Ala Gly Pro Leu Glu Val Pro Ala Asp Ser Arg
           35                  40                  45
   Val Phe Val Gln Ala Ala Leu Ala Arg Pro Ser Pro Arg Trp Gly Leu
       50                  55                  60
   Ala Leu His Arg Cys Ser Val Thr Pro Ser Ser Arg Pro Ala Pro Gly
   65                  70                  75                  80
   Pro Ala Leu Ala Leu Leu Arg Glu Gly Cys Pro Ala Asp Thr Ser Val
                   85                  90                  95
   Ala Phe Pro Pro Pro Pro Pro Pro Ser Pro Gly Ala Ala Arg Pro Ala
               100                 105                 110
   Arg Phe Ser Phe Arg Leu Arg Pro Val Phe Asn Ala Ser Val Gln Phe
           115                 120                 125
   Leu His Cys Gln Leu Ser Arg Cys Arg Arg Leu Arg Gly Val Arg Arg
       130                 135                 140
   Ala Pro Ala Pro Leu Thr Pro Pro Pro Pro Pro Pro Pro Ser Arg Cys
   145                 150                 155                 160
   Leu Pro Gln Asp Glu Ala Cys Ala Asp Thr Gly Ser Gly Ser Ala Glu
                   165                 170                 175
   Gly Leu Ala Ala Asp Gly Pro His Leu His Thr Leu Thr Gln Pro Ile
               180                 185                 190
   Val Val Thr Val Pro Arg Pro Pro Pro Arg Pro Pro Lys Ser Val Pro
           195                 200                 205
   Gly Arg Ala Val Arg Pro Glu Pro Pro Ala Pro Ala Pro Ala Ala Leu
       210                 215                 220
   Glu Pro Ala Pro Val Val Ala Leu Val Leu Ala Ala Phe Val Leu Gly
   225                 230                 235                 240
   Ala Ala Leu Ala Ala Gly Leu Gly Leu Val Cys Ala His Ser Ala Pro
                   245                 250                 255
   His Ala Pro Gly Pro Pro Ala Arg Ala Ser Pro Ser Gly Pro Gln Pro
               260                 265                 270
   Arg Arg Ser Gln
           275
<210> 5
   <211> 732
   <212> DNA
   <213> Homo sapiens
<220>
   <221> INSP215 full extracellular region nucleotide sequence
<400> 5
   atgctgggca ccgtgctgct gctggccctg ctcccaggga tcaccacctt acccagcggg    60
   ccacctgctc ccccgttccc cgcggcgccc ggcccctggc tgcgcagacc cctcttcagc   120
   ctgaagctgt ccgacacaga ggacgtcttt cctcgccgcg cggggccgct cgaggtcccg   180
   gccgacagcc gcgtgttcgt gcaggcggcc ttggcccgtc cctccccgcg ctggggcctg   240
   gccctgcacc gctgctcagt gacgccgtcc tcacgcccgg ccccggggcc cgccctggct   300
   ctgctgcgtg agggctgccc cgccgacacc tctgtcgcct tcccgccacc gccgccgccg   360
   agcccgggtg ccgcccgccc cgcgcgtttc agcttccgcc tgcgcccggt cttcaacgcc   420
   tcggtgcagt tcctgcactg ccagctgagc cgctgccgcc gcctccgggg agtccgccgg   480
   gcgcctgcgc ctctgacgcc gccgccgccg ccgccgccat cgcggtgtct gcctcaggac   540
   gaggcgtgcg ccgacactgg cagtggcagc gccgagggcc tggctgctga cggcccccac   600
   ctgcacacgc tgacgcagcc tatcgtggtc accgtgccgc ggccgccccc caggccgccc   660
   aagagtgtcc ccggccgtgc agtgcgccct gagcctcccg cgccggcccc cgcggccctg   720
   gaacccgcgc cg                                                       732
<210> 6
   <211> 244
   <212> PRT
   <213> Homo sapiens
<220>
   <221> INSP215 full extracellular region protein sequence
<400> 6
   Met Leu Gly Thr Val Leu Leu Leu Ala Leu Leu Pro Gly Ile Thr Thr
   1               5                   10                  15
   Leu Pro Ser Gly Pro Pro Ala Pro Pro Phe Pro Ala Ala Pro Gly Pro
               20                  25                  30
   Trp Leu Arg Arg Pro Leu Phe Ser Leu Lys Leu Ser Asp Thr Glu Asp
           35                  40                  45
   Val Phe Pro Arg Arg Ala Gly Pro Leu Glu Val Pro Ala Asp Ser Arg
       50                  55                  60
   Val Phe Val Gln Ala Ala Leu Ala Arg Pro Ser Pro Arg Trp Gly Leu
   65 70 75 80
   Ala Leu His Arg Cys Ser Val Thr Pro Ser Ser Arg Pro Ala Pro Gly
   85 90 95
   Pro Ala Leu Ala Leu Leu Arg Glu Gly Cys Pro Ala Asp Thr Ser Val
   100 105 110
   Ala Phe Pro Pro Pro Pro Pro Pro Ser Pro Gly Ala Ala Arg Pro Ala
   115 120 125
   Arg Phe Ser Phe Arg Leu Arg Pro Val Phe Asn Ala Ser Val Gln Phe
   130 135 140
   Leu His Cys Gln Leu Ser Arg Cys Arg Arg Leu Arg Gly Val Arg Arg
   145 150 155 160
   Ala Pro Ala Pro Leu Thr Pro Pro Pro Pro Pro Pro Pro Ser Arg Cys
   165 170 175
   Leu Pro Gln Asp Glu Ala Cys Ala Asp Thr Gly Ser Gly Ser Ala Glu
   180 185 190
   Gly Leu Ala Ala Asp Gly Pro His Leu His Thr Leu Thr Gln Pro Ile
   195 200 205
   Val Val Thr Val Pro Arg Pro Pro Pro Arg Pro Pro Lys Ser Val Pro
   210 215 220
   Gly Arg Ala Val Arg Pro Glu Pro Pro Ala Pro Ala Pro Ala Ala Leu
   225 230 235 240
   Glu Pro Ala Pro
<210> 7
   <211> 684
   <212> DNA
   <213> Homo sapiens
<220>
   <221> INSP215 mature extracellular region nucleotide sequence
<400> 7
   ttacccagcg ggccacctgc tcccccgttc cccgcggcgc ccggcccctg gctgcgcaga    60
   cccctcttca gcctgaagct gtccgacaca gaggacgtct ttcctcgccg cgcggggccg   120
   ctcgaggtcc cggccgacag ccgcgtgttc gtgcaggcgg ccttggcccg tccctccccg   180
   cgctggggcc tggccctgca ccgctgctca gtgacgccgt cctcacgccc ggccccgggg   240
   cccgccctgg ctctgctgcg tgagggctgc cccgccgaca cctctgtcgc cttcccgcca   300
   ccgccgccgc cgagcccggg tgccgcccgc cccgcgcgtt tcagcttccg cctgcgcccg   360
   gtcttcaacg cctcggtgca gttcctgcac tgccagctga gccgctgccg ccgcctccgg   420
   ggagtccgcc gggcgcctgc gcctctgacg ccgccgccgc cgccgccgcc atcgcggtgt   480
   ctgcctcagg acgaggcgtg cgccgacact ggcagtggca gcgccgaggg cctggctgct   540
   gacggccccc acctgcacac gctgacgcag cctatcgtgg tcaccgtgcc gcggccgccc   600
   cccaggccgc ccaagagtgt ccccggccgt gcagtgcgcc ctgagcctcc cgcgccggcc   660
   cccgcggccc tggaacccgc gccg                                          684
<210> 8
   <211> 228
   <212> PRT
   <213> Homo sapiens
<220>
   <221> INSP215 mature extracellular region protein sequence
<400> 8
   Leu Pro Ser Gly Pro Pro Ala Pro Pro Phe Pro Ala Ala Pro Gly Pro
   1               5                   10                  15
   Trp Leu Arg Arg Pro Leu Phe Ser Leu Lys Leu Ser Asp Thr Glu Asp
               20                  25                  30
   Val Phe Pro Arg Arg Ala Gly Pro Leu Glu Val Pro Ala Asp Ser Arg
           35                  40                  45
   Val Phe Val Gln Ala Ala Leu Ala Arg Pro Ser Pro Arg Trp Gly Leu
       50                  55                  60
   Ala Leu His Arg Cys Ser Val Thr Pro Ser Ser Arg Pro Ala Pro Gly
   65                  70                  75                  80
   Pro Ala Leu Ala Leu Leu Arg Glu Gly Cys Pro Ala Asp Thr Ser Val
                   85                  90                  95
   Ala Phe Pro Pro Pro Pro Pro Pro Ser Pro Gly Ala Ala Arg Pro Ala
               100                 105                 110
   Arg Phe Ser Phe Arg Leu Arg Pro Val Phe Asn Ala Ser Val Gln Phe
           115                 120                 125
   Leu His Cys Gln Leu Ser Arg Cys Arg Arg Leu Arg Gly Val Arg Arg
       130                 135                 140
   Ala Pro Ala Pro Leu Thr Pro Pro Pro Pro Pro Pro Pro Ser Arg Cys
   145                 150                 155                 160
   Leu Pro Gln Asp Glu Ala Cys Ala Asp Thr Gly Ser Gly Ser Ala Glu
                   165                 170                 175
   Gly Leu Ala Ala Asp Gly Pro His Leu His Thr Leu Thr Gln Pro Ile
               180                 185                 190
   Val Val Thr Val Pro Arg Pro Pro Pro Arg Pro Pro Lys Ser Val Pro
           195                 200                 205
   Gly Arg Ala Val Arg Pro Glu Pro Pro Ala Pro Ala Pro Ala Ala Leu
       210                 215                 220
   Glu Pro Ala Pro
   225
<210> 9
   <211> 435
   <212> DNA
   <213> Homo sapiens
<220>
   <221> INSP215 ZP/TGR3 subdomain nucleotide sequence
<400> 9
   ttcagcctga agctgtccga cacagaggac gtctttcctc gccgcgcggg gccgctcgag    60
   gtcccggccg acagccgcgt gttcgtgcag gcggccttgg cccgtccctc cccgcgctgg   120
   ggcctggccc tgcaccgctg ctcagtgacg ccgtcctcac gcccggcccc ggggcccgcc   180
   ctggctctgc tgcgtgaggg ctgccccgcc gacacctctg tcgccttccc gccaccgccg   240
   ccgccgagcc cgggtgccgc ccgccccgcg cgtttcagct tccgcctgcg cccggtcttc   300
   aacgcctcgg tgcagttcct gcactgccag ctgagccgct gccgccgcct ccggggagtc   360
   cgccgggcgc ctgcgcctct gacgccgccg ccgccgccgc cgccatcgcg gtgtctgcct   420
   caggacgagg cgtgc                                                    435
<210> 10
   <211> 145
   <212> PRT
   <213> Homo sapiens
<220>
   <221> INSP215 ZP/TGR3 subdomain protein sequence
<400> 10
   Phe Ser Leu Lys Leu Ser Asp Thr Glu Asp Val Phe Pro Arg Arg Ala
   1               5                   10                  15
   Gly Pro Leu Glu Val Pro Ala Asp Ser Arg Val Phe Val Gln Ala Ala
               20                  25                  30
   Leu Ala Arg Pro Ser Pro Arg Trp Gly Leu Ala Leu His Arg Cys Ser
           35                  40                  45
   Val Thr Pro Ser Ser Arg Pro Ala Pro Gly Pro Ala Leu Ala Leu Leu
       50                  55                  60
   Arg Glu Gly Cys Pro Ala Asp Thr Ser Val Ala Phe Pro Pro Pro Pro
   65                  70                  75                  80
   Pro Pro Ser Pro Gly Ala Ala Arg Pro Ala Arg Phe Ser Phe Arg Leu
                   85                  90                  95
   Arg Pro Val Phe Asn Ala Ser Val Gln Phe Leu His Cys Gln Leu Ser
               100                 105                 110
   Arg Cys Arg Arg Leu Arg Gly Val Arg Arg Ala Pro Ala Pro Leu Thr
           115                 120                 125
   Pro Pro Pro Pro Pro Pro Pro Ser Arg Cys Leu Pro Gln Asp Glu Ala
       130                 135                 140
   Cys
   145
<210> 11
   <211> 651
   <212> DNA
   <213> Homo sapiens
<220>
   <221> INSP215 exons 1-4 nucleotide sequence
<400> 11
   atgctgggca ccgtgctgct gctggccctg ctcccaggga tcaccacctt acccagcggg    60
   ccacctgctc ccccgttccc cgcggcgccc ggcccctggc tgcgcagacc cctcttcagc   120
   ctgaagctgt ccgacacaga ggacgtcttt cctcgccgcg cggggccgct cgaggtcccg   180
   gccgacagcc gcgtgttcgt gcaggcggcc ttggcccgtc cctccccgcg ctggggcctg   240
   gccctgcacc gctgctcagt gacgccgtcc tcacgcccgg ccccggggcc cgccctggct   300
   ctgctgcgtg agggctgccc cgccgacacc tctgtcgcct tcccgccacc gccgccgccg   360
   agcccgggtg ccgcccgccc cgcgcgtttc agcttccgcc tgcgcccggt cttcaacgcc   420
   tcggtgcagt tcctgcactg ccagctgagc cgctgccgcc gcctccgggg agtccgccgg   480
   gcgcctgcgc ctctgacgcc gccgccgccg ccgccgccat cgcggtgtct gcctcaggac   540
   gaggcgtgcg ccgacactgg cagtggcagc gccgagggcc tggctgctga cggcccccac   600
   ctgcacacgc tgacgcagcc tatcgtggtc accgtgccgc ggccgccccc c            651
<210> 12
   <211> 217
   <212> PRT
   <213> Homo sapiens
<220>
   <221> INSP215 exons 1-4 protein sequence
<400> 12
   Met Leu Gly Thr Val Leu Leu Leu Ala Leu Leu Pro Gly Ile Thr Thr
   1               5                   10                  15
   Leu Pro Ser Gly Pro Pro Ala Pro Pro Phe Pro Ala Ala Pro Gly Pro
               20                  25                  30
   Trp Leu Arg Arg Pro Leu Phe Ser Leu Lys Leu Ser Asp Thr Glu Asp
           35                  40                  45
   Val Phe Pro Arg Arg Ala Gly Pro Leu Glu Val Pro Ala Asp Ser Arg
       50                  55                  60
   Val Phe Val Gln Ala Ala Leu Ala Arg Pro Ser Pro Arg Trp Gly Leu
   65                  70                  75                  80
   Ala Leu His Arg Cys Ser Val Thr Pro Ser Ser Arg Pro Ala Pro Gly
                   85                  90                  95
   Pro Ala Leu Ala Leu Leu Arg Glu Gly Cys Pro Ala Asp Thr Ser Val
               100                 105                 110
   Ala Phe Pro Pro Pro Pro Pro Pro Ser Pro Gly Ala Ala Arg Pro Ala
           115                 120                 125
   Arg Phe Ser Phe Arg Leu Arg Pro Val Phe Asn Ala Ser Val Gln Phe
       130                 135                 140
   Leu His Cys Gln Leu Ser Arg Cys Arg Arg Leu Arg Gly Val Arg Arg
   145                 150                 155                 160
   Ala Pro Ala Pro Leu Thr Pro Pro Pro Pro Pro Pro Pro Ser Arg Cys
                   165                 170                 175
   Leu Pro Gln Asp Glu Ala Cys Ala Asp Thr Gly Ser Gly Ser Ala Glu
               180                 185                 190
   Gly Leu Ala Ala Asp Gly Pro His Leu His Thr Leu Thr Gln Pro Ile
           195                 200                 205
   Val Val Thr Val Pro Arg Pro Pro Pro
       210                 215
<210> 13
   <211> 603
   <212> DNA
   <213> Homo sapiens
<220>
   <221> INSP215 exons 1-4 mature nucleotide sequence
<400> 13
   ttacccagcg ggccacctgc tcccccgttc cccgcggcgc ccggcccctg gctgcgcaga    60
   cccctcttca gcctgaagct gtccgacaca gaggacgtct ttcctcgccg cgcggggccg   120
   ctcgaggtcc cggccgacag ccgcgtgttc gtgcaggcgg ccttggcccg tccctccccg   180
   cgctggggcc tggccctgca ccgctgctca gtgacgccgt cctcacgccc ggccccgggg   240
   cccgccctgg ctctgctgcg tgagggctgc cccgccgaca cctctgtcgc cttcccgcca   300
   ccgccgccgc cgagcccggg tgccgcccgc cccgcgcgtt tcagcttccg cctgcgcccg   360
   gtcttcaacg cctcggtgca gttcctgcac tgccagctga gccgctgccg ccgcctccgg   420
   ggagtccgcc gggcgcctgc gcctctgacg ccgccgccgc cgccgccgcc atcgcggtgt   480
   ctgcctcagg acgaggcgtg cgccgacact ggcagtggca gcgccgaggg cctggctgct   540
   gacggccccc acctgcacac gctgacgcag cctatcgtgg tcaccgtgcc gcggccgccc   600
   ccc                                                                 603
<210> 14
   <211> 201
   <212> PRT
   <213> Homo sapiens
<220>
   <221> INSP215 exons 1-4 mature protein sequence
<400> 14
   Leu Pro Ser Gly Pro Pro Ala Pro Pro Phe Pro Ala Ala Pro Gly Pro
   1               5                   10                  15
   Trp Leu Arg Arg Pro Leu Phe Ser Leu Lys Leu Ser Asp Thr Glu Asp
               20                  25                  30
   Val Phe Pro Arg Arg Ala Gly Pro Leu Glu Val Pro Ala Asp Ser Arg
           35                  40                  45
   Val Phe Val Gln Ala Ala Leu Ala Arg Pro Ser Pro Arg Trp Gly Leu
       50                  55                  60
   Ala Leu His Arg Cys Ser Val Thr Pro Ser Ser Arg Pro Ala Pro Gly
   65                  70                  75                  80
   Pro Ala Leu Ala Leu Leu Arg Glu Gly Cys Pro Ala Asp Thr Ser Val
                   85                  90                  95
   Ala Phe Pro Pro Pro Pro Pro Pro Ser Pro Gly Ala Ala Arg Pro Ala
               100                 105                 110
   Arg Phe Ser Phe Arg Leu Arg Pro Val Phe Asn Ala Ser Val Gln Phe
           115                 120                 125
   Leu His Cys Gln Leu Ser Arg Cys Arg Arg Leu Arg Gly Val Arg Arg
       130                 135                 140
   Ala Pro Ala Pro Leu Thr Pro Pro Pro Pro Pro Pro Pro Ser Arg Cys
   145                 150                 155                 160
   Leu Pro Gln Asp Glu Ala Cys Ala Asp Thr Gly Ser Gly Ser Ala Glu
                   165                 170                 175
   Gly Leu Ala Ala Asp Gly Pro His Leu His Thr Leu Thr Gln Pro Ile
               180                 185                 190
   Val Val Thr Val Pro Arg Pro Pro Pro
           195                 200
<210> 15
   <211> 13
   <212> PRT
   <213> Artificial Sequence
<220>
   <223> Linker sequence
<220>
   <221> Fusion protein linker sequence
<400> 15
   Glu Phe Gly Ala Gly Leu Val Leu Gly Gly Gln Phe Met
   1               5                   10
<210> 16
   <211> 1163
   <212> DNA
   <213> Predicted sequence
<220>
   <221> INSP215 predicted cDNA sequence
<400> 16
   ccttgccaag gacagggccg cctgcccacg tgcgggacct gggtgcagcc cagctggtgt    60
   ctgctctggc cccaaacact ccgacctcag ctggtccagc atgctgggca ccgtgctgct   120
   gctggccctg ctcccaggga tcaccacctt acccagcggg ccacctgctc ccccgttccc   180
   cgcggcgccc ggcccctggc tgcgcagacc cctcttcagc ctgaagctgt ccgacacaga   240
   ggacgtcttt cctcgccgcg cggggccgct cgaggtcccg gccgacagcc gcgtgttcgt   300
   gcaggcggcc ttggcccgtc cctccccgcg ctggggcctg gccctgcacc gctgctcagt   360
   gacgccgtcc tcacgcccgg ccccggggcc cgccctggct ctgctgcgtg agggctgccc   420
   cgccgacacc tctgtcgcct tcccgccacc gccgccgccg agcccgggtg ccgcccgccc   480
   cgcgcgtttc agcttccgcc tgcgcccggt cttcaacgcc tcggtgcagt tcctgcactg   540
   ccagctgagc cgctgccgcc gcctccgggg agtccgccgg gcgcctgcgc ctctgacgcc   600
   gccgccgccg ccgccgccat cgcggtgtct gcctcaggac gaggcgtgcg ccgacactgg   660
   cagtggcagc gccgagggcc tggctgctga cggcccccac ctgcacacgc tgacgcagcc   720
   tatcgtggtc accgtgccgc ggccgccccc caggccgccc aagagtgtcc ccggccgtgc   780
   agtgcgccct gagcctcccg cgccggcccc cgcggccctg gaacccgcgc cggtggtggc   840
   gctggtgttg gcagccttcg tgctgggcgc cgcgctggcc gccgggctgg gtctcgtctg   900
   tgcgcactca gcgccccacg cccctggccc gcccgcgaga gcctcgccca gcggtcccca   960
   gcccaggagg tcccagtgag gaagggatgg tgcgccccca acatggtccg gagatacacc  1020
   cagctaccaa ttcgggacca ggaccaacag gaccggaccc gcctccctgg acctcggacc  1080
   tgatgaggcc acgacccctg cgcttctctc ctccccctgt ccctcccacc tgtgctcaaa  1140
   ataaacctct ggactgaccg gct                                          1163
<210> 17
   <211> 993
   <212> DNA
   <213> Artificial Sequence
<220>
   <223> Clone insert
<220>
   <221> cDNA insert in Image clone 4875922
<400> 17
   ggcacgaggc cgccagctcc cccgttcccc gcggcgcccg gcccctggct gcgcagaccc    60
   ctcttcagcc tgaagctgtc cgacacagag gacgtctttc ctcgccgcgc ggggccgctc   120
   gaggtcccgg ccgacagccg cgtgttcgtg caggcggcct tggcccgtcc ctccccgcgc   180
   tggggcctgg ccctgcaccg ctgctcagtg acgccgtcct cacgcccggc cccggggccc   240
   gccctggctc tgctgcgtga gggctgcccc gccgacacct ctgtcgcctt cccgccaccg   300
   ccgccgccga gcccgggtgc cgcccgcccc gcgcgtttca gcttccgcct gcgcccggtc   360
   ttcaacgcct cggtgcagtt cctgcactgc cagctgagcc gctgccgccg cctccgggga   420
   gtccgccggg cgcctgcgcc tctgacgccg ccgccgccgc cgccgccatc gcggtgtctg   480
   cctcaggacg aggcgtgcgc cgacactggc agtggcagcg ccgagggcct ggctgctgac   540
   ggcccccacc tgcacacgct gacgcagcct atcgtggtca ccgtgccgcg gccgcccccc   600
   aggccgccca agagtgtccc cggccgtgca gtgcgccctg agcctcccgc gccggccccc   660
   gcggccctgg aacccgcgcc ggtggtggcg ctggtgttgg cagccttcgt gctgggcgcc   720
   gcgctggccg ccgggctggg tctcgtctgt gcgcactcag cgccccacgc ccctggcccg   780
   cccgcgagag cctcgcccag cggtccccag cccaggaggt cccagtgagg aagggatggt   840
   gcgcccccaa catggtccgg agatacaccc agctaccaat tcgggaccag gaccaacagg   900
   accggacccg cctccctgga cctcggacct gatgaggcca cgacccctgc gcttctctcc   960
   tccccctgtc cctcccacct gtgctcaaaa taa                                993
<210> 18
   <211> 894
   <212> DNA
   <213> Artificial Sequence
<220>
   <223> Fusion protein coding sequence
<220>
   <221> INSP215 full length with 6HIS tag nucleotide sequence
<400> 18
   atgctgggca ccgtgctgct gctggccctg ctcccaggga tcaccacctt acccagcggg    60
   ccacctgctc ccccgttccc cgcggcgccc ggcccctggc tgcgcagacc cctcttcagc   120
   ctgaagctgt ccgacacaga ggacgtcttt cctcgccgcg cggggccgct cgaggtcccg   180
   gccgacagcc gcgtgttcgt gcaggcggcc ttggcccgtc cctccccgcg ctggggcctg   240
   gccctgcacc gctgctcagt gacgccgtcc tcacgcccgg ccccggggcc cgccctggct   300
   ctgctgcgtg agggctgccc cgccgacacc tctgtcgcct tcccgccacc gccgccgccg   360
   agcccgggtg ccgcccgccc cgcgcgtttc agcttccgcc tgcgcccggt cttcaacgcc   420
   tcggtgcagt tcctgcactg ccagctgagc cgctgccgcc gcctccgggg agtccgccgg   480
   gcgcctgcgc ctctgacgcc gccgccgccg ccgccgccat cgcggtgtct gcctcaggac   540
   gaggcgtgcg ccgacactgg cagtggcagc gccgagggcc tggctgctga cggcccccac   600
   ctgcacacgc tgacgcagcc tatcgtggtc accgtgccgc ggccgccccc caggccgccc   660
   aagagtgtcc ccggccgtgc agtgcgccct gagcctcccg cgccggcccc cgcggccctg   720
   gaacccgcgc cggtggtggc gctggtgttg gcagccttcg tgctgggcgc cgcgctggcc   780
   gccgggctgg gtctcgtctg tgcgcactca gcgccccacg cccctggccc gcccgcgaga   840
   gcctcgccca gcggtcccca gcccaggagg tcccagcacc atcaccatca ccat         894
<210> 19
   <211> 298
   <212> PRT
   <213> Fusion protein
<220>
   <221> INSP215 full length with 6HIS tag protein sequence
<400> 19
   Met Leu Gly Thr Val Leu Leu Leu Ala Leu Leu Pro Gly Ile Thr Thr
   1               5                   10                  15
   Leu Pro Ser Gly Pro Pro Ala Pro Pro Phe Pro Ala Ala Pro Gly Pro
               20                  25                  30
   Trp Leu Arg Arg Pro Leu Phe Ser Leu Lys Leu Ser Asp Thr Glu Asp
           35                  40                  45
   Val Phe Pro Arg Arg Ala Gly Pro Leu Glu Val Pro Ala Asp Ser Arg
       50                  55                  60
   Val Phe Val Gln Ala Ala Leu Ala Arg Pro Ser Pro Arg Trp Gly Leu
   65                  70                  75                  80
   Ala Leu His Arg Cys Ser Val Thr Pro Ser Ser Arg Pro Ala Pro Gly
                   85                  90                  95
   Pro Ala Leu Ala Leu Leu Arg Glu Gly Cys Pro Ala Asp Thr Ser Val
               100                 105                 110
   Ala Phe Pro Pro Pro Pro Pro Pro Ser Pro Gly Ala Ala Arg Pro Ala
           115                 120                 125
   Arg Phe Ser Phe Arg Leu Arg Pro Val Phe Asn Ala Ser Val Gln Phe
       130                 135                 140
   Leu His Cys Gln Leu Ser Arg Cys Arg Arg Leu Arg Gly Val Arg Arg
   145                 150                 155                 160
   Ala Pro Ala Pro Leu Thr Pro Pro Pro Pro Pro Pro Pro Ser Arg Cys
                   165                 170                 175
   Leu Pro Gln Asp Glu Ala Cys Ala Asp Thr Gly Ser Gly Ser Ala Glu
               180                 185                 190
   Gly Leu Ala Ala Asp Gly Pro His Leu His Thr Leu Thr Gln Pro Ile
           195                 200                 205
   Val Val Thr Val Pro Arg Pro Pro Pro Arg Pro Pro Lys Ser Val Pro
       210                 215                 220
   Gly Arg Ala Val Arg Pro Glu Pro Pro Ala Pro Ala Pro Ala Ala Leu
   225                 230                 235                 240
   Glu Pro Ala Pro Val Val Ala Leu Val Leu Ala Ala Phe Val Leu Gly
                   245                 250                 255
   Ala Ala Leu Ala Ala Gly Leu Gly Leu Val Cys Ala His Ser Ala Pro
               260                 265                 270
   His Ala Pro Gly Pro Pro Ala Arg Ala Ser Pro Ser Gly Pro Gln Pro
           275                 280                 285
   Arg Arg Ser Gln His His His His His His
       290                 295
<210> 20
   <211> 720
   <212> DNA
   <213> Fusion protein coding sequence
<220>
   <221> INSP215 extracellular nucleotide sequence with 6HIS tag
<400> 20
   atgctgggca ccgtgctgct gctggccctg ctcccaggga tcaccacctt acccagcggg    60
   ccacctgctc ccccgttccc cgcggcgccc ggcccctggc tgcgcagacc cctcttcagc   120
   ctgaagctgt ccgacacaga ggacgtcttt cctcgccgcg cggggccgct cgaggtcccg   180
   gccgacagcc gcgtgttcgt gcaggcggcc ttggcccgtc cctccccgcg ctggggcctg   240
   gccctgcacc gctgctcagt gacgccgtcc tcacgcccgg ccccggggcc cgccctggct   300
   ctgctgcgtg agggctgccc cgccgacacc tctgtcgcct tcccgccacc gccgccgccg   360
   agcccgggtg ccgcccgccc cgcgcgtttc agcttccgcc tgcgcccggt cttcaacgcc   420
   tcggtgcagt tcctgcactg ccagctgagc cgctgccgcc gcctccgggg agtccgccgg   480
   gcgcctgcgc ctctgacgcc gccgccgccg ccgccgccat cgcggtgtct gcctcaggac   540
   gaggcgtgcg ccgacactgg cagtggcagc gccgagggcc tggctgctga cggcccccac   600
   ctgcacacgc tgacgcagcc tatcgtggtc accgtgccgc ggccgccccc caggccgccc   660
   aagagtgtcc ccggccgtgc agtgcgccct gagcctcccg cgcaccatca ccatcaccat   720
<210> 21
   <211> 240
   <212> PRT
   <213> Fusion protein
<220>
   <221> INSP215 extracellular protein sequence with 6HIS tag
<400> 21
   Met Leu Gly Thr Val Leu Leu Leu Ala Leu Leu Pro Gly Ile Thr Thr
   1               5                   10                  15
   Leu Pro Ser Gly Pro Pro Ala Pro Pro Phe Pro Ala Ala Pro Gly Pro
               20                  25                  30
   Trp Leu Arg Arg Pro Leu Phe Ser Leu Lys Leu Ser Asp Thr Glu Asp
           35                  40                  45
   Val Phe Pro Arg Arg Ala Gly Pro Leu Glu Val Pro Ala Asp Ser Arg
       50                  55                  60
   Val Phe Val Gln Ala Ala Leu Ala Arg Pro Ser Pro Arg Trp Gly Leu
   65                  70                  75                  80
   Ala Leu His Arg Cys Ser Val Thr Pro Ser Ser Arg Pro Ala Pro Gly
                   85                  90                  95
   Pro Ala Leu Ala Leu Leu Arg Glu Gly Cys Pro Ala Asp Thr Ser Val
               100                 105                 110
   Ala Phe Pro Pro Pro Pro Pro Pro Ser Pro Gly Ala Ala Arg Pro Ala
           115                 120                 125
   Arg Phe Ser Phe Arg Leu Arg Pro Val Phe Asn Ala Ser Val Gln Phe
       130                 135                 140
   Leu His Cys Gln Leu Ser Arg Cys Arg Arg Leu Arg Gly Val Arg Arg
   145                 150                 155                 160
   Ala Pro Ala Pro Leu Thr Pro Pro Pro Pro Pro Pro Pro Ser Arg Cys
                   165                 170                 175
   Leu Pro Gln Asp Glu Ala Cys Ala Asp Thr Gly Ser Gly Ser Ala Glu
               180                 185                 190
   Gly Leu Ala Ala Asp Gly Pro His Leu His Thr Leu Thr Gln Pro Ile
           195                 200                 205
   Val Val Thr Val Pro Arg Pro Pro Pro Arg Pro Pro Lys Ser Val Pro
       210                 215                 220
   Gly Arg Ala Val Arg Pro Glu Pro Pro Ala His His His His His His
   225                 230                 235                 240
<210> 22
   <211> 19
   <212> DNA
   <213> Artificial Sequence
<220>
   <223> Oligonucleotide PCR Primer
<220>
   <221> INSP215-F1
<400> 22
   cccaaacact ccgacctca 19
<210> 23
   <211> 19
   <212> DNA
   <213> Artificial Sequence
<220>
   <223> Oligonucleotide PCR Primer
<220>
   <221> INSP215-R1
<400> 23
   ttcctcactg ggacctcct 19
<210> 24
   <211> 21
   <212> DNA
   <213> Artificial Sequence
<220>
   <223> Oligonucleotide PCR Primer
<220>
   <221> INSP215-F1nest
<400> 24
   atgctgggca ccgtgctgct g 21
<210> 25
   <211> 20
   <212> DNA
   <213> Artificial Sequence
<220>
   <223> Oligonucleotide PCR Primer
<220>
   <221> INSP215-R1nest
<400> 25
   ctgggacctc ctgggctggg 20
<210> 26
   <211> 20
   <212> DNA
   <213> Artificial Sequence
<220>
   <223> Oligonucleotide PCR Primer
<220>
   <221> INSP215-F2
<400> 26
   ccccaaacac tccgacctca 20
<210> 27
   <211> 20
   <212> DNA
   <213> Artificial Sequence
<220>
   <223> Oligonucleotide PCR Primer
<220>
   <221> INSP215-R2
<400> 27
   agcacgaagg ctgccaacac 20
<210> 28
   <211> 18
   <212> DNA
   <213> Artificial Sequence
<220>
   <223> Oligonucleotide PCR Primer
<220>
   <221> INSP215-F3
<400> 28
   atgctgggca ccgtgctg 18
<210> 29
   <211> 18
   <212> DNA
   <213> Artificial Sequence
<220>
   <223> Oligonucleotide PCR Primer
<220>
   <221> INSP215-R3
<400> 29
   cggcgcgggt tccagggc 18
<210> 30
   <211> 20
   <212> DNA
   <213> Artificial Sequence
<220>
   <223> Oligonucleotide PCR Primer
<220>
   <221> INSP215-F4
<400> 30
   tgcgcagacc cctcttcagc 20
<210> 31
   <211> 21
   <212> DNA
   <213> Artificial Sequence
<220>
   <223> Oligonucleotide PCR Primer
<220>
   <221> INSP215-R4
<400> 31
   tgcacggccg gggacactct t 21
<210> 32
   <211> 48
   <212> DNA
   <213> Artificial Sequence
<220>
   <223> Oligonucleotide PCR Primer
<220>
   <221> INSP215-EX1
<400> 32
   ctcccaggga tcaccacctt acccagcggg ccacctgctc ccccgttc 48
<210> 33
   <211> 48
   <212> DNA
   <213> Artificial Sequence
<220>
   <223> Oligonucleotide PCR Primer
<220>
   <221> INSP215-EX2
<400> 33
   atgctgggca ccgtgctgct gctggccctg ctcccaggga tcaccacc 48
<210> 34
   <211> 33
   <212> DNA
   <213> Artificial Sequence
<220>
   <223> Oligonucleotide PCR Primer
<220>
   <221> INSP215-EX3
<400> 34
   gcaggcttcg ccaccatgct gggcaccgtg ctg 33
<210> 35
   <211> 32
   <212> DNA
   <213> Artificial Sequence
<220>
   <223> Oligonucleotide PCR Primer
<220>
   <221> INSP215-EX4
<400> 35
   tgatggtgat ggtgctggga cctcctgggc tg 32
<210> 36
   <211> 36
   <212> DNA
   <213> Artificial Sequence
<220>
   <223> Oligonucleotide PCR Primer
<220>
   <221> INSP215 Mut FP
<400> 36
   cgccctgagc ctcccgcgca ccatcaccat caccat 36
<210> 37
   <211> 36
   <212> DNA
   <213> Artificial Sequence
<220>
   <223> Oligonucleotide PCR Primer
<220>
   <221> INSP215 Mut RP
<400> 37
   atggtgatgg tgatggtgcg cgggaggctc agggcg 36
<210> 38
   <211> 37
   <212> DNA
   <213> Artificial Sequence
<220>
   <223> Oligonucleotide PCR Primer
<220>
   <221> GCP Forward
<400> 38
   ggggacaagt ttgtacaaaa aagcaggctt cgccacc 37
<210> 39
   <211> 51
   <212> DNA
   <213> Artificial Sequence
<220>
   <223> Oligonucleotide PCR Primer
<220>
   <221> GCP Reverse
<400> 39
   ggggaccact ttgtacaaga aagctgggtt tcaatggtga tggtgatggt g 51
<210> 40
   <211> 20
   <212> DNA
   <213> Artificial Sequence
<220>
   <223> Oligonucleotide PCR Primer
<220>
   <221> pEAK12F
<400> 40
   gccagcttgg cacttgatgt 20
<210> 41
   <211> 20
   <212> DNA
   <213> Artificial Sequence
<220>
   <223> Oligonucleotide PCR Primer
<220>
   <221> pEAK12R
<400> 41
   gatggaggtg gacgtgtcag 20
<210> 42
   <211> 18
   <212> DNA
   <213> Artificial Sequence
<220>
   <223> Oligonucleotide PCR Primer
<220>
   <221> 21M13
<400> 42
   tgtaaaacga cggccagt 18
<210> 43
   <211> 18
   <212> DNA
   <213> Artificial Sequence
<220>
   <223> Oligonucleotide PCR Primer
<220>
   <221> M13REV
<400> 43
   caggaaacag ctatgacc 18
<210> 44
   <211> 19
   <212> DNA
   <213> Artificial Sequence
<220>
   <223> Oligonucleotide PCR Primer
<220>
   <221> T7
<400> 44
   taatacgact cactatagg 19
<210> 45
   <211> 18
   <212> DNA
   <213> Artificial Sequence
<220>
   <223> PCR Primer
<220>
   <221> T3
<400> 45
   attaaccctc actaaagg 18
<210> 46
   <211> 95
   <212> PRT
   <213> Homo sapiens
<220>
   <221> Human ORFX ORF290 polypeptide sequence SEQ ID NO:580 in WO200058473
<400> 46
   Met Leu Gly Thr Val Leu Leu Leu Ala Leu Leu Pro Gly Ile Thr Thr
   1               5                   10                  15
   Leu Pro Ser Gly Pro Pro Ala Pro Pro Phe Pro Ala Ala Pro Gly Pro
               20                  25                  30
   Trp Leu Arg Arg Pro Leu Phe Ser Leu Lys Leu Ser Asp Thr Glu Asp
           35                  40                  45
   Val Phe Pro Arg Arg Ala Gly Pro Leu Glu Val Pro Ala Asp Ser Arg
       50                  55                  60
   Val Phe Val Gln Ala Ala Leu Ala Arg Pro Ser Pro Arg Trp Gly Leu
   65                  70                  75                  80
   Ala Leu His Arg Cys Ser Val Thr Pro Ser Ser Arg Pro Ala Pro
                   85                  90                  95
<210> 47
   <211> 316
   <212> PRT
   <213> Artificial Sequence
<220>
   <223> Homo sapiens
<220>
   <221> 33 KDA PROTEIN IPI00645072.1
<400> 47
   Met Gly Glu Ser Ala Ala Ala Thr Ala Ser Leu Phe Gln Arg Arg Arg
   1               5                   10                  15
   Arg Gly Arg Gly Gly Arg Val Thr Phe Pro Gly Gly Leu Lys Gly Ser
               20                  25                  30
   Ala Arg Phe Leu Ser Phe Gly Pro Pro Phe Pro Ala Pro Pro Ala Pro
           35                  40                  45
   Pro Phe Pro Ala Ala Pro Gly Pro Trp Leu Arg Arg Pro Leu Phe Ser
       50                  55                  60
   Leu Lys Leu Ser Asp Thr Glu Asp Val Phe Pro Arg Arg Ala Gly Pro
   65                  70                  75                  80
   Leu Glu Val Pro Ala Asp Ser Arg Val Phe Val Gln Ala Ala Leu Ala
                   85                  90                  95
   Arg Pro Ser Pro Arg Trp Gly Leu Ala Leu His Arg Cys Ser Val Thr
               100                 105                 110
   Pro Ser Ser Arg Pro Ala Pro Gly Pro Ala Leu Ala Leu Leu Arg Glu
           115                 120                 125
   Gly Cys Pro Ala Asp Thr Ser Val Ala Phe Pro Pro Pro Pro Pro Pro
       130                 135                 140
   Ser Pro Gly Ala Ala Arg Pro Ala Arg Phe Ser Phe Arg Leu Arg Pro
   145                 150                 155                 160
   Val Phe Asn Ala Ser Val Gln Phe Leu His Cys Gln Leu Ser Arg Cys
   165 170 175
   Arg Arg Leu Arg Gly Val Arg Arg Ala Pro Ala Pro Leu Thr Pro Pro
   180 185 190
   Pro Pro Pro Pro Pro Ser Arg Cys Leu Pro Gln Asp Glu Ala Cys Ala
   195 200 205
   Asp Thr Gly Ser Gly Ser Ala Glu Gly Leu Ala Ala Asp Gly Pro His
   210 215 220
   Leu His Thr Leu Thr Gln Pro Ile Val Val Thr Val Pro Arg Pro Pro
   225 230 235 240
   Pro Arg Pro Pro Lys Ser Val Pro Gly Arg Ala Val Arg Pro Glu Pro
   245 250 255
   Pro Ala Pro Ala Pro Ala Ala Leu Glu Pro Ala Pro Val Val Ala Leu
   260 265 270
   Val Leu Ala Ala Phe Val Leu Gly Ala Ala Leu Ala Ala Gly Leu Gly
   275 280 285
   Leu Val Cys Ala His Ser Ala Pro His Ala Pro Gly Pro Pro Ala Arg
   290 295 300
   Ala Ser Pro Ser Gly Pro Gln Pro Arg Arg Ser Gln
   305 310 315

## Description

The present invention relates to novel proteins containing a domain corresponding to the C-terminal half of a ZP domain similar to that identified in TGF-beta Receptor III and to the use of these proteins and nucleic acid sequences from the encoding genes in the diagnosis, prevention and treatment of diseases. In particular, the present invention relates to the use of these proteins for modulating the activity of proteins belonging to the TGF-beta superfamily.

### Background

The process of drug discovery is presently undergoing a fundamental revolution as the era of functional genomics comes of age. The term "functional genomics" applies to an approach utilising bioinformatics tools to ascribe function to protein sequences of interest. Such tools are becoming increasingly necessary as the speed of generation of sequence data is rapidly outpacing the ability of research laboratories to assign functions to these protein sequences.

As bioinformatics tools increase in potency and in accuracy, these tools are rapidly replacing the conventional techniques of biochemical characterisation. Indeed, the advanced bioinformatics tools used in identifying the present invention are now capable of outputting results in which a high degree of confidence can be placed.

Various institutions and commercial organisations are examining sequence data as they become available and significant discoveries are being made on an on-going basis. However, there remains a continuing need to identify and characterise further genes and the polypeptides that they encode, as targets for research and for drug discovery.

### Introduction to secreted proteins

The ability of cells to make and secrete extracellular proteins is central to many biological processes. Enzymes, growth factors, extracellular matrix proteins and signalling molecules are all secreted by cells. This is through fusion of a secretory vesicle with the plasma membrane. In most cases, but not all, proteins are directed to the endoplasmic reticulum and into secretory vesicles by a signal peptide. Signal peptides are cis-acting sequences that affect the transport of polypeptide chains from the cytoplasm to a membrane bound compartment such as a secretory vesicle. Polypeptides that are targeted to the secretory vesicles are either secreted into the extracellular matrix or are retained in the plasma membrane. The polypeptides that are retained in the plasma membrane will have one or more transmembrane domains. Examples of secreted proteins that play a central role in the functioning of a cell are cytokines, hormones, extracellular matrix proteins (adhesion molecules), proteases, and growth and differentiation factors.

### Introduction to cell-surface receptors

Cell-surface receptors are important components of any prokaryotic or eukaryotic cell and have been shown to play important roles in a large number of diverse physiological functions, many of which are involved in disease processes. Alteration of their activity is a means to alter the relevant disease phenotype and as such identification of novel cell-surface receptors is highly relevant.

### Introduction to Zona Pellucida proteins

The Zona Pellucida domain, or 'ZP' domain, is characterized by 8 highly conserved Cysteines and other polar or hydrophobic residues distributed over 260 amino acids that was first recognised in the mouse sperm receptors ZP2 and ZP3. However, many other proteins that are not mammalian egg coat proteins are now classified as containing ZP domains, including uromodulin, glycoprotein-2, a and β tectorins, transforming growth factor beta receptor III (TGR3) and endoglin (Bork P and Sander C, FEBS Lett. 1992, 300: 237-40; Jovine L et al., Nat Cell Biol. 2002, 4: 457-61). This domain is generally included in multidomain transmembrane proteins, wherein it is located in a single copy at the C-terminal section of the extracellular portion, and may perform various functions. It can mediate polymerisation of extracellular polypeptides but it can also be involved in the binding of a ligand. In fact, the C-terminal elements allowing the association to the cell membrane (a single transmembrane domain or a glycosyl-phosphatidylinositol anchor) can be separated proteoltically from the extracellular portion, and a mature form of the protein is secreted from the cell.

The ZP domain can be distinguished structurally and functionally in two halves, seemingly generated by duplication (Yonezawa N and Nakano M, Biochem Biophys Res Commun. 2003, 307: 877-82; Jovine L et al., Proc Natl Acad Sci U S A. 2004, 101: 5922-7). In particular, TGF-beta Receptor III contains a TGF-beta binding site in the C-terminal region of the ZP domain (WO 95/10610; Kaname S and Ruoslahti E, Biochem J. 1996, 315: 815-20).

Amongst the ZP domain-containing proteins, TGF-beta Receptor III is of particular importance, since it binds TGF-beta, a ligand itself belonging to a superfamily of proteins including not only TGF-beta but many other proteins, such as GDFs (Growth/Differention Factors), BMPs (Bone Morphogenic Proteins), Activins, or Inhibins, having therapeutic relevance (Tsuchida K et al., Curr Drug Targets Immune Endocr Metabol Disord. 2004, 4: 157-66; Chang H et al., Endocr Rev. 2002, 23: 787-823; de Caestecker M, Cytokine Growth Factor Rev. 2004, 15: 1-11). In fact, fragments of receptors for TGF-beta-like proteins have been disclosed in the literature having therapeutic utility (WO 95/10610; WO 97/05892; Bandyopadhyay A et al., Cancer Res. 2002, 62: 4690-5; Liu M et al., Am J Respir Crit Care Med. 2002, 165: 419-23; Vilchis-Landeros MM et al., Biochem J. 2001, 355: 215-22).

The variety of proteins known to contain the ZP domain and their physiological roles suggest that ZP domain-containing proteins may be important proteins involved in human pathologies such as cancers, retinopathies, neuropathies, reproductive disorders and developmental disorders.

Therefore, increasing knowledge of ZP domain containing proteins, and more preferably those homologous to TGF-beta Receptor III that can bind one or more members of the TGF-beta superfamily, is of extreme importance in increasing the understanding of the underlying pathways that lead to the disease states mentioned above, as well as other disease states, in particular those related to the specific modules and fragments, and in developing more effective therapies to treat disorders, such as cancers.

WO058473 discloses the nucleotide sequence GSN:AAC74735. WO058473 also discloses GSP:AAB40526.

EM EST:BG752983 discloses the 602732441F1 NIH_MGC_43 Homo sapiens cDNA clone IMAGE:4875922 5', mRNA sequence.

### THE INVENTION

The invention provides a polypeptide comprising the amino acid sequence as recited in SEQ ID NO:8.

The invention also provides a polypeptide comprising SEQ ID NO:10.

The invention also provides a polypeptide which is a fragment or a functional equivalent as recited in any one of the preceding claims, which has greater than 80% sequence identity with the amino acid sequence recited in SEQ ID NO:8, preferably greater than 85%, 90%. 95%, 98% or 99% sequence identity, and contains a ZP/TGR3 subdomain as found in any of the amino acid sequences encoded by SEQ ID NO:10, SEQ ID NO:12 or SEQ ID NO:14.

The present invention provides novel sequences containing a domain corresponding to the C-terminal half of a ZP domain similar to that identified in TGF-beta Receptor III (a ZP/TGR3 subdomain, from now on) that can have structural and functional features as indicated in the literature (Yonezawa N and Nakano M, Biochem Biophys Res Commun. 2003, 307: 877-82; Jovine L et al., Proc Natl Acad Sci U S A. 2004, 101: 5922-7). This sequence, alone or fused other sequences, can have a therapeutic effect in the treatment of diseases, in particular those associated with the excessive expression and secretion of a protein belonging to the TGF-beta protein superfamily. In particular, the invention is based on the finding that polypeptides of the present invention are inhibitors of growth factors. Preferably, the growth factors are TGF-beta like proteins. Preferably, the TGF-beta like protein is TGF-beta 1.

In a first aspect of the invention, there is provided a polypeptide which:
(i) comprises the amino acid sequence as recited in SEQ ID NO:8.

Also described herein is a polypeptide which:
(ii) is a fragment thereof which contains a ZP/TGR3 subdomain or has an antigenic determinant in common with the polypeptides of (i); or
(iii) is a functional equivalent of (i) or (ii).

Also described herein is a polypeptide according to part (i) of the first aspect of the invention may comprise the amino acid sequence as recited in SEQ ID NO:2, SEQ ID NO:4, SEQ ID NO:6, SEQ ID NO:19 or SEQ ID NO:21.

A polypeptide according to part (i) of the first aspect of the invention may consist of the amino acid sequence as recited in SEQ ID NO:2, SEQ ID NO:4, SEQ ID NO:6, SEQ ID NO:8, SEQ ID NO:19 or SEQ ID NO:21.

A polypeptide according to part (ii) of the above list may comprise the amino acid sequence as recited in SEQ ID NO:10. Such polypeptides may comprise the amino acid sequence as recited in SEQ ID NO:12 or SEQ ID NO:14. Such polypeptides may consist of the amino acid sequence as recited in SEQ ID NO:10, SEQ ID NO:12 or SEQ ID NO:14.

Also described herein is a polypeptide which is a functional equivalent according to part (iii) of the above list may be homologous to the amino acid sequence as recited in SEQ ID NO:8, and preferably contains a ZP/TGR3 subdomain.

The invention provides a polypeptide which is a fragment or a functional equivalent as recited above preferably has greater than 80% sequence identity with the amino acid sequence recited in SEQ ID NO:8 or with an active fragment thereof, preferably greater than 85%, 90%, 95%, 98% or 99% sequence identity, and preferably contains a ZP/TGR3 subdomain.

Also described herein is a polypeptide which is a functional equivalent as recited above, preferably exhibits significant structural homology with a polypeptide having the amino acid sequence recited in SEQ ID NO:8, and preferably contains a ZP/TGR3 subdomain.

A polypeptide which is a fragment as recited above having an antigenic determinant in common with the polypeptide of part (i) above may consist of 7 or more amino acid residues from the amino acid sequence recited in SEQ ID NO:8.

Although the Applicant does not wish to be bound by this theory, it is postulated that the first 16 amino acids of the full length INSP215 polypeptide form a signal peptide. The full length INSP215 polypeptide sequence without the postulated signal sequence is recited in SEQ ID NO:4. The INSP215 extracellular region polypeptide without the postulated signal sequence is recited in SEQ ID NO:8. The INSP215 exons 1-4 polypeptide without the postulated signal sequence is recited in SEQ ID NO:14.

The polypeptide having the sequence recited in SEQ ID NO:2 is referred to hereafter as "the INSP215 full length polypeptide". The polypeptide having the sequence recited in SEQ ID NO:4 is referred to hereafter as "the mature INSP215 full length polypeptide". The polypeptide having the sequence recited in SEQ ID NO:6 is referred to hereafter as "the INSP215 extracellular region polypeptide". The polypeptide having the sequence recited in SEQ ID NO:8 is referred to hereafter as "the mature INSP215 extracellular region polypeptide". The polypeptide having the sequence recited in SEQ ID NO:10 is referred to hereafter as "the INSP215 ZP/TGR3 subdomain polypeptide". The polypeptide having the sequence recited in SEQ ID NO:12 is referred to hereafter as "the INSP215 exons 1-4 polypeptide". The polypeptide having the sequence recited in SEQ ID NO:14 is referred to hereafter as "the mature INSP215 exons 1-4 polypeptide". The polypeptide having the sequence recited in SEQ ID NO:19 is referred to hereafter as "the INSP215 full length polypeptide with 6HIS tag". The polypeptide having the sequence recited in SEQ ID NO:21 is referred to hereafter as "the INSP215 extracellular region polypeptide with 6HIS tag".

The term "INSP215 polypeptides" as used herein includes polypeptides comprising or consisting of the mature INSP215 extracellular region polypeptide of SEQ ID NO:8, such as the INSP215 full length polypeptide, the mature INSP215 full length polypeptide, the INSP215 extracellular region polypeptide, the mature INSP215 extracellular region polypeptide, the INSP215 ZP/TGR3 subdomain polypeptide, the INSP215 exons 1-4 polypeptide, the mature INSP215 exons 1-4 polypeptide, the INSP215 full length polypeptide with 6HIS tag and the INSP215 extracellular region polypeptide with 6HIS tag.

By "contains a ZP/TGR3 subdomain" we refer to polypeptides that contain a domain corresponding to the C-terminal half of a ZP domain similar to that identified in TGF-beta Receptor III (the ZP/TGR3 subdomain) that can have structural and functional features as indicated in the literature (Yonezawa N and Nakano M, Biochem Biophys Res Commun. 2003, 307: 877-82; Jovine L et al., Proc Natl Acad Sci U S A. 2004, 101: 5922-7). Preferably, polypeptides that contain a ZP/TGR3 subdomain will comprise the INSP215 ZP/TGR3 subdomain polypeptide of SEQ ID NO:10.

It is known that a number of ZP domain-containing cell-surface receptors are secreted from the cell following cleavage of the polypeptide chain at a specific site between the ZP domain and the transmembrane region (or GPI anchor binding site). Thus, secreted forms of the INSP215 polypeptides are anticipated, and may for example comprise or consist of an amino acid sequence as recited in SEQ ID NO:6, SEQ ID NO:8, SEQ ID NO:10, SEQ ID NO:12, SEQ ID NO:14 or SEQ ID NO:21.

As noted above, the present invention provides novel polypeptide sequences containing a domain sequence similar to the C-terminal half of a ZP domain identified in TGF-beta Receptor III (the ZP/TGR3 subdomain).

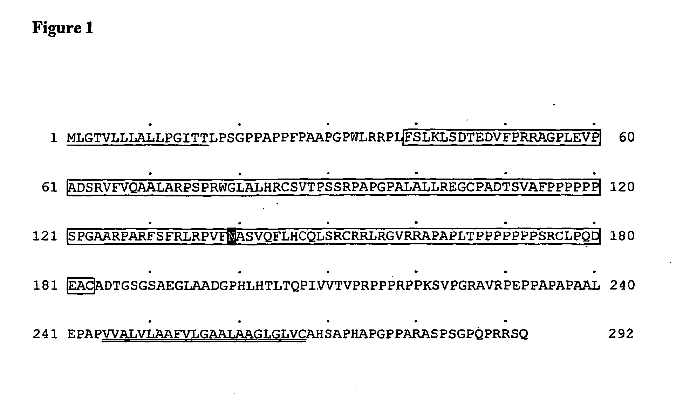

An open reading frame of 879 nucleotides (SEQ ID NO:1) encodes the INSP215 full length polypeptide (SEQ ID NO:2), a protein of 292 amino acids (see Figure 1). The INSP215 full length polypeptide contains a predicted signal peptide (residues 1-16) leading to a mature sequence of 276 amino acids (SEQ ID NO:4), encoded by a DNA sequence of 831 nucleotides (SEQ ID NO:3). The INSP215 full length polypeptide contains a predicted transmembrane region (residues 245-268) separating an intracellular region (residues 269-292) and an extracellular region (residues 1-244; SEQ ID NO:5 and SEQ ID NO:6). Further features of the extracellular region are the mature sequence (SEQ ID NO:7 and SEQ ID NO:8) containing the predicted ZP/TGR3 subdomain (SEQ ID NO:9 and SEQ ID NO:10) and a N-glycosylation site (located at residue 139). The ZP/TGR3 subdomain is found in the amino acid sequence encoded by the first four of the six exons encoding the INSP215 full length polypeptide (SEQ ID NOs 11-14).

If the INSP215 full length polypeptide is used as a BLAST query against non-redundant sequence public databases, a segment of INSP215 extracellular region is found to be highly homologous to the C-terminal half of the ZP/TGR3 domain (Bork P and Sander C, FEBS Lett. 1992, 300: 237-40; Jovine L et al., Nat Cell Biol. 2002, 4: 457-61). This similarity between INSP215 and TGF-beta Receptor III in the ZP/TGR3 domain is not limited to the regularly spaced cysteine residues, but also involves several other polar or hydrophobic residues, as well as a N-glycosylation site.

The expression of the INSP215 polypeptide is supported by the existence of numerous ESTs in humans, mice and rats (see the Examples herein).

The literature shows that the ZP/TGR3 domain can be distinguished structurally and functionally in two halves, and that a TGF-beta binding site is located in the C-terminal region of the ZP domain (WO 95/10610; Kaname S and Ruoslahti E, Biochem J. 1996, 315: 815-20). Accordingly, it is expected that the INSP215 polypeptides and functional fragments and/or fusion proteins based on the INSP215 polypeptides, in particular polypeptides comprising the amino acid sequence recited in SEQ ID NO:10, will bind one or more proteins belonging to the TGF-beta protein superfamily. When these INSP215-related sequences are secreted or soluble proteins, they can act as antagonists of proteins belonging to the TGF-beta protein superfamily.

On the basis of the annotation of the INSP215 polypeptides herein, it is now possible to design experiments to detect the presence of the INSP215 transcripts across a range of human tissue types to determine its tissue expression, in particular in normal and diseased tissues in order to establish more particularly the relevance of INSP215 in pathological contexts.

The cloning of the INSP215-related DNA sequences (either as cDNA or genomic exonic sequences, and exemplified in SEQ ID NO:1, 3, 5, 7, 9, 11 and 13) from tissues of human or mammalian origin will allow the expression of the corresponding encoded protein sequences in prokaryotic or eukaryotic expression systems and their subsequent purification and characterization as recombinant proteins.

Alternative recombinant INSP215 sequences can be produced by cloning in the appropriate expression vector the DNA encoding for the full sequence (SEQ ID NO:2), or for its selected fragments, in the form of signal peptide-containing (SEQ ID NO:6 and 12) or mature sequences (SEQ ID NO:4, 8, 10, and 14) maintaining the expected binding properties of the full protein towards proteins belonging to the TGF-beta superfamily. For example, transcripts including exons 1-4 (covering the entire ZP/TGR3 subdomain and the signal sequence) can be identified and/or cloned by primer hybridization or by PCR.

Whenever it is appropriate, heterologous DNA sequences encoding for protein sequences helping the expression, the secretion, and/or the detection of these recombinant sequences may be added in frame to the DNA encoding INSP215, or the derived protein sequences identified above. Examples of such heterologous sequences are signal peptides, immunoglobulin Fc regions, and histidine tags.

Soluble variants of INSP215 are expected to bind one or more members of the TGF-beta protein superfamily, capturing and retaining this protein from interacting with the signaling receptors, with the consequent inhibition of the proliferation and differentiation of multiple cell types normally induced by TGF-beta-like proteins. Therefore, the specific portions of INSP215 indicated herein as secreted or soluble proteins, which may be generated *in* vivo or *in vitro* following proteolytic digestion or alternative splicing, can have specific *in vivo* properties, in particular as antagonists of one or more protein belonging to the TGF-beta superfamily. Such polypeptides are therefore expected to be useful for the treatment of diseases, in particular cancers, fibrosis, scarring, hypertension, atherosclerosis, reproductive disorders, hepatic disorders, lung disorders, immune disorders, developmental disorders, skin disorders, or disorders of connective tissues (Tsuchida K et al., Curr Drug Targets Immune Endocr Metabol Disord. 2004, 4: 157-66; Chang H et al., Endocr Rev. 2002, 23: 787-823; de Caestecker M, Cytokine Growth Factor Rev. 2004, 15: 1-11; WO 95/10610; WO 97/05892; Bandyopadhyay A et al., Cancer Res. 2002, 62: 4690-5; Esparza-Lopez J et al., J Biol Chem. 2001, 276: 14588-96; Vilchis-Landeros MM et al., Biochem J. 2001, 355: 215-22).

The polypeptides disclosed herein may be useful in the treatment of diseases such as metastatic cancer, non-small-cell lung cancer, colon cancer, leukemia, testicular cancer, myeloma, lymphoma, colorectal cancer, prostate cancer, brain cancers, eye cancers, retinoblastoma, cancer of the uterus, cancer of the gut, cancer of the uterine cervix, endometrial carcinoma, lung cancer, cancer of the pancreas, chondrosarcoma, non-Hodgkin's lymphomas, ovarian sex cord-stromal tumors, ovarian carcinomas, gonadal tumors, renal cell cancer, B-chronic lymphocytic leukemia, gastric carcinoma, pancreatic carcinomas, pancreatic adenocarcinoma, small-cell lung cancer, gliomas, high-grade gliomas, malignant gliomas, gliosarcomas, anaplastic astrocytomas, glioblastomas, medulloblastoma, ependyoma, T-cell malignancies, bone marrow transplantations, fibrosis, idiopathic pulmonary fibrosis, scleroderma, post-operative scarring following glaucoma surgery, renal disease, diabetes, diabetic nephropathy, atherosclerosis, restrictive cardiomyopathy, angiogenesis disorder, bronchiolitis obliterans, cachexia, chronic obstructive pulmonary disease, wound healing, Crouzon's syndrome or glomerulonephritis.

Preferably, the leukemia is lymphocytic leukemia, acute lymphocytic leukemia, acute B cell leukemia, acute T cell leukemia, acute myelogenous leukemia, chronic myelogenous leukemia, or chronic lymphocytic leukemia.

Preferably the testicular cancer is seminoma, choriocarcinoma, embryonal carcinoma, teratoma or yolk sac tumor.

Preferably, the lung cancer is selected from benign or malignant primary tumors or from metastases from primary cancers of many other organs and tissues. Preferably the lung cancer is selected from primary lung tumors including bronchogenic carcinoma, bronchial carcinoid, chondromatous hamartoma (benign), solitary lymphoma, sarcoma (malignant) or multifocal lymphomas. Preferably, the bronchogenic carcinoma is selected from squamous cell carcinoma, undifferentiated small cell carcinoma, undifferentiated large cell carcinoma, adenocarcinoma or bronchioloalveolar carcinoma. Preferably, the bronchogenic carcinoma is squamous cell carcinoma or non-small cell lung carcinoma. Preferably the lung cancer is selected from metastases from primary cancers of the skin, breast, colon, prostate, kidney, thyroid, stomach, cervix, rectum, testis, and bone and from melanoma.

Preferably, the lymphoma is Hodgkin's Disease, small lymphocytic lymphoma (SLL/CLL), mantle cell lymphoma (MCL), follicular lymphoma (FL), marginal zone lymphoma (MZL) including extranodal (MALT lymphoma), nodal (monocytoid B-cell lymphoma) or splenic, diffuse large cell lymphoma, Burkitt's lymphoma, high grade Burkitt-like lymphoma or lymphoblastic lymphoma.

Recombinant INSP215 and the INSP215 polypeptides identified herein may be used to generate specific monoclonal or polyclonal antibodies that might then be used in the biochemical characterization of INSP215, as well as for diagnostic and/or therapeutic applications (in view of the information disclosed in the literature on fragments generated from these family of proteins). Alternatively, recombinant INSP215, and the INSP215 polypeptides identified herein, may be used in a wide variety of screening assays, in particular involving proteins included in the TGF-beta protein superfamily, for identifying specific ligands and associated biological activities.

Preferably, the activity of a soluble polypeptide of the present invention, alone or as part of a fusion protein, or an antagonist of a membrane bound polypeptide of the present invention, can be confirmed in at least one of the following assays:
a) in the modulation of apoptosis (for example by tunnel staining of activated caspase 3 by IHC), or
b) in the modulation of cell proliferation (e.g. use of Ki-67 or PCNA by IHC), or
c) in the modulation of angiogenesis or neovascularization (e.g. CD34 by immunohistochemistry (IHC), or
d) in the modulation of immunosuppression (e.g. NK cell activity, T-cell activation, macrophage activation markers by IHC), or
e) in the modulation of cell migration (e.g. markers of EMT by IHC, vimentin, E-cadherin, SNAIL), or
f) in the modulation of tumour-host interaction (e.g. growth-factor pathway activation; HGF, EGF, PDGF, VEGF), or
g) in the modulation of tumour growth, or
h) in the modulation of metastasis, or
i) in the modulation of cell survival, or
j) in the modulation of oocyte maturation, or
k) in the modulation of follicular growth, or
l) in the assay as described in Example 4 herein, or
m) in the syngeneic murine 4T1 mammary carcinoma model, or
n) in the floxed TGFBR2 allele mouse model, or
o) in mouse models of metastatic breast cancer (e.g. the MMTV/Neu mice), or
p) in rabbits subjected to modified glaucoma surgery, or
q) in a rat model of diabetes, or
r) in rats with established intracranial gliomas, or
s) in the C6 rat glioma model, or
t) in a rat model of Bronchiolitis obliterans as described by Liu et al. (Am. J. Respir. Crit. Care Med. 2002. Vol.165, pp.419-423), or
u) in the xenograft model as described by Bandyopadhyay et al. (Prostate 2005, Vol.63, Issue 1, pp.81-90).

In addition, activity of a soluble INSP215 or an antagonist of membrane bound INSP215 can be demonstrated in models of cancer as reviewed by Kamb and Lassota (Drug Discovery Today: Disease Models; Vol. 1, No. 1, 2004, pp. 31-36) and Grunt et al. (Drug Discovery Today: Disease Models; Vol. 1, No. 1, 2004, pp. 17-23), in models of lung cancer as reviewed by Lahm and Fischer (Drug Discovery Today: Disease Models; Vol. 1, No. 1, 2004, pp. 25-30), in models of multiple myeloma as reviewed by Caers et al. (Drug Discovery Today: Disease Models; Vol. 1, No. 4, 2004, pp. 373-380), in models of colon cancer as reviewed by Albanese et al. (Drug Discovery Today: Disease Models; in Press), in models of pancreatic cancer as reviewed by Thomas and Lowy (Drug Discovery Today: Disease Models; in Press), or in models of breast cancer as reviewed by Matulka and Wagner (Drug Discovery Today: Disease Models; in Press) and Gupta and Kuperwasser (Drug Discovery Today: Disease Models; Vol. 1, No. 1, 2004, pp. 9-16).

An "antigenic determinant" described herein may be a part of a polypeptide of the present invention, which binds to an antibody-combining site or to a T-cell receptor (TCR). Alternatively, an "antigenic determinant" may be a site on the surface of a polypeptide of the present invention to which a single antibody molecule binds. Generally an antigen has several or many different antigenic determinants and reacts with antibodies of many different specificities. Preferably, the antibody is immunospecific to a polypeptide of the invention. Preferably, the antibody is immunospecific to a polypeptide of the invention, which is not part of a fusion protein. Preferably, the antibody is immunospecific to INSP215 or a fragment thereof. Antigenic determinants usually consist of chemically active surface groupings of molecules, such as amino acids or sugar side chains, and can have specific three dimensional structural characteristics, as well as specific charge characteristics. Preferably, the "antigenic determinant" refers to a particular chemical group on a polypeptide of the present invention that is antigenic, i.e. that elicit a specific immune response.

The Human ORFX ORF290 polypeptide sequence SEQ ID NO:580 disclosed in WO200058473 (SEQ ID NO:46 herein) and the 33 KDA PROTEIN IPI00645072.1 (SEQ ID NO:47 herein), and their encoding nucleic acid sequences are specifically excluded from the scope of this invention.

In a second aspect, the invention provides a purified nucleic acid molecule which encodes a polypeptide of the first aspect of the invention

The term "purified nucleic acid molecule" preferably refers to a nucleic acid molecule of the invention that (1) has been separated from at least about 50 percent of proteins, lipids, carbohydrates, or other materials with which it is naturally found when total nucleic acid is isolated from the source cells, (2) is not linked to all or a portion of a polynucleotide to which the "purified nucleic acid molecule" is linked in nature, (3) is operably linked to a polynucleotide which it is not linked to in nature, or (4) does not occur in nature as part of a larger polynucleotide sequence. Preferably, the isolated nucleic acid molecule of the present invention is substantially free from any other contaminating nucleic acid molecule(s) or other contaminants that are found in its natural environment that would interfere with its use in polypeptide production or its therapeutic, diagnostic, prophylactic or research use. In a preferred embodiment, genomic DNA are specifically excluded from the scope of the invention. Preferably, genomic DNA larger than 10 kbp (kilo base pairs), 50 kbp, 100 kbp, 150 kbp, 200 kbp, 250 kbp or 300 kbp are specifically excluded from the scope of the invention. Preferably, the "purified nucleic acid molecule" consists of cDNA only.

Preferably, the purified nucleic acid molecule comprises the nucleic acid sequence as recited in SEQ ID NO:1 (encoding the INSP215 full length polypeptide), SEQ ID NO:3 (encoding the mature INSP215 full length polypeptide), SEQ ID NO:5 (encoding the INSP215 extracellular region polypeptide), SEQ ID NO:7 (encoding the mature INS215 extracellular region polypeptide), SEQ ID NO:9 (encoding the INSP215 ZP/TGR3 subdomain polypeptide), SEQ ID NO:11 (encoding the INSP215 exons 1-4 polypeptide), SEQ ID NO:13 (encoding the mature INSP215 exons 1-4 polypeptide), SEQ ID NO:16 (the INSP215 predicted cDNA sequence), SEQ ID NO:17 (the cDNA insert in Image clone 4875922), SEQ ID NO:18 (encoding the INSP215 full length polypeptide with 6HIS tag) or SEQ ID NO:20 (encoding the INSP215 extracellular polypeptide with 6HIS tag), or is a redundant equivalent or fragment of those sequences.

The invention further provides that the purified nucleic acid molecule consists of the nucleic acid sequence as recited in SEQ ID NO:1 (encoding the INSP215 full length polypeptide), SEQ ID NO:3 (encoding the mature INSP215 full length polypeptide), SEQ ID NO:5 (encoding the INSP215 extracellular region polypeptide), SEQ ID NO:7 (encoding the mature INSP215 extracellular region polypeptide), SEQ ID NO:9 (encoding the INSP215 ZP/TGR3 subdomain polypeptide), SEQ ID NO:11 (encoding the INSP215 exons 1-4 polypeptide), SEQ ID NO:13 (encoding the mature INSP215 exons 1-4 polypeptide), SEQ ID NO:16 (the INSP215 predicted cDNA sequence), SEQ ID NO:17 (the cDNA insert in Image clone 4875922), SEQ ID NO:18 (encoding the INSP215 full length polypeptide with 6HIS tag) or SEQ ID NO:20 (encoding the INSP215 extracellular polypeptide with 6HIS tag), or a redundant equivalent or fragment of these sequences.

In a third aspect, the disclosure provides a purified nucleic acid molecule which hybridizes under high stringency conditions with a nucleic acid molecule of the second aspect of the invention. High stringency hybridisation conditions are defined as overnight incubation at 42°C in a solution comprising 50% formamide, 5XSSC (150mM NaCl, 15mM trisodium citrate), 50mM sodium phosphate (pH7.6), 5x Denhardts solution, 10% dextran sulphate, and 20 microgram/ml denatured, sheared salmon sperm DNA, followed by washing the filters in 0.1X SSC at approximately 65°C.

In a fourth aspect, the invention provides a vector, such as an expression vector, that contains a nucleic acid molecule of the second or third aspect of the disclosure. Preferred vectors include those described in the Examples herein.

In a fifth aspect, the invention provides a host cell transformed with a vector of the fourth aspect of the invention.

In a sixth aspect, the invention provides a ligand which binds specifically to a polypeptide of the first aspect of the invention.

In a seventh aspect, the disclosure provides a compound that is effective to alter the expression of a natural gene which encodes a polypeptide of the first aspect of the invention or to regulate the activity of a polypeptide of the first aspect of the invention.

Such compounds may be identified using the assays and screening methods disclosed herein.

A compound of the seventh aspect of the disclosure may either increase (agonise) or decrease (antagonise) the level of expression of the gene or the activity of the polypeptide.

Importantly, the identification of the function of the INSP215 polypeptides allows for the design of screening methods capable of identifying compounds that are effective in the treatment and/or diagnosis of disease. Ligands and compounds according to the sixth and seventh aspects of the disclosure may be identified using such methods. These methods are included as aspects of the present disclosure.

Another aspect of this invention resides in the use of an INSP215 gene or polypeptide as a target for the screening of candidate drug modulators, particularly candidate drugs active against the diseases mentioned herein.

Also described herein are methods of screening of compounds for therapy of the diseases mentioned herein, comprising determining the ability of a compound to bind to an INSP215 gene or polypeptide, or a fragment thereof.

Also described herein are methods of screening of compounds for therapy of the diseases mentioned herein, comprising testing for modulation of the activity of an INSP215 gene or polypeptide, or a fragment thereof.

In an eighth aspect, the invention provides a polypeptide of the first aspect of the invention, or a nucleic acid molecule of the second or third aspect of the disclosure or a vector of the fourth aspect of the invention, or a host cell of the fifth aspect of the invention, or a ligand of the sixth aspect of the invention, or a compound of the seventh aspect of the disclosure, for use in therapy or diagnosis of diseases in which ZP domain-containing polypeptides are implicated. Such diseases include cell proliferative disorders, cancer, cardiovascular disorders, neurological disorders, metabolic disorders, infection, immune disorders, autoimmune disease, inflammation, genetic disorders and particularly retinopathies, reproductive disorders and developmental disorders. Preferably, the disease is a disease in which ZP/TGR3 domain-containing proteins are implicated, such as cancers, fibrosis, scarring, hypertension, atherosclerosis, reproductive disorders, hepatic disorders, lung disorders, immune disorders, developmental disorders, skin disorders and disorders of connective tissues. These moieties of the first, second, third, fourth, fifth, sixth or seventh aspect of the disclosure may also be used in the manufacture of a medicament for the treatment of such diseases.

In particular, the moieties of the first, second, third, fourth, fifth, sixth or seventh aspect of the disclosure may be useful in the treatment of diseases such as metastatic cancer, non-small-cell lung cancer, colon cancer, leukemia, testicular cancer, myeloma, lymphoma, colorectal cancer, prostate cancer, brain cancers, eye cancers, retinoblastoma, cancer of the uterus, cancer of the gut, cancer of the uterine cervix, endometrial carcinoma, lung cancer, cancer of the pancreas, chondrosarcoma, non-Hodgkin's lymphomas, ovarian sex cord-stromal tumors, ovarian carcinomas, gonadal tumors, renal cell cancer, B-chronic lymphocytic leukemia, gastric carcinoma, pancreatic carcinomas, pancreatic adenocarcinoma, small-cell lung cancer, gliomas, high-grade gliomas, malignant gliomas, gliosarcomas, anaplastic astrocytomas, glioblastomas, medulloblastoma, ependyoma, T-cell malignancies, bone marrow transplantations, fibrosis, idiopathic pulmonary fibrosis, scleroderma, post-operative scarring following glaucoma surgery, renal disease, diabetes, diabetic nephropathy, atherosclerosis, restrictive cardiomyopathy, angiogenesis disorder, bronchiolitis obliterans, cachexia, chronic obstructive pulmonary disease, wound healing, Crouzon's syndrome or glomerulonephritis.

Preferably, the leukemia is lymphocytic leukemia, acute lymphocytic leukemia, acute B cell leukemia, acute T cell leukemia, acute myelogenous leukemia, chronic myelogenous leukemia, or chronic lymphocytic leukemia.

Preferably the testicular cancer is seminoma, choriocarcinoma, embryonal carcinoma, teratoma or yolk sac tumor.

Preferably, the lung cancer is selected from benign or malignant primary tumors or from metastases from primary cancers of many other organs and tissues. Preferably the lung cancer is selected from primary lung tumors including bronchogenic carcinoma, bronchial carcinoid, chondromatous hamartoma (benign), solitary lymphoma, sarcoma (malignant) or multifocal lymphomas. Preferably, the bronchogenic carcinoma is selected from squamous cell carcinoma, undifferentiated small cell carcinoma, undifferentiated large cell carcinoma, adenocarcinoma or bronchioloalveolar carcinoma. Preferably, the bronchogenic carcinoma is squamous cell carcinoma or non-small cell lung carcinoma. Preferably the lung cancer is selected from metastases from primary cancers of the skin, breast, colon, prostate, kidney, thyroid, stomach, cervix, rectum, testis, and bone and from melanoma.

Preferably, the lymphoma is Hodgkin's Disease, small lymphocytic lymphoma (SLL/CLL), mantle cell lymphoma (MCL), follicular lymphoma (FL), marginal zone lymphoma (MZL) including extranodal (MALT lymphoma), nodal (monocytoid B-cell lymphoma) or splenic, diffuse large cell lymphoma, Burkitt's lymphoma, high grade Burkitt-like lymphoma or lymphoblastic lymphoma.

In a ninth aspect, the disclosure provides a method of diagnosing a disease in a patient, comprising assessing the level of expression of a natural gene encoding a polypeptide of the first aspect of the invention or the activity of a polypeptide of the first aspect of the invention in tissue from said patient and comparing said level of expression or activity to a control level, wherein a level that is different to said control level is indicative of disease. Such a method will preferably be carried out *in vitro.* Similar methods may be used for monitoring the therapeutic treatment of disease in a patient, wherein altering the level of expression or activity of a polypeptide or nucleic acid molecule over the period of time towards a control level is indicative of regression of disease.

A preferred method for detecting polypeptides of the first aspect of the invention comprises the steps of: (a) contacting a ligand, such as an antibody, of the sixth aspect of the invention with a biological sample under conditions suitable for the formation of a ligand-polypeptide complex; and (b) detecting said complex.

A number of different such methods according to the ninth aspect of the disclosure exist, as the skilled reader will be aware, such as methods of nucleic acid hybridization with short probes, point mutation analysis, polymerase chain reaction (PCR) amplification and methods using antibodies to detect aberrant protein levels. Similar methods may be used on a short or long term basis to allow therapeutic treatment of a disease to be monitored in a patient. The invention also provides kits that are useful in these methods for diagnosing disease.

Preferably, the disease diagnosed by a method of the ninth aspect of the disclosure is a disease in which ZP domain-containing polypeptides are implicated, or a disease in which ZP/TGR3 domain-containing proteins are implicated, as described above.

In a tenth aspect, the disclosure provides for the use of a polypeptide of the first aspect of the invention as a cell surface receptor or a soluble form thereof. Suitable uses of the polypeptides of this invention as cell-surface receptors will be clear to the skilled reader. Particular examples of suitable uses include screening methods to identify ligands and other agonist or antagonist molecules that are useful in therapy and diagnosis of diseases and conditions in which this category of protein are implicated. In particular, the invention provides for the use of a transmembrane or soluble form of a polypeptide of the first aspect of the invention for binding a protein belonging to the TGF-beta protein superfamily.

In an eleventh aspect, the disclosure provides a pharmaceutical composition comprising a polypeptide of the first aspect of the invention, or a nucleic acid molecule of the second or third aspect of the disclosure, or a vector of the fourth aspect of the invention, or a host cell of the fifth aspect of the invention, or a ligand of the sixth aspect of the invention, or a compound of the seventh aspect of the disclosure, in conjunction with a pharmaceutically-acceptable carrier.

In a twelfth aspect, the present disclosure provides a polypeptide of the first aspect of the invention, or a nucleic acid molecule of the second or third aspect of the invention, or a vector of the fourth aspect of the invention, or a host cell of the fifth aspect of the invention, or a ligand of the sixth aspect of the invention, or a compound of the seventh aspect of the disclosure, for use in the manufacture of a medicament for the diagnosis or treatment of a disease.

Preferably, the disease is a disease in which ZP domain-containing polypeptides are implicated, or a disease in which ZP/TGR3 domain-containing proteins are implicated, as described above.

In a thirteenth aspect, the disclosure provides a method of treating a disease in a patient comprising administering to the patient a polypeptide of the first aspect of the invention, or a nucleic acid molecule of the second or third aspect of the disclosure, or a vector of the fourth aspect of the invention, or a host cell of the fifth aspect of the invention, or a ligand of the sixth aspect of the invention, or a compound of the seventh aspect of the disclosure.

Preferably, the disease is a disease in which ZP domain-containing polypeptides are implicated, or a disease in which ZP/TGR3 domain-containing proteins are implicated, as described above.

For diseases in which the expression of a natural gene encoding a polypeptide of the first aspect of the invention, or in which the activity of a polypeptide of the first aspect of the invention, is lower in a diseased patient when compared to the level of expression or activity in a healthy patient, the polypeptide, nucleic acid molecule, vector, host cell, ligand or compound administered to the patient should be an agonist. Conversely, for diseases in which the expression of the natural gene or activity of the polypeptide is higher in a diseased patient when compared to the level of expression or activity in a healthy patient, the polypeptide, nucleic acid molecule, vector, host cell, ligand or compound administered to the patient should be an antagonist. Examples of such antagonists include antisense nucleic acid molecules, ribozymes and ligands, such as antibodies.

In a fourteenth aspect, the disclosure provides transgenic or knockout non-human animals that have been transformed to express higher, lower or absent levels of a polypeptide of the first aspect of the invention. Such transgenic animals are very useful models for the study of disease and may also be used in screening regimes for the identification of compounds that are effective in the treatment or diagnosis of such a disease.

Preferably, the disease is a disease in which ZP domain-containing polypeptides are implicated, or a disease in which ZP/TGR3 domain-containing proteins are implicated, as described above.

As used herein, "functional equivalent" refers to a protein or nucleic acid molecule that possesses functional or structural characteristics that are substantially similar to a polypeptide or nucleic acid molecule of the present invention. A functional equivalent of a protein may contain modifications depending on the necessity of such modifications for the performance of a specific function. The term "functional equivalent" is intended to include the fragments, mutants, hybrids, variants, analogs, or chemical derivatives of a molecule.

Preferably, the "functional equivalent" may be a protein or nucleic acid molecule that exhibits any one or more of the functional activities of the polypeptides of the present invention.

Preferably, the "functional equivalent" may be a protein or nucleic acid molecule that displays substantially similar activity compared with INSP215 or fragments thereof in a suitable assay for the measurement of biological activity or function. Preferably, the "functional equivalent" may be a protein or nucleic acid molecule that displays identical or higher activity compared with INSP215 or fragments thereof in a suitable assay for the measurement of biological activity or function. Preferably, the "functional equivalent" may be a protein or nucleic acid molecule that displays 50%, 60%, 70%, 80%, 90%, 95%, 98%, 99%, 100% or more activity compared with INSP215 or fragments thereof in a suitable assay for the measurement of biological activity or function.

Preferably, the "functional equivalent" may be a protein or polypeptide capable of exhibiting a substantially similar *in vivo* or *in vitro* activity as the polypeptides of the invention. Preferably, the "functional equivalent" may be a protein or polypeptide capable of interacting with other cellular or extracellular molecules in a manner substantially similar to the way in which the corresponding portion of the polypeptides of the invention would. For example, a "functional equivalent" would be able, in an immunoassay, to diminish the binding of an antibody to the corresponding peptide (*i.e.,* the peptide the amino acid sequence of which was modified to achieve the "functional equivalent") of the polypeptide of the invention, or to the polypeptide of the invention itself, where the antibody was raised against the corresponding peptide of the polypeptide of the invention. An equimolar concentration of the functional equivalent will diminish the aforesaid binding of the corresponding peptide by at least about 5%, preferably between about 5% and 10%, more preferably between about 10% and 25%, even more preferably between about 25% and 50%, and most preferably between about 40% and 50%.

For example, functional equivalents can be fully functional or can lack function in one or more activities. Thus, in the present invention, variations can affect the function, for example, of the activities of the polypeptide that reflect its possession of a ZP/TGR3 subdomain.

A summary of standard techniques and procedures which may be employed in order to utilise the invention is given below. It will be understood that this invention is not limited to the particular methodology, protocols, cell lines, vectors and reagents described. It is also to be understood that the terminology used herein is for the purpose of describing particular embodiments only and it is not intended that this terminology should limit the scope of the present invention. The extent of the invention is limited only by the terms of the appended claims.

Standard abbreviations for nucleotides and amino acids are used in this specification.

The practice of the present invention will employ, unless otherwise indicated, conventional techniques of molecular biology, microbiology, recombinant DNA technology and immunology, which are within the skill of those working in the art.

Such techniques are explained fully in the literature. Examples of particularly suitable texts for consultation include the following: Sambrook Molecular Cloning; A Laboratory Manual, Second Edition (1989); DNA Cloning, Volumes I and II (D.N Glover ed. 1985); Oligonucleotide Synthesis (M.J. Gait ed. 1984); Nucleic Acid Hybridization (B.D. Hames & S.J. Higgins eds. 1984); Transcription and Translation (B.D. Hames & S.J. Higgins eds. 1984); Animal Cell Culture (R.I. Freshney ed. 1986); Immobilized Cells and Enzymes (IRL Press, 1986); B. Perbal, A Practical Guide to Molecular Cloning (1984); the Methods in Enzymology series (Academic Press, Inc.), especially volumes 154 & 155; Gene Transfer Vectors for Mammalian Cells (J.H. Miller and M.P. Calos eds. 1987, Cold Spring Harbor Laboratory); Immunochemical Methods in Cell and Molecular Biology (Mayer and Walker, eds. 1987, Academic Press, London); Scopes, (1987) Protein Purification: Principles and Practice, Second Edition (Springer Verlag, N.Y.); and Handbook of Experimental Immunology, Volumes I-IV (D.M. Weir and C. C. Blackwell eds. 1986).

As used herein, the term "polypeptide" includes any peptide or protein comprising two or more amino acids joined to each other by peptide bonds or modified peptide bonds, i.e. peptide isosteres. This term refers both to short chains (peptides and oligopeptides) and to longer chains (proteins).

The polypeptide of the present invention may be in the form of a mature protein or may be a pre-, pro- or prepro- protein that can be activated by cleavage of the pre-, pro- or prepro-portion to produce an active mature polypeptide. In such polypeptides, the pre-, pro- or prepro- sequence may be a leader or secretory sequence or may be a sequence that is employed for purification of the mature polypeptide sequence.

The polypeptide of the first aspect of the invention may form part of a fusion protein. For example, it is often advantageous to include one or more additional amino acid sequences which may contain secretory or leader sequences, pro-sequences, sequences which aid in purification, or sequences that confer higher protein stability, for example during recombinant production. Alternatively or additionally, the mature polypeptide may be fused with another compound, such as a compound to increase the half-life of the polypeptide (for example, polyethylene glycol).

Polypeptides of the invention are useful on their own, as components of fusion proteins such as Fc fusion, and/or in combination with another agent. Preferably, the Fc fusion comprises a soluble polypeptide of the invention, i.e. a Fc:soluble receptor. A Fc:soluble receptor is a chimeric protein of the constant fragment (Fc) immunoglobulin G and the extracellular domain or fragment thereof of the receptor. It is known that Fc:soluble receptors are useful in the treatment of tumors (see for example Yingling et al. Nature Reviews 2004, Vol.3, pp.1011-1022). Preferably, the soluble polypeptide consists of the extracellular domain or a fragment thereof. Preferably, the soluble polypeptide consists of the cloned sequence (SEQ ID NO:21).

Preferably the agent is selected among:
1) TWIST, or
2) Small-molecule kinase inhibitors, or
3) Large-molecule TGF-beta signaling inhibitors, or
4) ERBB2/HER2 therapeutical agent such as an Erbb2 tyrosine kinase receptor inhibitor, or
5) Angiotensin-converting enzyme (ACE) inhibitor such as lisinopril, or
6) EGFR family tyrosine kinase receptor inhibitor such as gefitinib, or
7) Abl tyrosine kinase inhibitor such as imatinib, or
8) Immunomodulators.

Preferably, the small-molecule kinase inhibitor targets the receptor complex of TGF-β. Preferably, the receptor complex of TGF-β is TGF-β RI. Preferably, the small-molecule inhibitor TGF-β RI kinase inhibitor is selected among inhibitors based on a dihydropyrrolopyrazole scaffold such as LY550410 or LY580276 developed by Elli Lilly, inhibitors based on an imidazole scaffold such as SB-505124 developed by GlaxoSmithKline, inhibitors based on a pyrazolopyridine scaffold such as those developed by Elli Lilly, inhibitors based on a pyrazole scaffold such as those developed by GlaxoSmithKline, inhibitors based on an imidazopyridine scaffold such as those developed by Biogen, inhibitors based on a triazole scaffold such as those developed by Pfizer, inhibitors based on a pyridopyrimidine scaffold such as those developed by Johnson & Johnson, inhibitors based on an isothiazole scaffold such as those developed by Pfizer, a quinazoline-derived inhibitor such as SD-093, or the kinase inhibitor SD-208.

Preferably, the large-molecule TGF-beta signaling inhibitor is a soluble receptor, a neutralizing antibody or an antisense oligonucleotide.

Preferably, the soluble receptor is the Fc:soluble type II receptor antagonist SR2F. Bandyopadhyay *et al.* disclose the use of a recombinant soluble betaglycan (TGFbRIII) named sBG useful as a prostate cancer therapeutic agent after systemic administration in a xenograft model (Prostate 2005, Vol.63, Issue 1, pp.81-90). Liu et al. disclosed a soluble TGFβ type II receptor gene transfection able to inhibit fibrous airway obliteration in a rat model of bronchiolitis obliterans (Am. J. Respir. Crit. Care Med. 2002. Vol.165, pp.419-423),

Preferably, the neutralizing antibody is a recombinant human immunoglobilin G₄TGF-β₂ monoclonal antibody such as lerdelimumab, a monoclonal antibody that targets TGF-β1 such as metelimumab, or a pan-specific human anti- TGF-β antibody such as GC-1008.

Preferably, the antisense oligonucleotide targets the TGF-β ligand mRNA. Preferably, the antisense oligonucleotide is an antisense TGF-β2 vaccine (a TGF-β2 antisense-modified allogenic tumour-cell vaccine), AP-12009, NeuGene, an antisense oligonucleotide inhibitor as developed by NeXstar, or a TGF-β1-specific phosphorothioate antisense oligonucleotide such as AP-11014.

Preferably, the Erbb2 tyrosine kinase receptor inhibitor is Trastuzumab.

As such, TGF-beta antagonists are known and are used for the treatment of various diseases. Antisense Pharma GmbH developed AP-11014 for the treatment of non-small-cell lung, colorectal and prostate cancers, and AP-12009 for the treatment of gliomas, in particular high-grade gliomas, preferably anaplastic astrocytomas or glioblastomas. AVI BioPharma developed NeuGene for bone marrow transplantations. Biogen developed TGF-beta receptor inhibitors for the treatment of fibrosis. Cambridge Antibody Technology developed GC-1008 for the treatment of fibrosis, in particular of idiopathic pulmonary fibrosis, lerdelimumab for the treatment of for the prevention of post-operative scarring following glaucoma surgery, and metelimumab for the treatment of renal disease, fibrosis, scleroderma and diabetes, in particular of diabetic nephropathy. Eli Lilly developed TGF-beta inhibitors for the treatment of atherosclerosis, cancer and inflammation. NCI/NIH developed a TGF-beta antagonist for the treatment of cancer, in particular of metastatic cancer. NeXstar developed a TGF-beta antagonist for the treatment of an angiogenesis disorder. Scios developed a TGF-beta 1 antagonist for the treatment of chronic obstructive pulmonary disease, restrictive cardiomyopathy and cancer. Sumimoto Seika Chemicals developed a TGF-beta antagonist for the treatment of diabetic nephropathy. Telios developed anti-TGF-beta antibodies CAT for the treatment of fibrosis. NovaRx developed an antisense TGF-β2 vaccine for the treatment of gliomas, in particular malignant gliomas, e.g. gliosarcomas.

Preferably, the polypeptides of the present invention and/or the agent therapeutically modulate TGF-β signaling to affect metastasis without adversely affecting the tumour-suppressive nature of TGF-β. It is known that early epithelial cancers remain subject to the tumour-suppressive role of TGF-beta and retain a functional TGF-beta-mediated growth-inhibition pathway (Yingling *et al.* 2004). As pre-malignant lesions progress, TGF-β signalling mechanisms are altered and the role of TGF-β progressively changes from tumour suppressor to tumour promoter. TGF-β has a role in the conversion of early epithelial tumours to invasive, metastatic tumours by promotion of epithelial-to-mesenchymal transition (EMT). TGF-β acts to promote tumour progression and metastasis once the tumour has converted to a mesenchymal phenotype.

The polypeptides of the present invention, alone, as part of a fusion protein, and/or in combination with an agent, are preferably administered to cancers where TGF-β acts as a tumor promoter. Preferably, the polypeptides of the present invention, alone, as part of a fusion protein, and/or in combination with an agent, are administered to non-early epithelial cancers or non-primary lesions. Preferably, the non-epithelial cancers are invasive and/or metastatic cancers. Preferably, the polypeptides of the present invention, alone, as part of a fusion protein, and/or in combination with an agent, are administered to tumours that have converted to a mesenchymal phenotype.

The administration may be achieved via subconjunctival injections or intratumourally by convection-enhanced delivery or topical injection.

In a preferred embodiment, a polypeptide of the invention, that may comprise a sequence having at least 85% homology with an INSP215 polypeptide, is a fusion protein.

These fusion proteins can be obtained by cloning a polynucleotide encoding a polypeptide comprising a sequence having at least 85% homology with an INSP215 polypeptide in frame to the coding sequence for a heterologous protein sequence. Preferred fusion proteins include those described in the Examples herein, whose amino acid sequence is given in SEQ ID NO:19 and SEQ ID NO:21.

The term "heterologous", when used herein, is intended to designate any polypeptide other than a human INSP215 polypeptide. Examples of heterologous sequences, that can be comprised in the fusion proteins either at the N- or C-terminus, include: extracellular domains of membrane-bound protein, immunoglobulin constant regions (Fc regions), multimerization domains, domains of extracellular proteins, signal sequences, export sequences, and sequences allowing purification by affinity chromatography.

Many of these heterologous sequences are commercially available in expression plasmids since these sequences are commonly included in fusion proteins in order to provide additional properties without significantly impairing the specific biological activity of the protein fused to them (Terpe K, 2003, Appl Microbiol Biotechnol, 60:523-33). Examples of such additional properties are a longer lasting half-life in body fluids, the extracellular localization, or an easier purification procedure as allowed by the a stretch of Histidines forming the so-called "histidine tag" (Gentz et al. 1989, Proc Natl Acad Sci USA, 86:821-4) or by the "HA" tag, an epitope derived from the influenza hemagglutinin protein (Wilson et al. 1994, Cell, 37:767-78). If needed, the heterologous sequence can be eliminated by a proteolytic cleavage, for example by inserting a proteolytic cleavage site between the protein and the heterologous sequence, and exposing the purified fusion protein to the appropriate protease. These features are of particular importance for the fusion proteins since they facilitate their production and use in the preparation of pharmaceutical compositions. For example, the INSP215 polypeptide may be purified by means of a hexa-histidine peptide fused at the C-terminus of INSP215. When the fusion protein comprises an immunoglobulin region, the fusion may be direct, or via a short linker peptide which can be as short as 1 to 3 amino acid residues in length or longer, for example, 13 amino acid residues in length. Said linker may be a tripeptide of the sequence E-F-M (Glu-Phe-Met), for example, or a 13-amino acid linker sequence comprising Glu-Phe-Gly-Ala-Gly-Leu-Val-Leu-Gly-Gly-Gln-Phe-Met (SEQ ID NO:15) introduced between the sequence of the substances of the invention and the immunoglobulin sequence. The resulting fusion protein has improved properties, such as an extended residence time in body fluids (i.e. an increased half-life), increased specific activity, increased expression level, or the purification of the fusion protein is facilitated.

In a preferred embodiment, the protein is fused to the constant region of an Ig molecule. Preferably, it is fused to heavy chain regions, like the CH2 and CH3 domains of human IgG1, for example. Other isoforms of Ig molecules are also suitable for the generation of fusion proteins according to the present invention, such as isoforms IgG2 or IgG4, or other Ig classes, like IgM or IgA, for example. Fusion proteins may be monomeric or multimeric, hetero- or homomultimeric.

In a further preferred embodiment, the functional derivative comprises at least one moiety attached to one or more functional groups, which occur as one or more side chains on the amino acid residues. Preferably, the moiety is a polyethylene (PEG) moiety. PEGylation may be carried out by known methods, such as the ones described in WO99/55377, for example.

Polypeptides may contain amino acids other than the 20 gene-encoded amino acids, modified either by natural processes, such as by post-translational processing or by chemical modification techniques which are well known in the art. Among the known modifications which may commonly be present in polypeptides of the present invention are glycosylation, lipid attachment, sulphation, gamma-carboxylation, for instance of glutamic acid residues, hydroxylation and ADP-ribosylation. Other potential modifications include acetylation, acylation, amidation, covalent attachment of flavin, covalent attachment of a haeme moiety, covalent attachment of a nucleotide or nucleotide derivative, covalent attachment of a lipid derivative, covalent attachment of phosphatidylinositol, cross-linking, cyclization, disulphide bond formation, demethylation, formation of covalent cross-links, formation of cysteine, formation of pyroglutamate, formylation, GPI anchor formation, iodination, methylation, myristoylation, oxidation, proteolytic processing, phosphorylation, prenylation, racemization, selenoylation, transfer-RNA mediated addition of amino acids to proteins such as arginylation, and ubiquitination.

Modifications can occur anywhere in a polypeptide, including the peptide backbone, the amino acid side-chains and the amino or carboxyl termini. In fact, blockage of the amino or carboxyl terminus in a polypeptide, or both, by a covalent modification is common in naturally-occurring and synthetic polypeptides and such modifications may be present in polypeptides of the present invention.

The modifications that occur in a polypeptide often will be a function of how the polypeptide is made. For polypeptides that are made recombinantly, the nature and extent of the modifications in large part will be determined by the post-translational modification capacity of the particular host cell and the modification signals that are present in the amino acid sequence of the polypeptide in question. For instance, glycosylation patterns vary between different types of host cell.

The polypeptides of the present invention can be prepared in any suitable manner. Such polypeptides include, where the polypeptide is a naturally occurring polypeptide, isolated naturally-occurring polypeptides (for example purified from cell culture), recombinantly-produced polypeptides (including fusion proteins), synthetically-produced polypeptides and polypeptides that are produced by a combination of these methods. The term "isolated" does not denote the method by which the polypeptide is obtained or the level of purity of the preparation. Thus, such isolated species may be produced recombinantly, isolated directly from the cell or tissue of interest or produced synthetically based on the determined sequences.

The functionally-equivalent polypeptides of the first aspect of the invention may be polypeptides that are homologous to the INSP215 polypeptides. Two polypeptides are said to be "homologous", as the term is used herein, if the sequence of one of the polypeptides has a high enough degree of identity or similarity to the sequence of the other polypeptide. "Identity" indicates that at any particular position in the aligned sequences, the amino acid residue is identical between the sequences. "Similarity" indicates that, at any particular position in the aligned sequences, the amino acid residue is of a similar type between the sequences. Degrees of identity and similarity can be readily calculated (Computational Molecular Biology, Lesk, A.M., ed., Oxford University Press, New York, 1988; Biocomputing. Informatics and Genome Projects, Smith, D.W., ed., Academic Press, New York, 1993; Computer Analysis of Sequence Data, Part 1, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991).

Homologous polypeptides therefore include natural biological variants (for example, allelic variants or geographical variations within the species from which the polypeptides are derived) and mutants (such as mutants containing amino acid substitutions, insertions or deletions) of the INSP215 polypeptides. Such mutants may include polypeptides in which one or more of the amino acid residues are substituted with a conserved or non-conserved amino acid residue (preferably a conserved amino acid residue) and such substituted amino acid residue may or may not be one encoded by the genetic code. Typical such substitutions are among Ala, Val, Leu and Ile; among Ser and Thr; among the acidic residues Asp and Glu; among Asn and Gln; among the basic residues Lys and Arg; or among the aromatic residues Phe and Tyr. Particularly preferred are variants in which several, i.e. between 5 and 10, 1 and 5, 1 and 3, 1 and 2 or just 1 amino acids are substituted, deleted or added in any combination. Especially preferred are silent substitutions, additions and deletions, which do not alter the properties and activities of the protein. Also especially preferred in this regard are conservative substitutions. Such mutants also include polypeptides in which one or more of the amino acid residues includes a substituent group.

In accordance with the present invention, any substitution should be preferably a "conservative" or "safe" substitution, which is commonly defined a substitution introducing an amino acids having sufficiently similar chemical properties (e.g. a basic, positively charged amino acid should be replaced by another basic, positively charged amino acid), in order to preserve the structure and the biological function of the molecule.

The literature provide many models on which the selection of conservative amino acids substitutions can be performed on the basis of statistical and physico-chemical studies on the sequence and/or the structure of proteins (Rogov SI and Nekrasov AN, 2001). Protein design experiments have shown that the use of specific subsets of amino acids can produce foldable and active proteins, helping in the classification of amino acid "synonymous" substitutions which can be more easily accommodated in protein structure, and which can be used to detect functional and structural homologs and paralogs (Murphy LR *et al.,* 2000). The groups of synonymous amino acids and the groups of more preferred synonymous amino acids are shown in Table 1.

Specific, non-conservative mutations can be also introduced in the polypeptides of the invention with different purposes. Mutations reducing the affinity of the protein may increase its ability to be reused and recycled, potentially increasing its therapeutic potency (Robinson CR, 2002). Immunogenic epitopes eventually present in the polypeptides of the invention can be exploited for developing vaccines (Stevanovic S, 2002), or eliminated by modifying their sequence following known methods for selecting mutations for increasing protein stability, and correcting them (van den Burg B and Eijsink V, 2002; WO 02/05146, WO 00/34317, WO 98/52976).

Preferred alternative, synonymous groups for amino acids derivatives included in peptide mimetics are those defined in Table 2. A non-exhaustive list of amino acid derivatives also include aminoisobutyric acid (Aib), hydroxyproline (Hyp), 1,2,3,4-tetrahydroisoquinoline-3-COOH, indoline-2carboxylic acid, 4-difluoro-proline, L- thiazolidine-4-carboxylic acid, L-homoproline, 3,4-dehydro-proline, 3,4-dihydroxy-phenylalanine, cyclohexyl-glycine, and phenylglycine.

By "amino acid derivative" is intended an amino acid or amino acid-like chemical entity other than one of the 20 genetically encoded naturally occurring amino acids. In particular, the amino acid derivative may contain substituted or non-substituted, linear, branched, or cyclic alkyl moieties, and may include one or more heteroatoms. The amino acid derivatives can be made de novo or obtained from commercial sources (Calbiochem-Novabiochem AG, Switzerland; Bachem, USA).

Various methodologies for incorporating unnatural amino acids derivatives into proteins, using both *in vitro* and *in vivo* translation systems, to probe and/or improve protein structure and function are disclosed in the literature (Dougherty DA, 2000). Techniques for the synthesis and the development of peptide mimetics, as well as non-peptide mimetics, are also well known in the art (Golebiowski A *et al.,* 2001; Hruby VJ and Balse PM, 2000; Sawyer TK, in "Structure Based Drug Design", edited by Veerapandian P, Marcel Dekker Inc., pg. 557-663, 1997).

Such mutants also include polypeptides in which one or more of the amino acid residues includes a substituent group.

Typically, greater than 30% identity between two polypeptides is considered to be an indication of functional equivalence. Preferably, functionally equivalent polypeptides of the first aspect of the invention have a degree of sequence identity with the INSP215 polypeptides, or with active fragments thereof, of greater than 80%. More preferred polypeptides have degrees of identity of greater than 85%, 90%, 95%, 98% or 99%, respectively.

The functionally-equivalent polypeptides of the first aspect of the invention may also be polypeptides which have been identified using one or more techniques of structural alignment. For example, the Inpharmatica Genome Threader technology that forms one aspect of the search tools used to generate the Biopendium search database may be used (see co-pending PCT application PCT/GB01/01105, published as WO 01/69507) to identify polypeptides of presently-unknown function which, while having low sequence identity as compared to the INSP215 polypeptides, are predicted to be ZP/TGR3 subdomain-containing polypeptides, by virtue of sharing significant structural homology with the INSP215 polypeptide sequences. By "significant structural homology" is meant that the Inpharmatica Genome Threader predicts two proteins to share structural homology with a certainty of 10% and above.

The polypeptides of the first aspect of the invention also include fragments of the INSP215 polypeptides and fragments of the functional equivalents of the INSP215 polypeptides, provided that those fragments contain a ZP/TGR3 subdomain, or have an antigenic determinant in common with the INSP215 polypeptides.

As used herein, the term "fragment" refers to a polypeptide having an amino acid sequence that is the same as part, but not all, of the amino acid sequence of the INSP215 polypeptides or one of their functional equivalents. The fragments should comprise at least n consecutive amino acids from the sequence and, depending on the particular sequence, n preferably is 7 or more (for example, 8, 10, 12, 14, 16, 18, 20 or more). Small fragments may form an antigenic determinant.

Nucleic acids according to the invention are preferably 10-2000 nucleotides in length, preferably 100-1750 nucleotides, preferably 500-1500, preferably 600-1200, preferably 750-1000 nucleotides in length. Polypeptides according to the invention are preferably 10-700 amino acids in length, preferably 50-600, preferably 100-500, preferably 200-400, preferably 300-375 amino acids in length.

Fragments of the INSP215 exon polypeptides and the INSP215 polypeptides may consist of combinations of 2, 3, 4, 5 or 6 neighbouring exon sequences in the INSP215 polypeptide, respectively. Preferably, exons are combined in order to match the domains in INSP215 identified herein.

Such fragments may be "free-standing", i.e. not part of or fused to other amino acids or polypeptides, or they may be comprised within a larger polypeptide of which they form a part or region. When comprised within a larger polypeptide, the fragment of the invention most preferably forms a single continuous region. For instance, certain preferred embodiments relate to a fragment having a pre - and/or pro- polypeptide region fused to the amino terminus of the fragment and/or an additional region fused to the carboxyl terminus of the fragment. However, several fragments may be comprised within a single larger polypeptide.

The polypeptides of the present invention or their immunogenic fragments (comprising at least one antigenic determinant) can be used to generate ligands, such as polyclonal or monoclonal antibodies, that are immunospecific for the polypeptides. Such antibodies may be employed to isolate or to identify clones expressing the polypeptides of the invention or to purify the polypeptides by affinity chromatography. The antibodies may also be employed as diagnostic or therapeutic aids, amongst other applications, as will be apparent to the skilled reader.

The term "immunospecific" means that the antibodies have substantially greater affinity for the polypeptides of the invention than their affinity for other related polypeptides in the prior art. As used herein, the term "antibody" refers to intact molecules as well as to fragments thereof, such as Fab, F(ab')2 and Fv, which are capable of binding to the antigenic determinant in question. Such antibodies thus bind to the polypeptides of the first aspect of the invention.

By "substantially greater affinity" we mean that there is a measurable increase in the affinity for a polypeptide of the invention as compared with the affinity for known ZP/TGR3 domain-containing polypeptides.

Preferably, the affinity is at least 1.5-fold, 2-fold, 5-fold 10-fold, 100-fold, 10³-fold, 10⁴-fold, 10⁵-fold or 10⁶-fold greater for a polypeptide of the invention than for known ZP/TGR3 domain-containing polypeptides.

Preferably, there is a measurable increase in the affinity for a polypeptide of the invention as compared with known ZP domain containing proteins.

If polyclonal antibodies are desired, a selected mammal, such as a mouse, rabbit, goat or horse, may be immunised with a polypeptide of the first aspect of the invention. The polypeptide used to immunise the animal can be derived by recombinant DNA technology or can be synthesized chemically. If desired, the polypeptide can be conjugated to a carrier protein. Commonly used carriers to which the polypeptides may be chemically coupled include bovine serum albumin, thyroglobulin and keyhole limpet haemocyanin. The coupled polypeptide is then used to immunise the animal. Serum from the immunised animal is collected and treated according to known procedures, for example by immunoaffinity chromatography.

Monoclonal antibodies to the polypeptides of the first aspect of the invention can also be readily produced by one skilled in the art. The general methodology for making monoclonal antibodies using hybridoma technology is well known (see, for example, Kohler, G. and Milstein, C., Nature 256: 495-497 (1975); Kozbor et al., Immunology Today 4: 72 (1983); Cole et al., 77-96 in Monoclonal Antibodies and Cancer Therapy, Alan R. Liss, Inc. (1985).

Panels of monoclonal antibodies produced against the polypeptides of the first aspect of the invention can be screened for various properties, i.e., for isotype, epitope, affinity, etc. Monoclonal antibodies are particularly useful in purification of the individual polypeptides against which they are directed. Alternatively, genes encoding the monoclonal antibodies of interest may be isolated from hybridomas, for instance by PCR techniques known in the art, and cloned and expressed in appropriate vectors.

Chimeric antibodies, in which non-human variable regions are joined or fused to human constant regions (see, for example, Liu et al., Proc. Natl. Acad. Sci. USA, 84, 3439 (1987)), may also be of use.

The antibody may be modified to make it less immunogenic in an individual, for example by humanisation (see Jones et al., Nature, 321, 522 (1986); Verhoeyen et al., Science, 239, 1534 (1988); Kabat et al., J. Immunol., 147, 1709 (1991); Queen et al., Proc. Natl Acad. Sci. USA, 86, 10029 (1989); Gorman et al., Proc. Natl Acad. Sci. USA, 88, 34181 (1991); and Hodgson et al., Bio/Technology, 9, 421 (1991)). The term "humanised antibody", as used herein, refers to antibody molecules in which the CDR amino acids and selected other amino acids in the variable domains of the heavy and/or light chains of a non-human donor antibody have been substituted in place of the equivalent amino acids in a human antibody. The humanised antibody thus closely resembles a human antibody but has the binding ability of the donor antibody.

In a further alternative, the antibody may be a "bispecific" antibody, that is an antibody having two different antigen binding domains, each domain being directed against a different epitope.

Phage display technology may be utilised to select genes which encode antibodies with binding activities towards the polypeptides of the invention either from repertoires of PCR amplified V-genes of lymphocytes from humans screened for possessing the relevant antibodies, or from naive libraries (McCafferty, J. et al., (1990), Nature 348, 552-554; Marks, J. et al., (1992) Biotechnology 10, 779-783). The affinity of these antibodies can also be improved by chain shuffling (Clackson, T. et al., (1991) Nature 352, 624-628).

Antibodies generated by the above techniques, whether polyclonal or monoclonal, have additional utility in that they may be employed as reagents in immunoassays, radioimmunoassays (RIA) or enzyme-linked immunosorbent assays (ELISA). In these applications, the antibodies can be labelled with an analytically-detectable reagent such as a radioisotope, a fluorescent molecule or an enzyme.

Preferred nucleic acid molecules of the second and third aspects of the disclosure are those which encode the polypeptide sequence recited in SEQ ID NO:2, SEQ ID NO:4, SEQ ID NO:6, SEQ ID NO:8, SEQ ID NO:10, SEQ ID NO: 12, SEQ ID NO:14, SEQ ID NO:19 or SEQ ID NO:21 and functionally equivalent polypeptides. These nucleic acid molecules may be used in the methods and applications described herein. The nucleic acid molecules of the invention preferably comprise at least n consecutive nucleotides from the sequences disclosed herein where, depending on the particular sequence, n is 10 or more (for example, 12, 14, 15, 18, 20, 25, 30, 35, 40 or more).

The nucleic acid molecules of the invention also include sequences that are complementary to nucleic acid molecules described above (for example, for antisense or probing purposes).

Nucleic acid molecules of the present invention may be in the form of RNA, such as mRNA, or in the form of DNA, including, for instance cDNA, synthetic DNA or genomic DNA. Such nucleic acid molecules may be obtained by cloning, by chemical synthetic techniques or by a combination thereof. The nucleic acid molecules can be prepared, for example, by chemical synthesis using techniques such as solid phase phosphoramidite chemical synthesis, from genomic or cDNA libraries or by separation from an organism. RNA molecules may generally be generated by the *in vitro* or *in vivo* transcription of DNA sequences.

The nucleic acid molecules may be double-stranded or single-stranded. Single-stranded DNA may be the coding strand, also known as the sense strand, or it may be the non-coding strand, also referred to as the anti-sense strand.

The term "nucleic acid molecule" also includes analogues of DNA and RNA, such as those containing modified backbones, and peptide nucleic acids (PNA). The term "PNA", as used herein, refers to an antisense molecule or an anti-gene agent which comprises an oligonucleotide of at least five nucleotides in length linked to a peptide backbone of amino acid residues, which preferably ends in lysine. The terminal lysine confers solubility to the composition. PNAs may be pegylated to extend their lifespan in a cell, where they preferentially bind complementary single stranded DNA and RNA and stop transcript elongation (Nielsen, P.E. et al. (1993) Anticancer Drug Des. 8:53-63).

A nucleic acid molecule which encodes the polypeptide of SEQ ID NO:2 may be identical to the coding sequence of the nucleic acid molecule shown in SEQ ID NO:1. A nucleic acid molecule which encodes the polypeptide of SEQ ID NO:4 may be identical to the coding sequence of the nucleic acid molecule shown in SEQ ID NO:3. A nucleic acid molecule which encodes the polypeptide of SEQ ID NO:6 may be identical to the coding sequence of the nucleic acid molecule shown in SEQ ID NO:5. A nucleic acid molecule which encodes the polypeptide of SEQ ID NO:8 may be identical to the coding sequence of the nucleic acid molecule shown in SEQ ID NO:7. A nucleic acid molecule which encodes the polypeptide of SEQ ID NO:10 may be identical to the coding sequence of the nucleic acid molecule shown in SEQ ID NO:9. A nucleic acid molecule which encodes the polypeptide of SEQ ID NO:12 may be identical to the coding sequence of the nucleic acid molecule shown in SEQ ID NO:11. A nucleic acid molecule which encodes the polypeptide of SEQ ID NO:14 may be identical to the coding sequence of the nucleic acid molecule shown in SEQ ID NO:13. A nucleic acid molecule which encodes the polypeptide of SEQ ID NO:19 may be identical to the coding sequence of the nucleic acid molecule shown in SEQ ID NO:18. A nucleic acid molecule which encodes the polypeptide of SEQ ID NO:21 may be identical to the coding sequence of the nucleic acid molecule shown in SEQ ID NO:20.

These molecules also may have a different sequence which, as a result of the degeneracy of the genetic code, encodes a polypeptide of SEQ ID NO:2, SEQ ID NO:4, SEQ ID NO:6, SEQ ID NO:8, SEQ ID NO:10, SEQ ID NO:12, SEQ ID NO:14, SEQ ID NO:19, or SEQ ID NO:21. Such nucleic acid molecules that encode the polypeptide of SEQ ID NO:2, SEQ ID NO:4, SEQ ID NO:6, SEQ ID NO:8, SEQ ID NO:10, SEQ ID NO:12, SEQ ID NO:14, SEQ ID NO:19 or SEQ ID NO:21 may include, but are not limited to, the coding sequence for the mature polypeptide by itself, the coding sequence for the mature polypeptide and additional coding sequences, such as those encoding a leader or secretory sequence, such as a pro-, pre- or prepro- polypeptide sequence; the coding sequence of the mature polypeptide, with or without the aforementioned additional coding sequences, together with further additional, non-coding sequences, including non-coding 5' and 3' sequences, such as the transcribed, non translated sequences that play a role in transcription (including termination signals), ribosome binding and mRNA stability. The nucleic acid molecules may also include additional sequences which encode additional amino acids, such as those which provide additional functionalities.

The nucleic acid molecules of the second and third aspects of the disclosure may also encode the fragments or the functional equivalents of the polypeptides and fragments of the first aspect of the invention. Such a nucleic acid molecule may be a naturally-occurring variant such as a naturally-occurring allelic variant, or the molecule may be a variant that is not known to occur naturally. Such non-naturally occurring variants of the nucleic acid molecule may be made by mutagenesis techniques, including those applied to nucleic acid molecules, cells or organisms.

Among variants in this regard are variants that differ from the aforementioned nucleic acid molecules by nucleotide substitutions, deletions or insertions. The substitutions, deletions or insertions may involve one or more nucleotides. The variants may be altered in coding or non-coding regions or both. Alterations in the coding regions may produce conservative or non-conservative amino acid substitutions, deletions or insertions.

The nucleic acid molecules of the invention can also be engineered, using methods generally known in the art, for a variety of reasons, including modifying the cloning, processing, and/or expression of the gene product (the polypeptide). DNA shuffling by random fragmentation and PCR reassembly of gene fragments and synthetic oligonucleotides are included as techniques which may be used to engineer the nucleotide sequences. Site-directed mutagenesis may be used to insert new restriction sites, alter glycosylation patterns, change codon preference, produce splice variants, introduce mutations and so forth.

Nucleic acid molecules which encode a polypeptide of the first aspect of the invention may be ligated to a heterologous sequence so that the combined nucleic acid molecule encodes a fusion protein. Such combined nucleic acid molecules are included within the second or third aspects of the invention. For example, to screen peptide libraries for inhibitors of the activity of the polypeptide, it may be useful to express, using such a combined nucleic acid molecule, a fusion protein that can be recognised by a commercially-available antibody. A fusion protein may also be engineered to contain a cleavage site located between the sequence of the polypeptide of the invention and the sequence of a heterologous protein so that the polypeptide may be cleaved and purified away from the heterologous protein.

The nucleic acid molecules of the invention also include antisense molecules that are partially complementary to nucleic acid molecules encoding polypeptides of the present invention and that therefore hybridize to the encoding nucleic acid molecules (hybridization). Such antisense molecules, such as oligonucleotides, can be designed to recognise, specifically bind to and prevent transcription of a target nucleic acid encoding a polypeptide of the invention, as will be known by those of ordinary skill in the art (see, for example, Cohen, J.S., Trends in Pharm. Sci., 10, 435 (1989), Okano, J. Neurochem. 56, 560 (1991); O'Connor, J. Neurochem 56, 560 (1991); Lee et al., Nucleic Acids Res 6, 3073 (1979); Cooney et al., Science 241, 456 (1988); Dervan et al., Science 251, 1360 (1991).

The term "hybridization" as used here refers to the association of two nucleic acid molecules with one another by hydrogen bonding. Typically, one molecule will be fixed to a solid support and the other will be free in solution. Then, the two molecules may be placed in contact with one another under conditions that favour hydrogen bonding. Factors that affect this bonding include: the type and volume of solvent; reaction temperature; time of hybridization; agitation; agents to block the non-specific attachment of the liquid phase molecule to the solid support (Denhardt's reagent or BLOTTO); the concentration of the molecules; use of compounds to increase the rate of association of molecules (dextran sulphate or polyethylene glycol); and the stringency of the washing conditions following hybridization (see Sambrook *et al*. [*supra*]).

The inhibition of hybridization of a completely complementary molecule to a target molecule may be examined using a hybridization assay, as known in the art (see, for example, Sambrook *et al.* [*supra*]). A substantially homologous molecule will then compete for and inhibit the binding of a completely homologous molecule to the target molecule under various conditions of stringency, as taught in Wahl, G.M. and S.L. Berger (1987; Methods Enzymol. 152:399-407) and Kimmel, A.R (1987; Methods Enzymol. 152:507-511).

"Stringency" refers to conditions in a hybridization reaction that favour the association of very similar molecules over association of molecules that differ. High stringency hybridisation conditions are defined as overnight incubation at 42°C in a solution comprising 50% formamide, 5XSSC (150mM NaCl, 15mM trisodium citrate), 50mM sodium phosphate (pH7.6), 5x Denhardts solution, 10% dextran sulphate, and 20 microgram/ml denatured, sheared salmon sperm DNA, followed by washing the filters in 0.1X SSC at approximately 65°C. Low stringency conditions involve the hybridisation reaction being carried out at 35°C (see Sambrook *et al.* [*supra*]). Preferably, the conditions used for hybridization are those of high stringency.

Preferred embodiments of this aspect of the invention are nucleic acid molecules that are at least 70% identical over their entire length to a nucleic acid molecule encoding the INSP215 polypeptides (SEQ ID NO:2, SEQ ID NO:4, SEQ ID NO:6, SEQ ID NO:8, SEQ ID NO:10, SEQ ID NO:12, SEQ ID NO:14, SEQ ID NO:19, or SEQ ID NO:21) and nucleic acid molecules that are substantially complementary to such nucleic acid molecules. Preferably, a nucleic acid molecule according to this aspect of the invention comprises a region that is at least 80% identical over its entire length to such coding sequences, or is a nucleic acid molecule that is complementary thereto. In this regard, nucleic acid molecules at least 90%, preferably at least 95%, more preferably at least 98%, 99% or more identical over their entire length to the same are particularly preferred. Preferred embodiments in this respect are nucleic acid molecules that encode polypeptides which retain substantially the same biological function or activity as the INSP215 polypeptides.

Also described herein is a process for detecting a nucleic acid molecule of the invention, comprising the steps of: (a) contacting a nucleic probe according to the invention with a biological sample under hybridizing conditions to form duplexes; and (b) detecting any such duplexes that are formed.

As discussed additionally below in connection with assays that may be utilised according to the invention, a nucleic acid molecule as described above may be used as a hybridization probe for RNA, cDNA or genomic DNA, in order to isolate full-length cDNAs and genomic clones encoding the INSP215 polypeptides and to isolate cDNA and genomic clones of homologous or orthologous genes that have a high sequence similarity to the gene encoding this polypeptide.

In this regard, the following techniques, among others known in the art, may be utilised and are discussed below for purposes of illustration. Methods for DNA sequencing and analysis are well known and are generally available in the art and may, indeed, be used to practice many of the embodiments of the invention discussed herein. Such methods may employ such enzymes as the Klenow fragment of DNA polymerase I, Sequenase (US Biochemical Corp, Cleveland, OH), Taq polymerase (Perkin Elmer), thermostable T7 polymerase (Amersham, Chicago, IL), or combinations of polymerases and proof-reading exonucleases such as those found in the ELONGASE Amplification System marketed by Gibco/BRL (Gaithersburg, MD). Preferably, the sequencing process may be automated using machines such as the Hamilton Micro Lab 2200 (Hamilton, Reno, NV), the Peltier Thermal Cycler (PTC200; MJ Research, Watertown, MA) and the ABI Catalyst and 373 and 377 DNA Sequencers (Perkin Elmer).

One method for isolating a nucleic acid molecule encoding a polypeptide with an equivalent function to that of the INSP215 polypeptides is to probe a genomic or cDNA library with a natural or artificially-designed probe using standard procedures that are recognised in the art (see, for example, "Current Protocols in Molecular Biology", Ausubel et al. (eds). Greene Publishing Association and John Wiley Interscience, New York, 1989,1992). Probes comprising at least 15, preferably at least 30, and more preferably at least 50, contiguous bases that correspond to, or are complementary to, nucleic acid sequences from the appropriate encoding gene (SEQ ID NO:1, SEQ ID NO:3, SEQ ID NO:5, SEQ ID NO:7, SEQ ID NO:9, SEQ ID NO:11, SEQ ID NO:13, SEQ ID NO:18, or SEQ ID NO:20), are particularly useful probes. Such probes may be labelled with an analytically-detectable reagent to facilitate their identification. Useful reagents include, but are not limited to, radioisotopes, fluorescent dyes and enzymes that are capable of catalysing the formation of a detectable product. Using these probes, the ordinarily skilled artisan will be capable of isolating complementary copies of genomic DNA, cDNA or RNA polynucleotides encoding proteins of interest from human, mammalian or other animal sources and screening such sources for related sequences, for example, for additional members of the family, type and/or subtype.

In many cases, isolated cDNA sequences will be incomplete, in that the region encoding the polypeptide will be cut short, normally at the 5' end. Several methods are available to obtain full length cDNAs, or to extend short cDNAs. Such sequences may be extended utilising a partial nucleotide sequence and employing various methods known in the art to detect upstream sequences such as promoters and regulatory elements. For example, one method which may be employed is based on the method of Rapid Amplification of cDNA Ends (RACE; see, for example, Frohman et al., PNAS USA 85, 8998-9002, 1988). Recent modifications of this technique, exemplified by the Marathon^{™} technology (Clontech Laboratories Inc.), for example, have significantly simplified the search for longer cDNAs. A slightly different technique, termed "restriction-site" PCR, uses universal primers to retrieve unknown nucleic acid sequence adjacent a known locus (Sarkar, G. (1993) PCR Methods Applic. 2:318-322). Inverse PCR may also be used to amplify or to extend sequences using divergent primers based on a known region (Triglia, T. et αl. (1988) Nucleic Acids Res. 16:8186). Another method which may be used is capture PCR which involves PCR amplification of DNA fragments adjacent a known sequence in human and yeast artificial chromosome DNA (Lagerstrom, M. et al. (1991) PCR Methods Applic., 1, 111-119). Another method which may be used to retrieve unknown sequences is that of Parker, J.D. et al. (1991); Nucleic Acids Res. 19:3055-3060). Additionally, one may use PCR, nested primers, and PromoterFinder^{™} libraries to walk genomic DNA (Clontech, Palo Alto, CA). This process avoids the need to screen libraries and is useful in finding intron/exon junctions.

When screening for full-length cDNAs, it is preferable to use libraries that have been size-selected to include larger cDNAs. Also, random-primed libraries are preferable, in that they will contain more sequences that contain the 5' regions of genes. Use of a randomly primed library may be especially preferable for situations in which an oligo d(T) library does not yield a full-length cDNA. Genomic libraries may be useful for extension of sequence into 5' non-transcribed regulatory regions.

Also described herein, the nucleic acid molecules of the present invention may be used for chromosome localisation. In this technique, a nucleic acid molecule is specifically targeted to, and can hybridize with, a particular location on an individual human chromosome. The mapping of relevant sequences to chromosomes according to the present invention is an important step in the confirmatory correlation of those sequences with the gene-associated disease. Once a sequence has been mapped to a precise chromosomal location, the physical position of the sequence on the chromosome can be correlated with genetic map data. Such data are found in, for example, V. McKusick, Mendelian Inheritance in Man (available on-line through Johns Hopkins University Welch Medical Library). The relationships between genes and diseases that have been mapped to the same chromosomal region are then identified through linkage analysis (coinheritance of physically adjacent genes). This provides valuable information to investigators searching for disease genes using positional cloning or other gene discovery techniques. Once the disease or syndrome has been crudely localised by genetic linkage to a particular genomic region, any sequences mapping to that area may represent associated or regulatory genes for further investigation. The nucleic acid molecule may also be used to detect differences in the chromosomal location due to translocation, inversion, etc. among normal, carrier, or affected individuals.

The nucleic acid molecules of the present invention are also valuable for tissue localisation. Such techniques allow the determination of expression patterns of the polypeptide in tissues by detection of the mRNAs that encode them. These techniques include *in situ* hybridization techniques and nucleotide amplification techniques, such as PCR. Results from these studies provide an indication of the normal functions of the polypeptide in the organism. In addition, comparative studies of the normal expression pattern of mRNAs with that of mRNAs encoded by a mutant gene provide valuable insights into the role of mutant polypeptides in disease. Such inappropriate expression may be of a temporal, spatial or quantitative nature.

Gene silencing approaches may also be undertaken to down-regulate endogenous expression of a gene encoding a polypeptide of the invention. RNA interference (RNAi) (Elbashir, SM et al., Nature 2001, 411, 494-498) is one method of sequence specific post-transcriptional gene silencing that may be employed. Short dsRNA oligonucleotides are synthesised *in vitro* and introduced into a cell. The sequence specific binding of these dsRNA oligonucleotides triggers the degradation of target mRNA, reducing or ablating target protein expression.

Efficacy of the gene silencing approaches assessed above may be assessed through the measurement of polypeptide expression (for example, by Western blotting), and at the RNA level using TaqMan-based methodologies.

The vectors of the present invention comprise nucleic acid molecules of the invention and may be cloning or expression vectors. The host cells of the invention, which may be transformed, transfected or transduced with the vectors of the invention may be prokaryotic or eukaryotic.

The polypeptides of the invention may be prepared in recombinant form by expression of their encoding nucleic acid molecules in vectors contained within a host cell. Such expression methods are well known to those of skill in the art and many are described in detail by Sambrook *et al.* (*supra*) and Fernandez & Hoeffler (1998, eds. "Gene expression systems. Using nature for the art of expression". Academic Press, San Diego, London, Boston, New York, Sydney, Tokyo, Toronto).

Generally, any system or vector that is suitable to maintain, propagate or express nucleic acid molecules to produce a polypeptide in the required host may be used. The appropriate nucleotide sequence may be inserted into an expression system by any of a variety of well-known and routine techniques, such as, for example, those described in Sambrook *et al.,* (*supra*). Generally, the encoding gene can be placed under the control of a control element such as a promoter, ribosome binding site (for bacterial expression) and, optionally, an operator, so that the DNA sequence encoding the desired polypeptide is transcribed into RNA in the transformed host cell.

Examples of suitable expression systems include, for example, chromosomal, episomal and virus-derived systems, including, for example, vectors derived from: bacterial plasmids, bacteriophage, transposons, yeast episomes, insertion elements, yeast chromosomal elements, viruses such as baculoviruses, papova viruses such as SV40, vaccinia viruses, adenoviruses, fowl pox viruses, pseudorabies viruses and retroviruses, or combinations thereof, such as those derived from plasmid and bacteriophage genetic elements, including cosmids and phagemids. Human artificial chromosomes (HACs) may also be employed to deliver larger fragments of DNA than can be contained and expressed in a plasmid.

The pDONR221-INSP215-6HIS, pDONR221-INSP215-EC-6HIS, pDEST12.2_INSP215-EC-6HIS, pEAK12d_ INSP215-EC-6HIS, pENTR_INSP215-6HIS, pEAK12d_INSP215-6HIS, and pDEST12.2_INSP215-6HIS vectors are preferred examples of suitable vectors for use in accordance with this invention.

Particularly suitable expression systems include microorganisms such as bacteria transformed with recombinant bacteriophage, plasmid or cosmid DNA expression vectors; yeast transformed with yeast expression vectors; insect cell systems infected with virus expression vectors (for example, baculovirus); plant cell systems transformed with virus expression vectors (for example, cauliflower mosaic virus, CaMV; tobacco mosaic virus, TMV) or with bacterial expression vectors (for example, Ti or pBR322 plasmids); or animal cell systems. Cell-free translation systems can also be employed to produce the polypeptides of the invention.

Introduction of nucleic acid molecules encoding a polypeptide of the present invention into host cells can be effected by methods described in many standard laboratory manuals, such as Davis et al., Basic Methods in Molecular Biology (1986) and Sambrook *et al*., (*supra*). Particularly suitable methods include calcium phosphate transfection, DEAE-dextran mediated transfection, transvection, microinjection, cationic lipid-mediated transfection, electroporation, transduction, scrape loading, ballistic introduction or infection (see Sambrook *et al.,* 1989 [*supra*]; Ausubel *et al.,* 1991 [*supra*]; Spector, Goldman & Leinwald, 1998). In eukaryotic cells, expression systems may either be transient (for example, episomal) or permanent (chromosomal integration) according to the needs of the system.

The encoding nucleic acid molecule may or may not include a sequence encoding a control sequence, such as a signal peptide or leader sequence, as desired, for example, for secretion of the translated polypeptide into the lumen of the endoplasmic reticulum, into the periplasmic space or into the extracellular environment. These signals may be endogenous to the polypeptide or they may be heterologous signals. Leader sequences can be removed by the bacterial host in post-translational processing.

In addition to control sequences, it may be desirable to add regulatory sequences that allow for regulation of the expression of the polypeptide relative to the growth of the host cell. Examples of regulatory sequences are those which cause the expression of a gene to be increased or decreased in response to a chemical or physical stimulus, including the presence of a regulatory compound or to various temperature or metabolic conditions. Regulatory sequences are those non-translated regions of the vector, such as enhancers, promoters and 5' and 3' untranslated regions. These interact with host cellular proteins to carry out transcription and translation. Such regulatory sequences may vary in their strength and specificity. Depending on the vector system and host utilised, any number of suitable transcription and translation elements, including constitutive and inducible promoters, may be used. For example, when cloning in bacterial systems, inducible promoters such as the hybrid lacZ promoter of the Bluescript phagemid (Stratagene, LaJolla, CA) or pSportl™ plasmid (Gibco BRL) and the like may be used. The baculovirus polyhedrin promoter may be used in insect cells. Promoters or enhancers derived from the genomes of plant cells (for example, heat shock, RUBISCO and storage protein genes) or from plant viruses (for example, viral promoters or leader sequences) may be cloned into the vector. In mammalian cell systems, promoters from mammalian genes or from mammalian viruses are preferable. If it is necessary to generate a cell line that contains multiple copies of the sequence, vectors based on SV40 or EBV may be used with an appropriate selectable marker.

An expression vector is constructed so that the particular nucleic acid coding sequence is located in the vector with the appropriate regulatory sequences, the positioning and orientation of the coding sequence with respect to the regulatory sequences being such that the coding sequence is transcribed under the "control" of the regulatory sequences, i.e., RNA polymerase which binds to the DNA molecule at the control sequences transcribes the coding sequence. In some cases it may be necessary to modify the sequence so that it may be attached to the control sequences with the appropriate orientation; i.e., to maintain the reading frame.

The control sequences and other regulatory sequences may be ligated to the nucleic acid coding sequence prior to insertion into a vector. Alternatively, the coding sequence can be cloned directly into an expression vector that already contains the control sequences and an appropriate restriction site.

For long-term, high-yield production of a recombinant polypeptide, stable expression is preferred. For example, cell lines which stably express the polypeptide of interest may be transformed using expression vectors which may contain viral origins of replication and/or endogenous expression elements and a selectable marker gene on the same or on a separate vector. Following the introduction of the vector, cells may be allowed to grow for 1-2 days in an enriched media before they are switched to selective media. The purpose of the selectable marker is to confer resistance to selection, and its presence allows growth and recovery of cells that successfully express the introduced sequences. Resistant clones of stably transformed cells may be proliferated using tissue culture techniques appropriate to the cell type.

Mammalian cell lines available as hosts for expression are known in the art and include many immortalised cell lines available from the American Type Culture Collection (ATCC) including, but not limited to, Chinese hamster ovary (CHO), HeLa, baby hamster kidney (BHK), monkey kidney (COS), C127,3T3, BHK, HEK 293, Bowes melanoma and human hepatocellular carcinoma (for example Hep G2) cells and a number of other cell lines.

In the baculovirus system, the materials for baculovirus/insect cell expression systems are commercially available in kit form from, *inter alia,* Invitrogen, San Diego CA (the "MaxBac" kit). These techniques are generally known to those skilled in the art and are described fully in Summers and Smith, Texas Agricultural Experiment Station Bulletin No. 1555 (1987). Particularly suitable host cells for use in this system include insect cells such as Drosophila S2 and Spodoptera Sf9 cells.

There are many plant cell culture and whole plant genetic expression systems known in the art. Examples of suitable plant cellular genetic expression systems include those described in US 5,693,506; US 5,659,122; and US 5,608,143. Additional examples of genetic expression in plant cell culture has been described by Zenk, Phytochemistry 30, 3861-3863 (1991).

In particular, all plants from which protoplasts can be isolated and cultured to give whole regenerated plants can be utilised, so that whole plants are recovered which contain the transferred gene. Practically all plants can be regenerated from cultured cells or tissues, including but not limited to all major species of sugar cane, sugar beet, cotton, fruit and other trees, legumes and vegetables.

Examples of particularly preferred bacterial host cells include *streptococci, staphylococci, E. coli, Streptomyces* and *Bacillus subtilis* cells.

Examples of particularly suitable host cells for fungal expression include yeast cells (for example, *S. cerevisiae*) and *Aspergillus* cells.

Any number of selection systems are known in the art that may be used to recover transformed cell lines. Examples include the herpes simplex virus thymidine kinase (Wigler, M. et al. (1977) Cell 11:223-32) and adenine phosphoribosyltransferase (Lowy, I. et al. (1980) Cell 22:817-23) genes that can be employed in tk- or aprt± cells, respectively.

Also, antimetabolite, antibiotic or herbicide resistance can be used as the basis for selection; for example, dihydrofolate reductase (DHFR) that confers resistance to methotrexate (Wigler, M. et al. (1980) Proc. Natl. Acad. Sci. 77:3567-70); npt, which confers resistance to the aminoglycosides neomycin and G-418 (Colbere-Garapin, F. et al. (1981) J. Mol. Biol. 150:1-14) and als or pat, which confer resistance to chlorsulfuron and phosphinotricin acetyltransferase, respectively. Additional selectable genes have been described, examples of which will be clear to those of skill in the art.

Although the presence or absence of marker gene expression suggests that the gene of interest is also present, its presence and expression may need to be confirmed. For example, if the relevant sequence is inserted within a marker gene sequence, transformed cells containing the appropriate sequences can be identified by the absence of marker gene function. Alternatively, a marker gene can be placed in tandem with a sequence encoding a polypeptide of the invention under the control of a single promoter. Expression of the marker gene in response to induction or selection usually indicates expression of the tandem gene as well.

Alternatively, host cells that contain a nucleic acid sequence encoding a polypeptide of the invention and which express said polypeptide may be identified by a variety of procedures known to those of skill in the art. These procedures include, but are not limited to, DNA-DNA or DNA-RNA hybridizations and protein bioassays, for example, fluorescence activated cell sorting (FACS) or immunoassay techniques (such as the enzyme-linked immunosorbent assay [ELISA] and radioimmunoassay [RIA]), that include membrane, solution, or chip based technologies for the detection and/or quantification of nucleic acid or protein (see Hampton, R. et al. (1990) Serological Methods, a Laboratory Manual, APS Press, St Paul, MN) and Maddox, D.E. et al. (1983) J. Exp. Med, 158, 1211-1216).

A wide variety of labels and conjugation techniques are known by those skilled in the art and may be used in various nucleic acid and amino acid assays. Means for producing labelled hybridization or PCR probes for detecting sequences related to nucleic acid molecules encoding polypeptides of the present invention include oligolabelling, nick translation, end-labelling or PCR amplification using a labelled polynucleotide. Alternatively, the sequences encoding the polypeptide of the invention may be cloned into a vector for the production of an mRNA probe. Such vectors are known in the art, are commercially available, and may be used to synthesise RNA probes *in vitro* by addition of an appropriate RNA polymerase such as T7, T3 or SP6 and labelled nucleotides. These procedures may be conducted using a variety of commercially available kits (Pharmacia & Upjohn, (Kalamazoo, MI); Promega (Madison WI); and U.S. Biochemical Corp., Cleveland, OH)).

Suitable reporter molecules or labels, which may be used for ease of detection, include radionuclides, enzymes and fluorescent, chemiluminescent or chromogenic agents as well as substrates, cofactors, inhibitors, magnetic particles, and the like.

Nucleic acid molecules according to the present invention may also be used to create transgenic animals, particularly rodent animals. This may be done locally by modification of somatic cells, or by germ line therapy to incorporate heritable modifications. Such transgenic animals may be particularly useful in the generation of animal models for drug molecules effective as modulators of the polypeptides of the present invention.

The polypeptide can be recovered and purified from recombinant cell cultures by well-known methods including ammonium sulphate or ethanol precipitation, acid extraction, anion or cation exchange chromatography, phosphocellulose chromatography, hydrophobic interaction chromatography, affinity chromatography, hydroxylapatite chromatography and lectin chromatography. High performance liquid chromatography is particularly useful for purification. Well known techniques for refolding proteins may be employed to regenerate an active conformation when the polypeptide is denatured during isolation and or purification.

Specialised vector constructions may also be used to facilitate purification of proteins, as desired, by joining sequences encoding the polypeptides of the invention to a nucleotide sequence encoding a polypeptide domain that will facilitate purification of soluble proteins. Examples of such purification-facilitating domains include metal chelating peptides such as histidine-tryptophan modules that allow purification on immobilised metals, protein A domains that allow purification on immobilised immunoglobulin, and the domain utilised in the FLAGS extension/affinity purification system (Immunex Corp., Seattle, WA). The inclusion of cleavable linker sequences such as those specific for Factor XA or enterokinase (Invitrogen, San Diego, CA) between the purification domain and the polypeptide of the invention may be used to facilitate purification. One such expression vector provides for expression of a fusion protein containing the polypeptide of the invention fused to several histidine residues preceding a thioredoxin or an enterokinase cleavage site. The histidine residues facilitate purification by IMAC (immobilised metal ion affinity chromatography as described in Porath, J. et al. (1992), Prot. Exp. Purif. 3: 263-281) while the thioredoxin or enterokinase cleavage site provides a means for purifying the polypeptide from the fusion protein. A discussion of vectors which contain fusion proteins is provided in Kroll, D.J. et al. (1993; DNA Cell Biol. 12:441-453).

If the polypeptide is to be expressed for use in screening assays, generally it is preferred that it be produced at the surface of the host cell in which it is expressed. In this event, the host cells may be harvested prior to use in the screening assay, for example using techniques such as fluorescence activated cell sorting (FACS) or immunoaffinity techniques. If the polypeptide is secreted into the medium, the medium can be recovered in order to recover and purify the expressed polypeptide. If polypeptide is produced intracellularly, the cells must first be lysed before the polypeptide is recovered.

Alternatively, it may be preferred that the polypeptides of the invention be expressed as cell-surface fusion proteins. In this event, the host cells may be harvested prior to use in the screening assay, for example using techniques such as fluorescence activated cell sorting (FACs) or immunoaffinity techniques.

As indicated above, the present invention also provides novel targets and methods for the screening of drug candidates or leads. These screening methods include binding assays and/or functional assays, and may be performed in vitro, in cell systems or in animals.

In this regard, a particular object of this invention resides in the use of an INSP215 polypeptide as a target for screening candidate drugs for treating or preventing disorders in which ZP domain containing proteins, such as TGF beta Receptor III, are implicated.

Also described herein are methods of selecting biologically active compounds, said methods comprising contacting a candidate compound with an INSP215 gene or polypeptide, and selecting compounds that bind said gene or polypeptide.

Also described herein are methods of selecting biologically active compounds, said method comprising contacting a candidate compound with recombinant host cell expressing a INSP215 polypeptide with a candidate compound, and selecting compounds that bind said INSP215 polypeptide at the surface of said cells and/or that modulate the activity of the INSP215 polypeptide.

A "biologically active" compound denotes any compound having biological activity in a subject, preferably therapeutic activity, more preferably a compound that can be used for treating disorders in which ZP domain containing proteins, such as TGF beta Receptor III, are implicated, or as a lead to develop drugs for treating disorders in which ZP domain containing proteins, such as TGF beta Receptor III, are implicated. A "biologically active" compound preferably is a compound that modulates the activity of a INSP215 polypeptide.

The above methods may be conducted *in vitro,* using various devices and conditions, including with immobilized reagents, and may further comprise an additional step of assaying the activity of the selected compounds in a model of a disorder in which ZP domain containing proteins, such as TGF beta Receptor III, are implicated, such as an animal model.

Binding to a target gene or polypeptide provides an indication as to the ability of the compound to modulate the activity of said target, and thus to affect a pathway leading to disorder in a subject. The determination of binding may be performed by various techniques, such as by labelling of the candidate compound, by competition with a labelled reference ligand, *etc.* For *in vitro* binding assays, the polypeptides may be used in essentially pure form, in suspension, immobilized on a support, or expressed in a membrane (intact cell, membrane preparation, liposome, *etc*.).

Modulation of activity includes, without limitation, stimulation of the surface expression of a receptor, and modulation of multimerization of said receptor (*e.g*., the formation of multimeric complexes with other sub-units), *etc.* The cells used in the assays may be any recombinant cell (*i.e*., any cell comprising a recombinant nucleic acid encoding a INSP215 polypeptide) or any cell that expresses an endogenous INSP215 polypeptide. Examples of such cells include, without limitation, prokaryotic cells (such as bacteria) and eukaryotic cells (such as yeast cells, mammalian cells, insect cells, plant cells, *etc*.). Specific examples include E.coli, *Pichia pastoris, Hansenula polymorpha, Schizosaccharomyces pombe, Kluyveromyces* or *Saccharomyces* yeasts, mammalian cell lines (*e.g.,* Vero cells, CHO cells, 3T3 cells, COS cells, etc.) as well as primary or established mammalian cell cultures (e.g., produced from fibroblasts, embryonic cells, epithelial cells, nervous cells, adipocytes, *etc*.).

Preferred selected compounds are agonists of INSP215, *i.e*., compounds that can bind to INSP215 and mimic the activity of an endogenous ligand thereof.

Also described herein is a method of selecting biologically active compounds, said method comprising contacting in vitro a test compound with a INSP215 polypeptide according to the present invention and determining the ability of said test compound to modulate the activity of said INSP215 polypeptide.

Also described herein is a method of selecting biologically active compounds, said method comprising contacting in vitro a test compound with a INSP215 gene according to the present invention and determining the ability of said test compound to modulate the expression of said INSP215 gene, preferably to stimulate expression thereof.

Also described herein is a method of screening, selecting or identifying active compounds, the method comprising contacting a test compound with a recombinant host cell comprising a reporter construct, said reporter construct comprising a reporter gene under the control of a INSP215 gene promoter, and selecting the test compounds that modulate (*e.g*. stimulate or reduce, preferably stimulate) expression of the reporter gene.

The polypeptide of the invention can be used to screen libraries of compounds in any of a variety of drug screening techniques. Such compounds may activate (agonise) or inhibit (antagonise) the level of expression of the gene or the activity of the polypeptide of the invention. Preferred compounds are effective to alter the expression of a natural gene which encodes a polypeptide of the first aspect of the invention or to regulate the activity of a polypeptide of the first aspect of the invention.

Agonist or antagonist compounds may be isolated from, for example, cells, cell-free preparations, chemical libraries or natural product mixtures. These agonists or antagonists may be natural or modified substrates, ligands, enzymes, receptors or structural or functional mimetics. For a suitable review of such screening techniques, see Coligan *et al.,* Current Protocols in Immunology 1(2):Chapter 5 (1991).

Compounds that are most likely to be good antagonists are molecules that bind to the polypeptide of the invention without inducing the biological effects of the polypeptide upon binding to it. Potential antagonists include small organic molecules, peptides, polypeptides and antibodies that bind to the polypeptide of the invention and thereby inhibit or extinguish its activity. In this fashion, binding of the polypeptide to normal cellular binding molecules may be inhibited, such that the normal biological activity of the polypeptide is prevented.

The polypeptide of the invention that is employed in such a screening technique may be free in solution, affixed to a solid support, borne on a cell surface or located intracellularly. In general, such screening procedures may involve using appropriate cells or cell membranes that express the polypeptide that are contacted with a test compound to observe binding, or stimulation or inhibition of a functional response. The functional response of the cells contacted with the test compound is then compared with control cells that were not contacted with the test compound. Such an assay may assess whether the test compound results in a signal generated by activation of the polypeptide, using an appropriate detection system. Inhibitors of activation are generally assayed in the presence of a known agonist and the effect on activation by the agonist in the presence of the test compound is observed.

Methods for generating detectable signals in the types of assays described herein will be known to those of skill in the art. A particular example is cotransfecting a construct expressing a polypeptide according to the invention, or a fragment such as the LBD, in fusion with the GAL4 DNA binding domain, into a cell together with a reporter plasmid, an example of which is pFR-Luc (Stratagene Europe, Amsterdam, The Netherlands). This particular plasmid contains a synthetic promoter with five tandem repeats of GAL4 binding sites that control the expression of the luciferase gene. When a potential ligand is added to the cells, it will bind the GAL4-polypeptide fusion and induce transcription of the luciferase gene. The level of the luciferase expression can be monitored by its activity using a luminescence reader (see, for example, Lehman et al. JBC 270, 12953, 1995; Pawar et al. JBC, 277, 39243, 2002).

A preferred method for identifying an agonist or antagonist compound of a polypeptide of the present invention comprises:
(a) contacting a cell expressing on the surface thereof the polypeptide according to the first aspect of the invention, the polypeptide being associated with a second component capable of providing a detectable signal in response to the binding of a compound to the polypeptide, with a compound to be screened under conditions to permit binding to the polypeptide; and
(b) determining whether the compound binds to and activates or inhibits the polypeptide by measuring the level of a signal generated from the interaction of the compound with the polypeptide.

A further preferred method for identifying an agonist or antagonist of a polypeptide of the invention comprises:
(a) contacting a cell expressing on the surface thereof the polypeptide, the polypeptide being associated with a second component capable of providing a detectable signal in response to the binding of a compound to the polypeptide, with a compound to be screened under conditions to permit binding to the polypeptide; and
(b) determining whether the compound binds to and activates or inhibits the polypeptide by comparing the level of a signal generated from the interaction of the compound with the polypeptide with the level of a signal in the absence of the compound.

For example, a method such as FRET detection of a ligand bound to the polypeptide in the presence of peptide co-activators (Norris et al., Science 285, 744, 1999) might be used. The general methods that are described above may further comprise conducting the identification of agonist or antagonist in the presence of labelled or unlabelled ligand for the polypeptide.

In another embodiment of the method for identifying an agonist or antagonist of a polypeptide of the present invention comprises:
determining the inhibition of binding of a ligand to cells which have a polypeptide of the invention on the surface thereof, or to cell membranes containing such a polypeptide, in the presence of a candidate compound under conditions to permit binding to the polypeptide, and determining the amount of ligand bound to the polypeptide. A compound capable of causing reduction of binding of a ligand is considered to be an agonist or antagonist. Preferably the ligand is labelled.

More particularly, a method of screening for a polypeptide antagonist or agonist compound comprises the steps of:
(a) incubating a labelled ligand with a whole cell expressing a polypeptide according to the invention on the cell surface, or a cell membrane containing a polypeptide of the invention,
(b) measuring the amount of labelled ligand bound to the whole cell or the cell membrane;
(c) adding a candidate compound to a mixture of labelled ligand and the whole cell or the cell membrane of step (a) and allowing the mixture to attain equilibrium;
(d) measuring the amount of labelled ligand bound to the whole cell or the cell membrane after step (c); and
(e) comparing the difference in the labelled ligand bound in step (b) and (d), such that the compound which causes the reduction in binding in step (d) is considered to be an agonist or antagonist.

Simple binding assays may be used, in which the adherence of a test compound to a surface bearing the polypeptide is detected by means of a label directly or indirectly associated with the test compound or in an assay involving competition with a labelled competitor. In another embodiment, competitive drug screening assays may be used, in which neutralising antibodies that are capable of binding the polypeptide specifically compete with a test compound for binding. In this manner, the antibodies can be used to detect the presence of any test compound that possesses specific binding affinity for the polypeptide.

Screening assays known in the art that are suitable for the identification of modulators of the polypeptides of this invention include the GAL4 assay (for example, Jurgen M. Lehmann et al., J. Biol. Chem., Jun 1995; 270: 12953- 12956; Raivio T. et al., J. Clin. Endocrinol. Metab. 86 (2001) 1539- 44), biophysical techniques such as surface plasmon resonance (supplied by Biacore AB, Uppsala, Sweden) and assays based on fluorescence polarisation or FRET for the ligand-induced binding of the co-activator peptide (Mukherjee R. et al., J Steroid Biochem. Mol. Biol. 2002 Jul;81(3):217-25).

Assays may also be designed to detect the effect of added test compounds on the production of mRNA encoding the polypeptide in cells. For example, an ELISA may be constructed that measures secreted or cell-associated levels of polypeptide using monoclonal or polyclonal antibodies by standard methods know in the art, and this can be used to search for compounds that may inhibit or enhance the production of the polypeptide from suitably manipulated cells or tissues. The formation of binding complexes between the polypeptide and the compound being tested may then be measured.

Another technique for drug screening which may be used provides for high throughput screening of compounds having suitable binding affinity to the polypeptide of interest (see International patent application WO84/03564). In this method, large numbers of different small test compounds are synthesised on a solid substrate, which may then be reacted with the polypeptide of the invention and washed. One way of immobilising the polypeptide is to use non-neutralising antibodies. Bound polypeptide may then be detected using methods that are well known in the art. Purified polypeptide can also be coated directly onto plates for use in the aforementioned drug screening techniques.

Assay methods include those that involve the use of the genes and polypeptides of the invention in overexpression or ablation assays. Such assays involve the manipulation of levels of these genes/polypeptides in cells and assessment of the impact of this manipulation event on the physiology of the manipulated cells. For example, such experiments reveal details of signalling and metabolic pathways in which the particular genes/polypeptides are implicated, generate information regarding the identities of polypeptides with which the studied polypeptides interact and provide clues as to methods by which related genes and proteins are regulated.

Another technique for drug screening which may be used provides for high throughput screening of compounds having suitable binding affinity to the polypeptide of interest (see International patent application WO84/03564). In this method, large numbers of different small test compounds are synthesised on a solid substrate, which may then be reacted with the polypeptide of the invention and washed. One way of immobilising the polypeptide is to use non-neutralising antibodies. Bound polypeptide may then be detected using methods that are well known in the art. Purified polypeptide can also be coated directly onto plates for use in the aforementioned drug screening techniques.

The polypeptide of the invention may be used to identify membrane-bound or soluble receptors, through standard receptor binding techniques that are known in the art, such as ligand binding and cross-linking assays in which the polypeptide is labelled with a radioactive isotope, is chemically modified, or is fused to a peptide sequence that facilitates its detection or purification, and incubated with a source of the putative receptor (for example, a composition of cells, cell membranes, cell supernatants, tissue extracts, or bodily fluids). The efficacy of binding may be measured using biophysical techniques such as surface plasmon resonance (supplied by Biacore AB, Uppsala, Sweden) and spectroscopy. Binding assays may be used for the purification and cloning of the receptor, but may also identify agonists and antagonists of the polypeptide, that compete with the binding of the polypeptide to its receptor. Standard methods for conducting screening assays are well understood in the art.

Also described herein is the use of a INSP215 polypeptide or fragment thereof, whereby the fragment is preferably a INSP215 gene-specific fragment, for isolating or generating an agonist or stimulator of the INSP215 polypeptide for the treatment of a disorder, wherein said agonist or stimulator is selected from the group consisting of:
1. a specific antibody or fragment thereof including: a)a chimeric, b) a humanized or c) a fully human antibody, as well as;
2. a bispecific or multispecific antibody,
3. a single chain (*e.g*. scFv) or
4. single domain antibody, or
5. a peptide- or non-peptide mimetic derived from said antibodies or
6. an antibody-mimetic such as a) an anticalin or b) a fibronectin-based binding molecule (e.g. trinectin or adnectin).

The generation of peptide- or non-peptide mimetics from antibodies is known in the art (Saragovi *et al.,* 1991 and Saragovi *et al.,* 1992).

Anticalins are also known in the art (Vogt *et al.,* 2004). Fibronectin-based binding molecules are described in US6818418 and WO2004029224.

Furthermore, the test compound may be of various origin, nature and composition, such as any small molecule, nucleic acid, lipid, peptide, polypeptide including an antibody such as a chimeric, humanized or fully human antibody or an antibody fragment, peptide- or non-peptide mimetic derived therefrom as well as a bispecific or multispecific antibody, a single chain (*e.g*. scFv) or single domain antibody or an antibody-mimetic such as an anticalin or fibronectin-based binding molecule (*e.g*. trinectin or adnectin), *etc*., in isolated form or in mixture or combinations.

Also described herein is a screening kit useful in the methods for identifying agonists, antagonists, ligands, receptors, substrates, enzymes, that are described above.

The disclosure includes the agonists, antagonists, ligands, receptors, substrates and enzymes, and other compounds which modulate the activity or antigenicity of the polypeptide of the invention discovered by the methods that are described above.

As mentioned above, it is envisaged that the various moieties of the invention (*i.e*. the polypeptides of the first aspect of the invention, a nucleic acid molecule of the second or third aspect of the invention, a vector of the fourth aspect of the invention, a host cell of the fifth aspect of the invention, a ligand of the sixth aspect of the invention, a compound of the seventh aspect of the disclosure) may be useful in the therapy or diagnosis of diseases. To assess the utility of the moieties of the invention for treating or diagnosing a disease one or more of the assays described herein may be carried out. Note that although some of the assays refer to the test compound as being a protein/polypeptide, a person skilled in the art will readily be able to adapt the following assays so that the other moieties of the invention may also be used as the "test compound".

Also described herein are pharmaceutical compositions comprising a polypeptide, nucleic acid, ligand or compound of the disclosure in combination with a suitable pharmaceutical carrier. These compositions may be suitable as therapeutic or diagnostic reagents, as vaccines, or as other immunogenic compositions, as outlined in detail below.

According to the terminology used herein, a composition containing a polypeptide, nucleic acid, ligand or compound [X] is "substantially free of" impurities [herein, Y] when at least 85% by weight of the total X+Y in the composition is X. Preferably, X comprises at least about 90% by weight of the total of X+Y in the composition, more preferably at least about 95%, 98% or even 99% by weight.

The pharmaceutical compositions should preferably comprise a therapeutically effective amount of the polypeptide, nucleic acid molecule, ligand, or compound of the invention. The term "therapeutically effective amount" as used herein refers to an amount of a therapeutic agent needed to treat, ameliorate, or prevent a targeted disease or condition, or to exhibit a detectable therapeutic or preventative effect. For any compound, the therapeutically effective dose can be estimated initially either in cell culture assays, for example, of neoplastic cells, or in animal models, usually mice, rabbits, dogs, or pigs. The animal model may also be used to determine the appropriate concentration range and route of administration. Such information can then be used to determine useful doses and routes for administration in humans.

The precise effective amount for a human subject will depend upon the severity of the disease state, general health of the subject, age, weight, and gender of the subject, diet, time and frequency of administration, drug combination(s), reaction sensitivities, and tolerance/response to therapy. This amount can be determined by routine experimentation and is within the judgement of the clinician. Generally, an effective dose will be from 0.01 mg/kg to 50 mg/kg, preferably 0.05 mg/kg to 10 mg/kg. Compositions may be administered individually to a patient or may be administered in combination with other agents, drugs or hormones.

A pharmaceutical composition may also contain a pharmaceutically acceptable carrier, for administration of a therapeutic agent. Such carriers include antibodies and other polypeptides, genes and other therapeutic agents such as liposomes, provided that the carrier does not itself induce the production of antibodies harmful to the individual receiving the composition, and which may be administered without undue toxicity. Suitable carriers may be large, slowly metabolised macromolecules such as proteins, polysaccharides, polylactic acids, polyglycolic acids, polymeric amino acids, amino acid copolymers and inactive virus particles.

Pharmaceutically acceptable salts can be used therein, for example, mineral acid salts such as hydrochlorides, hydrobromides, phosphates, sulphates, and the like; and the salts of organic acids such as acetates, propionates, malonates, benzoates, and the like. A thorough discussion of pharmaceutically acceptable carriers is available in Remington's Pharmaceutical Sciences (Mack Pub. Co., N.J. 1991).

Pharmaceutically acceptable carriers in therapeutic compositions may additionally contain liquids such as water, saline, glycerol and ethanol. Additionally, auxiliary substances, such as wetting or emulsifying agents, pH buffering substances, and the like, may be present in such compositions. Such carriers enable the pharmaceutical compositions to be formulated as tablets, pills, dragees, capsules, liquids, gels, syrups, slurries, suspensions, and the like, for ingestion by the patient.

Once formulated, the compositions described herein can be administered directly to the subject. The subjects to be treated can be animals; in particular, human subjects can be treated.

The pharmaceutical compositions described herein may be administered by any number of routes including, but not limited to, oral, intravenous, intramuscular, intraarterial, intramedullary, intrathecal, intraventricular, transdermal or transcutaneous applications (for example, see WO98/20734), subcutaneous, intraperitoneal, intranasal, enteral, topical, sublingual, intravaginal or rectal means. Gene guns or hyposprays may also be used to administer the pharmaceutical compositions of the invention. Typically, the therapeutic compositions may be prepared as injectables, either as liquid solutions or suspensions; solid forms suitable for solution in, or suspension in, liquid vehicles prior to injection may also be prepared.

Direct delivery of the compositions will generally be accomplished by injection, subcutaneously, intraperitoneally, intravenously or intramuscularly, or delivered to the interstitial space of a tissue. The compositions can also be administered into a lesion. Dosage treatment may be a single dose schedule or a multiple dose schedule.

If the activity of the polypeptide of the invention is in excess in a particular disease state, several approaches are available. One approach comprises administering to a subject an inhibitor compound (antagonist) as described above, along with a pharmaceutically acceptable carrier in an amount effective to inhibit the function of the polypeptide, such as by blocking the binding of ligands, substrates, enzymes, receptors, or by inhibiting a second signal, and thereby alleviating the abnormal condition. Preferably, such antagonists are antibodies. Most preferably, such antibodies are chimeric and/or humanised to minimise their immunogenicity, as described previously.

In another approach, soluble forms of the polypeptide that retain binding affinity for the ligand, substrate, enzyme, receptor, in question, may be administered. Typically, the polypeptide may be administered in the form of fragments that retain the relevant portions.

In an alternative approach, expression of the gene encoding the polypeptide can be inhibited using expression blocking techniques, such as the use of antisense nucleic acid molecules (as described above), either internally generated or separately administered. Modifications of gene expression can be obtained by designing complementary sequences or antisense molecules (DNA, RNA, or PNA) to the control, 5' or regulatory regions (signal sequence, promoters, enhancers and introns) of the gene encoding the polypeptide. Similarly, inhibition can be achieved using "triple helix" base-pairing methodology. Triple helix pairing is useful because it causes inhibition of the ability of the double helix to open sufficiently for the binding of polymerases, transcription factors, or regulatory molecules. Recent therapeutic advances using triplex DNA have been described in the literature (Gee, J.E. et al. (1994) In: Huber, B.E. and B.I. Carr, Molecular and Immunologic Approaches, Futura Publishing Co., Mt. Kisco, NY). The complementary sequence or antisense molecule may also be designed to block translation of mRNA by preventing the transcript from binding to ribosomes. Such oligonucleotides may be administered or may be generated *in situ* from expression *in vivo.*

In addition, expression of the polypeptide of the invention may be prevented by using ribozymes specific to its encoding mRNA sequence. Ribozymes are catalytically active RNAs that can be natural or synthetic (see for example Usman, N, et al., Curr. Opin. Struct. Biol (1996) 6(4), 527-33). Synthetic ribozymes can be designed to specifically cleave mRNAs at selected positions thereby preventing translation of the mRNAs into functional polypeptide. Ribozymes may be synthesised with a natural ribose phosphate backbone and natural bases, as normally found in RNA molecules. Alternatively the ribozymes may be synthesised with non-natural backbones, for example, 2'-O-methyl RNA, to provide protection from ribonuclease degradation and may contain modified bases.

RNA molecules may be modified to increase intracellular stability and half-life. Possible modifications include, but are not limited to, the addition of flanking sequences at the 5' and/or 3' ends of the molecule or the use of phosphorothioate or 2' O-methyl rather than phosphodiesterase linkages within the backbone of the molecule. This concept is inherent in the production of PNAs and can be extended in all of these molecules by the inclusion of non-traditional bases such as inosine, queosine and butosine, as well as acetyl-, methyl-, thio- and similarly modified forms of adenine, cytidine, guanine, thymine and uridine which are not as easily recognised by endogenous endonucleases.

For treating abnormal conditions related to an under-expression of the polypeptide of the invention and its activity, several approaches are also available. One approach comprises administering to a subject a therapeutically effective amount of a compound that activates the polypeptide, i.e., an agonist as described above, to alleviate the abnormal condition. Alternatively, a therapeutic amount of the polypeptide in combination with a suitable pharmaceutical carrier may be administered to restore the relevant physiological balance of polypeptide.

Gene therapy may be employed to effect the endogenous production of the polypeptide by the relevant cells in the subject. Gene therapy is used to treat permanently the inappropriate production of the polypeptide by replacing a defective gene with a corrected therapeutic gene.

Gene therapy described herein can occur *in vivo* or *ex vivo*. *Ex vivo* gene therapy requires the isolation and purification of patient cells, the introduction of a therapeutic gene and introduction of the genetically altered cells back into the patient In contrast, *in vivo* gene therapy does not require isolation and purification of a patient's cells.

The therapeutic gene is typically "packaged" for administration to a patient. Gene delivery vehicles may be non-viral, such as liposomes, or replication-deficient viruses, such as adenovirus as described by Berkner, K.L., in Curr. Top. Microbiol. Immunol., 158, 39-66 (1992) or adeno-associated virus (AAV) vectors as described by Muzyczka, N., in Curr. Top. Microbiol. Immunol., 158, 97-129 (1992) and U.S. Patent No. 5,252,479. For example, a nucleic acid molecule encoding a polypeptide of the invention may be engineered for expression in a replication-defective retroviral vector. This expression construct may then be isolated and introduced into a packaging cell transduced with a retroviral plasmid vector containing RNA encoding the polypeptide, such that the packaging cell now produces infectious viral particles containing the gene of interest. These producer cells may be administered to a subject for engineering cells *in vivo* and expression of the polypeptide *in vivo* (see Chapter 20, Gene Therapy and other Molecular Genetic-based Therapeutic Approaches, (and references cited therein) in Human Molecular Genetics (1996), T Strachan and A P Read, BIOS Scientific Publishers Ltd).

Another approach is the administration of "naked DNA" in which the therapeutic gene is directly injected into the bloodstream or muscle tissue.

*In situ*ations in which the polypeptides or nucleic acid molecules of the invention are disease-causing agents, the invention provides that they can be used in vaccines to raise antibodies against the disease causing agent.

Vaccines described herein may either be prophylactic (ie. to prevent infection) or therapeutic (i.e. to treat disease after infection). Such vaccines comprise immunising antigen(s), immunogen(s), polypeptide(s), protein(s) or nucleic acid, usually in combination with pharmaceutically-acceptable carriers as described above, which include any carrier that does not itself induce the production of antibodies harmful to the individual receiving the composition: Additionally, these carriers may function as immunostimulating agents ("adjuvants"). Furthermore, the antigen or immunogen may be conjugated to a bacterial toxoid, such as a toxoid from diphtheria, tetanus, cholera, H. pylori, and other pathogens.

Since polypeptides may be broken down in the stomach, vaccines comprising polypeptides are preferably administered parenterally (for instance, subcutaneous, intramuscular, intravenous, or intradermal injection). Formulations suitable for parenteral administration include aqueous and non-aqueous sterile injection solutions which may contain antioxidants, buffers, bacteriostats and solutes which render the formulation isotonic with the blood of the recipient, and aqueous and non-aqueous sterile suspensions which may include suspending agents or thickening agents.

The vaccine formulations described herein may be presented in unit-dose or multi-dose containers. For example, sealed ampoules and vials and may be stored in a freeze-dried condition requiring only the addition of the sterile liquid carrier immediately prior to use. The dosage will depend on the specific activity of the vaccine and can be readily determined by routine experimentation.

Genetic delivery of antibodies that bind to polypeptides according to the invention may also be effected, for example, as described in International patent application WO98/55607.

The technology referred to as jet injection (see, for example, www.powderject.com) may also be useful in the formulation of vaccine compositions.

A number of suitable methods for vaccination and vaccine delivery systems are described in International patent application WO00/29428.

This invention also relates to the use of nucleic acid molecules according to the present invention as diagnostic reagents. Detection of a mutated form of the gene characterised by the nucleic acid molecules of the invention which is associated with a dysfunction will provide a diagnostic tool that can add to, or define, a diagnosis of a disease, or susceptibility to a disease, which results from under-expression, over-expression or altered spatial or temporal expression of the gene. Individuals carrying mutations in the gene may be detected at the DNA level by a variety of techniques.

Overexpression of certain polypeptides of the present invention, in particular of membrane-bound forms, may correlate with disease progression and prognosis. As such, polypeptides of the present invention, preferably membrane-bound forms, are useful as markers for disease progression and/or prognosis.

It is believed that certain tissues in mammals with cancer or inflammatory disease contain significantly greater INSP215 protein gene copy number and express significantly enhanced levels of the INSP215 protein and mRNA encoding the INSP215 protein when compared to a corresponding "standard" mammal, i.e., a mammal of the same species not having the cancer or inflammatory disease. Enhanced levels of the INSP215 protein will be detected in certain body fluids (e.g., sera, plasma, urine, synovial and spinal fluid) from mammals with cancer or inflammatory disease when compared to sera from mammals of the same species not having the cancer or inflammatory disease. Thus, the invention provides a method useful during tumor or inflammatory disease diagnosis, which involves assaying the expression level of the gene encoding the INSP215 protein or the gene copy number in mammalian cells or body fluid and comparing the gene expression level or gene copy number with a standard INSP215 protein gene expression level or gene copy number, whereby an increase in the gene expression level or gene copy number over the standard is indicative of certain tumors or inflammatory disease.

Where a tumor or inflammatory disease diagnosis has already been made according to conventional methods, the present invention is useful as a prognostic indicator.

By "assaying the expression level of the gene encoding the INSP215 protein" is intended qualitatively or quantitatively measuring or estimating the level of the INSP215 protein or the level of the mRNA encoding the INSP215 in a first biological sample either directly (e.g., by determining or estimating absolute protein level or mRNA level) or relatively (e.g., by comparing to the INSP215 protein level or mRNA level in a second biological sample). By "assaying the copy number of the gene encoding the INSP215 protein" is intended qualitatively or quantitatively measuring or estimating the gene copy number in a first biological sample either directly (e.g., by determining or estimating absolute gene copy number) or relatively (e.g., by comparing to the INSP215 protein gene copy number in a second biological sample).

Preferably, the INSP215 protein level, mRNA level, or gene copy number in the first biological sample is measured or estimated and compared to a standard INSP215 protein level, mRNA level, or gene copy number, the standard being taken from a second biological sample obtained from an individual not having the cancer or inflammatory disease. Alternatively, where the method is used as a prognostic indicator, both the first and second biological samples can be taken from individuals having the cancer or inflammatory disease and the relative expression levels or copy number will be measured to determine prognosis. As will be appreciated in the art, once a standard INSP215 protein level, mRNA level, or gene copy number is known, it can be used repeatedly as a standard for comparison.

By "biological sample" is intended any biological sample obtained from an individual, cell line, tissue culture, or other source which contains INSP215 protein or mRNA. Methods for obtaining tissue biopsies and body fluids from mammals are well known in the art. Where the biological sample is to include mRNA, a tissue biopsy is the preferred source.

The present invention is useful for detecting cancer and inflammatory disease in mammals. In particular the invention is useful during diagnosis or prognosis in mammals of the types of cancers and inflammatory diseases mentioned in the present invention. Preferred mammals include monkeys, apes, cats, dogs, cows, pigs, horses, rabbits and humans. Particularly preferred are humans.

Nucleic acid molecules for diagnosis may be obtained from a subject's cells, such as from blood, urine, saliva, tissue biopsy or autopsy material. The genomic DNA may be used directly for detection or may be amplified enzymatically by using PCR, ligase chain reaction (LCR), strand displacement amplification (SDA), or other amplification techniques (see Saiki et al., Nature, 324, 163-166 (1986); Bej, et al., Crit. Rev. Biochem. Molec. Biol., 26, 301-334 (1991); Birkenmeyer et al., J. Virol. Meth., 35, 117-126 (1991); Van Brunt, J., Bio/Technology, 8, 291-294 (1990)) prior to analysis.

Also described herein is a method of diagnosing a disease in a patient, comprising assessing the level of expression of a natural gene encoding a polypeptide according to the invention and comparing said level of expression to a control level, wherein a level that is different to said controls level is indicative of disease. The method may comprise the steps of:
a)contacting a sample of tissue from the patient with a nucleic acid probe under stringent conditions that allow the formation of a hybrid complex between a nucleic acid molecule of the invention and the probe;
b)contacting a control sample with said probe under the same conditions used in step a);
c)and detecting the presence of hybrid complexes in said samples;
wherein detection of levels of the hybrid complex in the patient sample that differ from levels of the hybrid complex in the control sample is indicative of disease.

Also described herein is a diagnostic method comprising the steps of:
a)obtaining a tissue sample from a patient being tested for disease;
b)isolating a nucleic acid molecule according to the invention from said tissue sample; and
c)diagnosing the patient for disease by detecting the presence of a mutation in the nucleic acid molecule which is associated with disease.

To aid the detection of nucleic acid molecules in the above-described methods, an amplification step, for example using PCR, may be included.

Deletions and insertions can be detected by a change in the size of the amplified product in comparison to the normal genotype. Point mutations can be identified by hybridizing amplified DNA to labelled RNA of the invention or alternatively, labelled antisense DNA sequences of the invention. Perfectly-matched sequences can be distinguished from mismatched duplexes by RNase digestion or by assessing differences in melting temperatures. The presence or absence of the mutation in the patient may be detected by contacting DNA with a nucleic acid probe that hybridises to the DNA under stringent conditions to form a hybrid double-stranded molecule, the hybrid double-stranded molecule having an unhybridised portion of the nucleic acid probe strand at any portion corresponding to a mutation associated with disease; and detecting the presence or absence of an unhybridised portion of the probe strand as an indication of the presence or absence of a disease-associated mutation in the corresponding portion of the DNA strand.

Such diagnostics are particularly useful for prenatal and even neonatal testing.

Point mutations and other sequence differences between the reference gene and "mutant" genes can be identified by other well-known techniques, such as direct DNA sequencing or single-strand conformational polymorphism, (see Orita et al., Genomics, 5, 874-879 (1989)). For example, a sequencing primer may be used with double-stranded PCR product or a single-stranded template molecule generated by a modified PCR. The sequence determination is performed by conventional procedures with radiolabelled nucleotides or by automatic sequencing procedures with fluorescent-tags. Cloned DNA segments may also be used as probes to detect specific DNA segments. The sensitivity of this method is greatly enhanced when combined with PCR. Further, point mutations and other sequence variations, such as polymorphisms, can be detected as described above, for example, through the use of allele-specific oligonucleotides for PCR amplification of sequences that differ by single nucleotides.

DNA sequence differences may also be detected by alterations in the electrophoretic mobility of DNA fragments in gels, with or without denaturing agents, or by direct DNA sequencing (for example, Myers et al., Science (1985) 230:1242). Sequence changes at specific locations may also be revealed by nuclease protection assays, such as RNase and S1 protection or the chemical cleavage method (see Cotton et al., Proc. Natl. Acad. Sci. USA (1985) 85: 4397-4401).

In addition to conventional gel electrophoresis and DNA sequencing, mutations such as microdeletions, aneuploidies, translocations, inversions, can also be detected by *in situ* analysis (see, for example, Keller et al., DNA Probes, 2nd Ed., Stockton Press, New York, N.Y., USA (1993)), that is, DNA or RNA sequences in cells can be analysed for mutations without need for their isolation and/or immobilisation onto a membrane. Fluorescence *in situ* hybridization (FISH) is presently the most commonly applied method and numerous reviews of FISH have appeared (see, for example, Trachuck et al., Science, 250, 559-562 (1990), and Trask et al., Trends, Genet., 7, 149-154 (1991)).

Also described herein is an array of oligonucleotide probes comprising a nucleic acid molecule according to the invention can be constructed to conduct efficient screening of genetic variants, mutations and polymorphisms. Array technology methods are well known and have general applicability and can be used to address a variety of questions in molecular genetics including gene expression, genetic linkage, and genetic variability (see for example: M.Chee et al., Science (1996), Vol 274, pp 610-613).

Also described herein, the array is prepared and used according to the methods described in PCT application WO95/11995 (Chee et al); Lockhart, D. J. et al. (1996) Nat. Biotech. 14: 1675-1680); and Schena, M. et al. (1996) Proc. Natl. Acad. Sci. 93: 10614-10619). Oligonucleotide pairs may range from two to over one million. The oligomers are synthesized at designated areas on a substrate using a light-directed chemical process. The substrate may be paper, nylon or other type of membrane, filter, chip, glass slide or any other suitable solid support. An oligonucleotide may be synthesized on the surface of the substrate by using a chemical coupling procedure and an ink jet application apparatus, as described in PCT application W095/25116 (Baldeschweiler et al). A "gridded" array analogous to a dot (or slot) blot may be used to arrange and link cDNA fragments or oligonucleotides to the surface of a substrate using a vacuum system, thermal, UV, mechanical or chemical bonding procedures. An array, such as those described above, may be produced by hand or by using available devices (slot blot or dot blot apparatus), materials (any suitable solid support), and machines (including robotic instruments), and may contain 8, 24, 96, 384, 1536 or 6144 oligonucleotides, or any other number between two and over one million which lends itself to the efficient use of commercially-available instrumentation.

In addition to the methods discussed above, diseases may be diagnosed by methods comprising determining, from a sample derived from a subject, an abnormally decreased or increased level of polypeptide or mRNA. Decreased or increased expression can be measured at the RNA level using any of the methods well known in the art for the quantitation of polynucleotides, such as, for example, nucleic acid amplification, for instance PCR, RT-PCR, RNase protection, Northern blotting and other hybridization methods.

Assay techniques that can be used to determine levels of a polypeptide of the present invention in a sample derived from a host are well-known to those of skill in the art and are discussed in some detail above (including radioimmunoassays, competitive-binding assays, Western Blot analysis and ELISA assays). This aspect of the invention provides a diagnostic method which comprises the steps of: (a) contacting a ligand as described above with a biological sample under conditions suitable for the formation of a ligand-polypeptide complex; and (b) detecting said complex.

Protocols such as ELISA, RIA, and FACS for measuring polypeptide levels may additionally provide a basis for diagnosing altered or abnormal levels of polypeptide expression. Normal or standard values for polypeptide expression are established by combining body fluids or cell extracts taken from normal mammalian subjects, preferably humans, with antibody to the polypeptide under conditions suitable for complex formation The amount of standard complex formation may be quantified by various methods, such as by photometric means.

Antibodies which specifically bind to a polypeptide of the invention may be used for the diagnosis of conditions or diseases characterised by expression of the polypeptide, or in assays to monitor patients being treated with the polypeptides, nucleic acid molecules, ligands and other compounds of the invention. Antibodies useful for diagnostic purposes may be prepared in the same manner as those described above for therapeutics. Diagnostic assays for the polypeptide include methods that utilise the antibody and a label to detect the polypeptide in human body fluids or extracts of cells or tissues. The antibodies may be used with or without modification, and may be labelled by joining them, either covalently or non-covalently, with a reporter molecule. A wide variety of reporter molecules known in the art may be used, several of which are described above.

Quantities of polypeptide expressed in subject, control and disease samples from biopsied tissues are compared with the standard values. Deviation between standard and subject values establishes the parameters for diagnosing disease. Diagnostic assays may be used to distinguish between absence, presence, and excess expression of polypeptide and to monitor regulation of polypeptide levels during therapeutic intervention. Such assays may also be used to evaluate the efficacy of a particular therapeutic treatment regimen in animal studies, in clinical trials or in monitoring the treatment of an individual patient.

A diagnostic kit described herein may comprise:
(a) a nucleic acid molecule of the present invention;
(b) a polypeptide of the present invention; or
(c) a ligand of the present invention.

Also described herein is a diagnostic kit which may comprise a first container containing a nucleic acid probe that hybridises under stringent conditions with a nucleic acid molecule according to the invention; a second container containing primers useful for amplifying the nucleic acid molecule; and instructions for using the probe and primers for facilitating the diagnosis of disease. The kit may further comprise a third container holding an agent for digesting unhybridised RNA.

Also described herein a diagnostic kit may comprise an array of nucleic acid molecules, at least one of which may be a nucleic acid molecule according to the invention.

To detect polypeptide according to the invention, a diagnostic kit may comprise one or more antibodies that bind to a polypeptide according to the invention; and a reagent useful for the detection of a binding reaction between the antibody and the polypeptide.

Such kits will be of use in diagnosing a disease or susceptibility to disease in which ZP domain containing polypeptides are implicated, or a disease in which ZP/TGR3 subdomain-containing proteins are implicated, as described above.

Various aspects and embodiments of the present invention will now be described in more detail by way of example, with particular reference to the INSP215 polypeptides.

It will be appreciated that modification of detail may be made without departing from the scope of the invention.

### Brief description of the Figures

**Figure 1**: INSP215 protein sequence with the predicted signal sequence underlined (residues 1-16), the ZP/TGR3 subdomain boxed (residues 39-183), the transmembrane region double underlined (residues 245-268), and N-glycosylation site highlighted (residue 139).
**Figure 2****:** Alignment of INSP215 protein sequence with the human genomic DNA encoding this protein (as determined on the basis of the assembly hg16 of July 2003).
**Figure 3****:** Alignment of the ZP/TGR3 subdomain of INSP215 and of TGF-beta receptor type III (hTGFR3; NCBI Acc. No. Q03167). Identical residues are indicated with an asterisk (*).
The cysteines are also highlighted. The conserved hydrophobic residues are indicated with 'h'. The conserved polar residues are indicated with 'p'. The conserved N-glycosylation site is boxed. The number of the first and the last residues indicated in the alignment is written aside the protein name for each line (the numbering reflects that of the corresponding full protein).

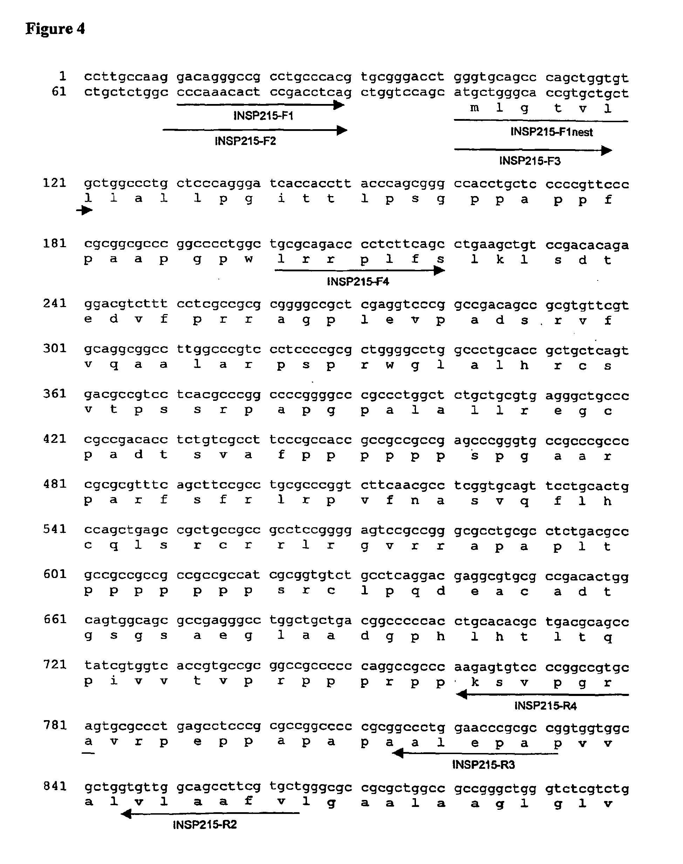

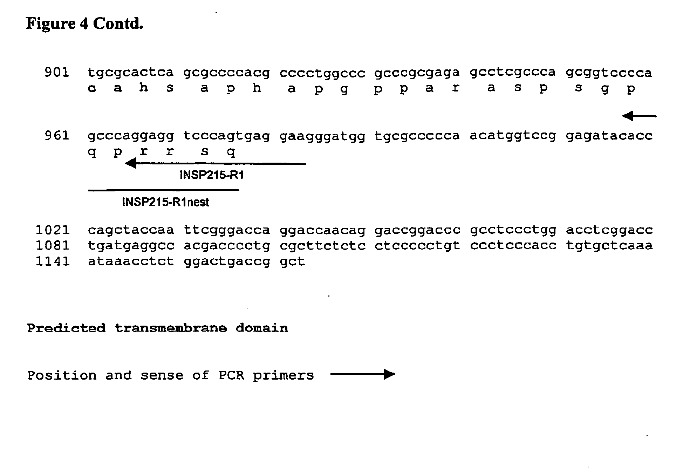

**Figure 4**: Nucleotide sequence of INSP215 prediction with translation.

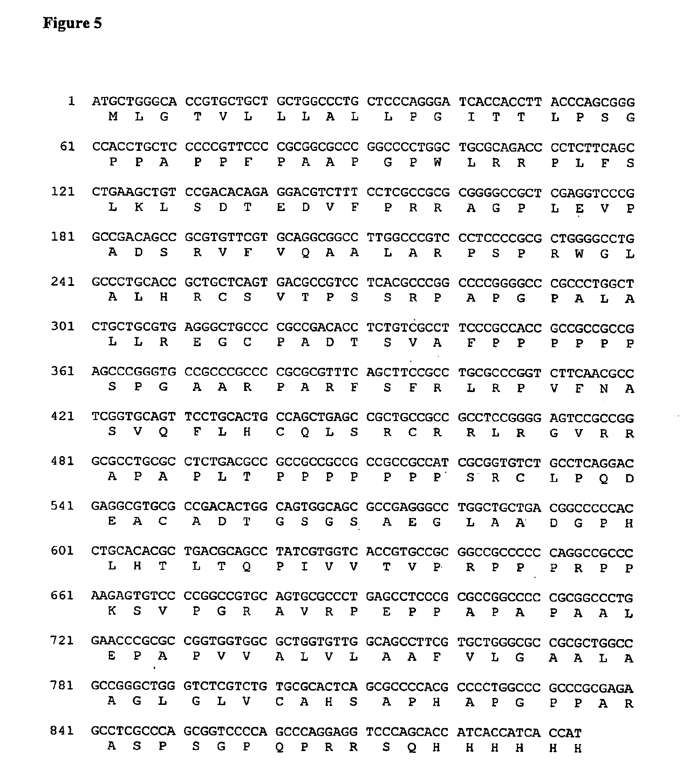

**Figure 5**: Nucleotide sequence with translation of the assembled INSP215 PCR product.

**TABLE 1**

| **Amino Acid** | **Synonymous Groups** | **More Preferred Synonymous Groups** |
|---|---|---|
| **Ser** | Gly, Ala, Ser, Thr, Pro | Thr, Ser |
| **Arg** | Asn, Lys, Gln, Arg, His | Arg, Lys, His |
| **Leu** | Phe, Ile, Val, Leu, Met | Ile, Val, Leu, Met |
| **Pro** | Gly, Ala, Ser, Thr, Pro | Pro |
| **Thr** | Gly, Ala, Ser, Thr, Pro | Thr, Ser |
| **Ala** | Gly, Thr, Pro, Ala, Ser | Gly, Ala |
| **Val** | Met, Phe, Ile, Leu, Val | Met, Ile, Val, Leu |
| **Gly** | Ala, Thr, Pro, Ser, Gly | Gly, Ala |
| **Ile** | Phe, Ile, Val, Leu, Met | Ile, Val, Leu, Met |
| **Phe** | Trp, Phe,Tyr | Tyr, Phe |
| **Tyr** | Trp, Phe,Tyr | Phe, Tyr |
| **Cys** | Ser, Thr, Cys | Cys |
| **His** | Asn, Lys, Gln, Arg, His | Arg, Lys, His |
| **Gln** | Glu, Asn, Asp, Gln | Asn, Gln |
| **Asn** | Glu, Asn, Asp, Gln | Asn, Gln |
| **Lys** | Asn, Lys, Gln, Arg, His | Arg, Lys, His |
| **Asp** | Glu, Asn, Asp, Gln | Asp, Glu |
| **Glu** | Glu, Asn, Asp, Gln | Asp, Glu |
| **Met** | Phe, Ile, Val, Leu, Met | Ile, Val, Leu, Met |
| **Trp** | Trp, Phe,Tyr | Trp |

**TABLE 2**

| **Amino Acid** | **Synonymous Groups** |
|---|---|
| Ser | D-Ser, Thr, D-Thr, allo-Thr, Met, D-Met, Met(O), D-Met(O), L-Cys, D-Cys |
| Arg | D-Arg, Lys, D-Lys, homo-Arg, D-homo-Arg, Met, Ile, D-.Met, D-Ile, Orn, D-Orn |
| Leu | D-Leu, Val, D-Val, AdaA, AdaG, Leu, D-Leu, Met, D-Met |
| Pro | D-Pro, L-I-thioazolidine-4-carboxylic acid, D-or L-1-oxazolidine-4-carboxylic acid |
| Thr | D-Thr, Ser, D-Ser, allo-Thr, Met,D-Met, Met(O), D-Met(O), Val, D-Val |
| Ala | D-Ala, Gly, Aib, B-Ala, Acp, L-Cys, D-Cys |
| Val | D-Val, Leu, D-Leu, Ile, D-Ile, Met, D-Met, AdaA, AdaG |
| Gly | Ala, D-Ala, Pro, D-Pro, Aib, .beta.-Ala, Acp |
| Ile | D-Ile, Val, D-Val, AdaA, AdaG, Leu, D-Leu, Met, D-Met |
| Phe | D-Phe, Tyr, D-Thr, L-Dopa, His, D-His, Trp, D-Trp, Trans-3,4, or 5-phenylproline, AdaA, AdaG, cis-3,4, or 5-phenylproline, Bpa, D-Bpa |
| Tyr | D-Tyr, Phe, D-Phe, L-Dopa, His, D-His |
| Cys | D-Cys, S--Me--Cys, Met, D-Met, Thr, D-Thr |
| Gln | D-Gln, Asn, D-Asn, Glu, D-Glu, Asp, D-Asp |
| Asn | D-Asn, Asp, D-Asp, Glu, D-Glu, Gln, D-Gln |
| Lys | D-Lys, Arg, D-Arg, homo-Arg, D-homo-Arg, Met, D-Met, Ile, D-Ile, Orn, D-Om |
| Asp | D-Asp, D-Asn, Asn, Glu, D-Glu, Gln, D-Gln |
| Glu | D-Glu, D-Asp, Asp, Asn, D-Asn, Gln, D-Gln |
| Met | D-Met, S--Me-Cys, Ile, D-Ile, Leu, D-Leu, Val, D-Val |

### Examples

### Example 1: Identification of INS215

An open reading frame of 879 nucleotides (SEQ ID NO:1) encodes the INSP215 full length polypeptide (SEQ ID NO:2), a protein of 292 amino acids (see Figure 1). The INSP215 full length polypeptide contains a predicted signal peptide (residues 1-16) leading a mature sequence of 276 amino acids (SEQ ID NO:4), encoded by a DNA sequence of 831 nucleotides (SEQ ID NO:3). The INSP215 full length polypeptide contains a predicted transmembrane region (residues 245-268) separating an intracellular region (residues 269-292) and an extracellular region (residues 1-244; SEQ ID NO:5 and SEQ ID NO:6). Further features of the extracellular region are the mature sequence (SEQ ID NO:7 and SEQ ID NO:8) containing the predicted ZP/TGR3 subdomain (SEQ ID NO:9 and SEQ ID NO:10) and a N-glycosylation site (located at residue 139).

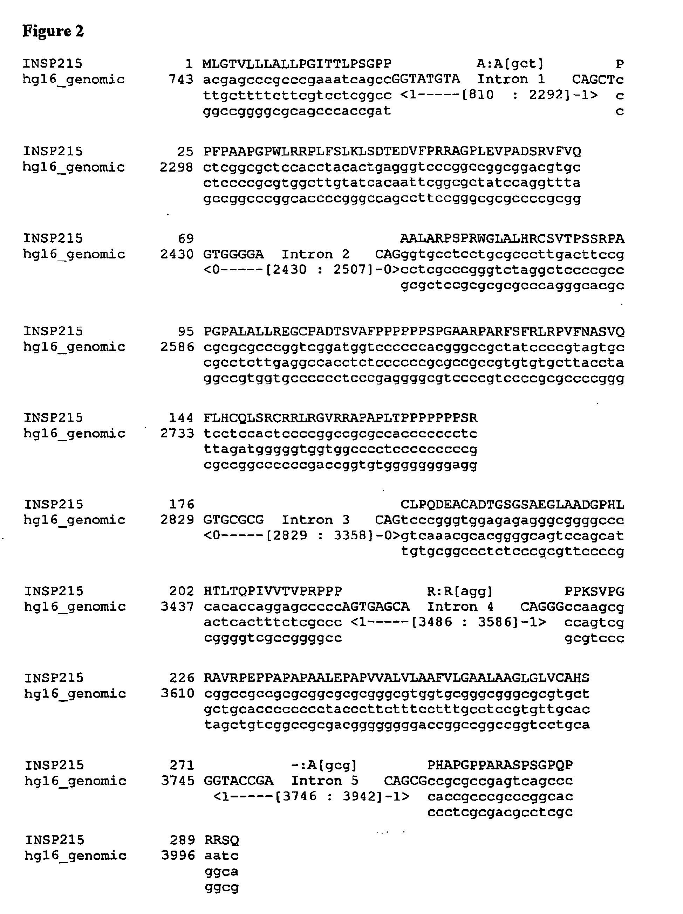

The ZP/TGR3 subdomain is found in the amino acid sequence encoded by the first four of the six exons encoding the INSP215 full length polypeptide (SEQ ID NOs 11-14; Figure 2).

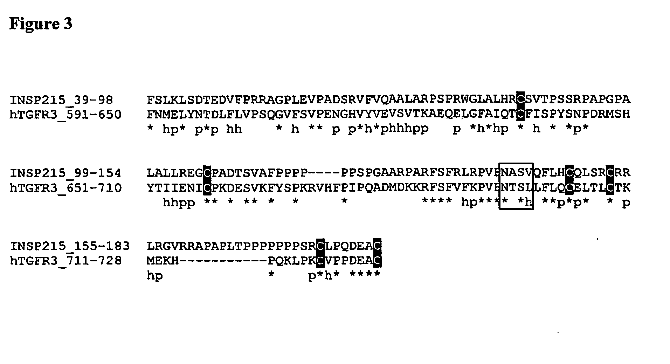

If INSP215 is used as a BLAST query against non-redundant sequence public databases, a segment of INSP215 extracellular region is highly homologous to the C-terminal ZP/TGR3 subdomain (Bork P and Sander C, FEBS Lett. 1992, 300: 237-40; Jovine L et al., Nat Cell Biol. 2002, 4: 457-61, Yonezawa N and Nakano M, Biochem Biophys Res Commun. 2003, 307: 877-82; Jovine L et al., Proc Natl Acad Sci U S A. 2004, 101: 5922-7). This similarity between INSP215 and TGF-beta Receptor III is not limited to the regularly spaced six Cysteines (residues 85, 106, 147, 152, 176, and 183 in INSP215; figures 1 and 3), but also involves several other polar or hydrophobic residues, as well as a N-glycosylation site (located in position 139 and 695 of these sequences, respectively; see Figures 1 and 3).

Preliminary data on the expression of INSP215 show that this protein has a specific up/down-regulation profile, in particular with reference to tumor cells isolated from colon, prostate, brain, and neuronal tissues.

The literature shows that the ZP/TGR3 domain can be distinguished structurally and functionally in two halves, and that a TGF-beta binding site is located in the C-terminal region of the ZP/TGR3 domain (WO 95/10610; Kaname S and Ruoslahti E, Biochem J. 1996, 315: 815-20). Accordingly, it is expected that the INSP215 polypeptides and functional fragments and/or fusion proteins based on the INSP215 polypeptides, in particular polypeptides comprising the amino acid sequence recited in SEQ ID NO:10, will bind one or more proteins belonging to the TGF-beta protein superfamily. When these INSP215-related sequences are secreted or soluble proteins, they can act as antagonists of proteins belonging to the TGF-beta protein superfamily.

On the basis of the annotation of the INSP215 polypeptides herein, it is now possible to design experiments to detect the presence of the INSP215 transcripts across a range of human tissue types to determine its tissue expression, in particular in normal and diseased tissues in order to establish more particularly the relevance of INSP215 in pathological contexts.

The cloning of the INSP215-related DNA sequences (either as cDNA or genomic exonic sequences, and exemplified in SEQ ID NO:1, 3, 5, 7, 9, 11 and 13) from tissues of human or mammalian origin will allow the expression of the corresponding encoded protein sequences in prokaryotic or eukaryotic expression systems and their subsequent purification and characterization as recombinant proteins.

Alternative recombinant INSP215 sequences can be produced by cloning in the appropriate expression vector the DNA encoding for the full sequence (SEQ ID NO:2), or for its selected fragments, in the form of signal peptide-containing (SEQ ID NO:6 and 12) or mature sequences (SEQ ID NO:4, 8, 10, and 14) maintaining the expected binding properties of the full protein towards proteins belonging to the TGF-beta superfamily. For example, transcripts including exons 1-4 (covering the entire ZP/TGR3 subdomain and the signal sequence) can be identified and/or cloned by primer hybridization or by PCR.

Whenever it is appropriate, heterologous DNA sequences encoding for protein sequences helping the expression, the secretion, and/or the detection of these recombinant sequences may be added in frame to the DNA encoding INSP215, or the derived protein sequences identified above. Examples of such heterologous sequences are signal peptides, immunoglobulin Fc regions, homo/heterodimerizing domains, and histidine tags. For example, fusion proteins containing the TGF-beta Receptor III extracellular domain and a heterodimerizing coil domain dimerize efficiently and bind TGF-beta with high affinity, providing a molecule with enhanced antagonistic properties against TGF-beta (De Crescenzo G et al., J Biol Chem. 2004: 279: 26013-8).

Soluble variants of INSP215 are expected to bind one or more members of the TGF-beta protein superfamily, capturing and retaining this protein from interacting with the signaling receptors, with the consequent inhibition of the proliferation and differentiation of multiple cell types normally induced by TGF-beta-like proteins. Therefore, the specific portions of INSP215 indicated herein as secreted or soluble proteins, which may be generated *in vivo* or *in vitro* following proteolytic digestion or alternative splicing, can have specific *in vivo* properties, in particular as antagonists of one or more protein belonging to the TGF-beta superfamily.

Such polypeptides are therefore expected to be useful for the treatment of diseases, in particular cancers, fibrosis, scarring, hypertension, atherosclerosis, reproductive disorders, hepatic disorders, lung disorders, immune disorders, developmental disorders, skin disorders, or disorders of connective tissues (Tsuchida K et al., Curr Drug Targets Immune Endocr Metabol Disord. 2004, 4: 157-66; Chang H et al., Endocr Rev. 2002, 23: 787-823; de Caestecker M, Cytokine Growth Factor Rev. 2004, 15: 1-11; WO 95/10610; WO 97/05892; Bandyopadhyay A et al., Cancer Res. 2002, 62: 4690-5; Liu M et al., Am J Respir Crit Care Med. 2002, 165: 419-23; Vilchis-Landeros MM et al., Biochem J. 2001, 355: 215-22).

The polypeptides disclosed herein may be useful in the treatment of diseases such as metastatic cancer, non-small-cell lung cancer, colon cancer, leukemia, testicular cancer, myeloma, lymphoma, colorectal cancer, prostate cancer, brain cancers, eye cancers, retinoblastoma, cancer of the uterus, cancer of the gut, cancer of the uterine cervix, endometrial carcinoma, lung cancer, cancer of the pancreas, chondrosarcoma, non-Hodgkin's lymphomas, ovarian sex cord-stromal tumors, ovarian carcinomas, gonadal tumors, renal cell cancer, B-chronic lymphocytic leukemia, gastric carcinoma, pancreatic carcinomas, pancreatic adenocarcinoma, small-cell lung cancer, gliomas, high-grade gliomas, malignant gliomas, gliosarcomas, anaplastic astrocytomas, glioblastomas, medulloblastoma, ependyoma, T-cell malignancies, bone marrow transplantations, fibrosis, idiopathic pulmonary fibrosis, scleroderma, post-operative scarring following glaucoma surgery, renal disease, diabetes, diabetic nephropathy, atherosclerosis, restrictive cardiomyopathy, angiogenesis disorder, bronchiolitis obliterans, cachexia, chronic obstructive pulmonary disease, wound healing, Crouzon's syndrome or glomerulonephritis.

Preferably, the leukemia is lymphocytic leukemia, acute lymphocytic leukemia, acute B cell leukemia, acute T cell leukemia, acute myelogenous leukemia, chronic myelogenous leukemia, or chronic lymphocytic leukemia.

Preferably the testicular cancer is seminoma, choriocarcinoma, embryonal carcinoma, teratoma or yolk sac tumor.

Preferably, the lung cancer is selected from benign or malignant primary tumors or from metastases from primary cancers of many other organs and tissues. Preferably the lung cancer is selected from primary lung tumors including bronchogenic carcinoma, bronchial carcinoid, chondromatous hamartoma (benign), solitary lymphoma, sarcoma (malignant) or multifocal lymphomas. Preferably, the bronchogenic carcinoma is selected from squamous cell carcinoma, undifferentiated small cell carcinoma, undifferentiated large cell carcinoma, adenocarcinoma or bronchioloalveolar carcinoma. Preferably, the bronchogenic carcinoma is squamous cell carcinoma or non-small cell lung carcinoma. Preferably the lung cancer is selected from metastases from primary cancers of the skin, breast, colon, prostate, kidney, thyroid, stomach, cervix, rectum, testis, and bone and from melanoma.

Preferably, the lymphoma is Hodgkin's Disease, small lymphocytic lymphoma (SLL/CLL), mantle cell lymphoma (MCL), follicular lymphoma (FL), marginal zone lymphoma (MZL) including extranodal (MALT lymphoma), nodal (monocytoid B-cell lymphoma) or splenic, diffuse large cell lymphoma, Burkitt's lymphoma, high grade Burkitt-like lymphoma or lymphoblastic lymphoma.

Recombinant INSP215 and the derived protein sequences identified above may be used to generate specific monoclonal or polyclonal antibodies that might then be used in the biochemical characterization of INSP215, as well as for diagnostic and/or therapeutic applications (in view of the information disclosed in the literature on fragments generated from these family of proteins). Alternatively, recombinant INSP215, and the derived protein sequences identified above, may be used in a wide variety of screening assays, in particular involving proteins included in the TGF-beta protein superfamily, for identifying specific ligands and associated biological activities.

### Example 2: Cloning of INSP215

INSP215 was a full length prediction for a ZP domain-containing protein of 292 amino acids (876 bp) encoded in 6 exons. A predicted signal peptide was identified within the sequence. A transmembrane domain was predicted spanning amino acids 245-268.

Interrogation of public EST sequence databases using the INSP215 sequence identified a number of ESTs which corresponded to it. These ESTs were mostly derived from eye, brain, lung, testis, or pancreas cDNA templates. One EST sequence which was derived from retinal epithelium cDNA, GenBank Accession BG752983, appeared to represent INSP215 from exon 2 to approximately the end of the predicted cds. This sequence corresponded to IMAGE clone ID 4875922. The IMAGE clone was purchased from ATCC and the insert sequenced using primers T7 and SP6. The IMAGE clone was found to contain the entire INSP215 cds from exon 2 onwards, as expected, and continued into the 3'-UTR to a polyA tract. The sequence upstream of the start of INSP215 exon 2 in the IMAGE clone (15 bp) did not correspond to INSP215 and did not contain a methionine. Several attempts were made to clone the INSP215 sequence, as described below.

Two pairs of nested PCR primers, INSP215-F1/INSP215-R1 and INSP215-F1nest/INSP215-R1nest (Table 3, Figure 4), were designed to amplify the full length of the INSP215 sequence in products of 913 bp and 876 bp, respectively. These primer pairs were tested as nested primers (i.e. the INSP215-F1nest/INSP215-R1nest primer pair were used in PCR2 with the products of the INSP215-F1/INSP215-R1 PCR1 as templates) using AmpliTaq on candidate cDNA library and individual cDNA templates, and then on the entire collection of cDNA libraries and pools of cDNA templates. No relevant products were obtained.

A third primer pair, INSP215-F2/INSP215-R2 (Table 3, Figure 4), was designed to amplify a 795 bp product containing the INSP215 predicted extracellular domain (INSP215-EC). This pair was tested in PCR using AmpliTaq on the collection of cDNA libraries and pools of cDNA templates, but again no relevant products were obtained.

The 3 primer pairs INSP215-FI/INSP215-R1, INSP215-F1nest/INSP215-R1nest, and INSP215-F2/INSP215-R2 were then tested on eye cDNA and retina cDNA and library templates (the strongest candidate templates) using Platinum Taq HiFi. The INSP215-F1/INSP215-R1 products were then used as templates for nested PCR using INSP215-F1nest/INSP215-R1nest and INSP215-F2/INSP215-R2. No relevant products were obtained from any of these amplifications.

A fourth primer pair, INSP215-F3/INSP215-R3 (Table 3, Figure 4), was designed to amplify a 732 bp product, again containing the predicted INSP215 extracellular domain. The INSP215-F2 primer had been positioned in the genomic sequence upstream of the predicted cds, but the INSP215-F3 primer was positioned within the predicted cds. Thus even if there was an unpredicted intron present just upstream of the start site, which had caused the INSP215-F2/INSP215-R2 primers to fail, the INSP215-F3/INSP215-R3 primers should work. The INSP215-F3/INSP215-R3 primer pair was tested on the retina and eye cDNA templates using both AmpliTaq and Platinum Taq HiFi. No relevant products were obtained.

A fifth primer pair, INSP215-F4/INSP215-R4 (Table 3, Figure 4), was designed to amplify the region of the prediction (exon 2 - exon 5) represented in the EST sequence GenBank Accession BG752983 (see above). This primer pair was also tested on the retina and eye cDNA templates using both AmpliTaq and Platinum Taq HiFi. No relevant products were obtained.

The INSP215 predicted cds was therefore created by addition of exon 1 (60 nucleotides) to the 5' end of INSP215 exon 2 (in Image clone 4875922) by PCR. The resultant full length cDNA was then subcloned with a 6HIS tag into pEAK12d for expression in HEK293 cells under the name of INSP215 (Figure 5). The resultant protein was below the limit of detection by western blot analysis, using antibodies directed against the 6HIS tag. The lack of secretion was almost certainly due to the fact that as the full length cDNA contains a transmembrane domain it would not be secreted. Therefore an expression construct only containing the extracellular domain of INSP215 (amino acids 1-234) was generated by deletion of the transmembrane and intracellular domains of INSP215 in the gateway entry clone pDONR221-INSP215-6HIS (plasmid ID. 17435) to generate pDONR221-INSP215-EC-6HIS (plasmid ID. 18175) which was subsequently transferred into the mammalian cell expression vectors pDEST12.2 and pEAK12d for production of recombinant INSP215 EC-6HIS in vivo and in vitro respectively. The resultant plasmids are pDEST12.2_INSP215-EC-6HIS and pEAK12d_INSP215-EC-6HIS. Plasmid pEAK12d_INSP215-EC-6HIS was transfected into HEK293/EBNA cells to produce recombinant INSP215SEC-6HIS. The 6HIS tagged protein is secreted.

### Example 3: Construction of mammalian cell expression vectors for INSP215

The plasmid containing the cds of INSP215 from exon 2 onwards (IMAGE clone ID 4875922) was used as PCR template to generate pEAK12d and pDEST12.2 expression clones containing the INSP215 ORF sequence with a 3' sequence encoding a 6HIS tag using the Gateway^{™} cloning methodology (Invitrogen). The predicted exon 1 (the first 60 bases of the prediction including start codon) was added by PCR.

### 3.1 Generation of Gateway compatible INSP215 ORF fused to an in frame 6HIS tag sequence.

The first stage of the Gateway cloning process involves a four step PCR reaction which generates the ORF of INSP215 flanked at the 5' end by an attB1 recombination site and Kozak sequence, and flanked at the 3' end by a sequence encoding an in-frame 6 histidine (6HIS) tag, a stop codon and the attB2 recombination site (Gateway compatible cDNA). The 60 missing bases at the 5' end of the INSP215 prediction were added by two step PCR reaction with the INSP215-EX1 and INSP215-EX2 primers.

The first PCR reaction (in a final volume of 50 µl) contains respectively: 1 µl (30 ng) of plasmid IMAGE clone ID 4875922, 1.5 µl dNTPs (10 mM), 10 µl of 10X Pfx polymerase buffer, 1 µl MgSO4 (50 mM), 2X PCRₓ Enhancer solution (Invitrogen), 0.5 µl each of gene specific primer (100 µM) (INSP215-EX1 and INSP215-EX4), and 0.5 µl Platinum Pfx DNA polymerase (Invitrogen). The PCR reaction was performed using an initial denaturing step of 95 °C for 2 min, followed by 20 cycles of 94 °C for 15 s; 55 °C for 30 s and 68 °C for 2 min; and a holding cycle of 4 °C. The amplification product PCR1 was directly cleaned-up using the Wizard PCR Clean-up DNA Purification System (Promega) and recovered in 50 µl sterile water according to the manufacturer's instructions. A 10 µl aliquot was visualized on 0.8 % agarose gel in 1 X TAE buffer (Invitrogen) in order to verify that the product was of the expected molecular weight (859 bp).

**Table 3:**

| **Primer** | **Sequence (5' - 3')** |
|---|---|
| INSP127-F1 | CCCAAACACTCCGACCTCA |
| INSP127-R1 | TTCCTCACTGGGACCTCCT |
| INSP127-F1nest | ATGCTGGGCACCGTGCTGCTG |
| INSP127-R1nest | CTGGGACCTCCTGGGCTGGG |
| INSP127-F2 | CCCCAAACACTCCGACCTCA |
| INSP127-R2 | AGCACGAAGGCTGCCAACAC |
| INSP127-F3 | ATGCTGGGCACCGTGCTG |
| INSP127-R3 | CGGCGCGGGTTCCAGGGC |
| INSP127-F4 | TGCGCAGACCCCTCTTCAGC |
| INSP127-R4 | TGCACGGCCGGGGACACTCTT |
| INSP215-EX1 | ***CTCCCAGGGATCACCACC*TTACCCAGCGGG**CCACCTGCTCCCCCGTTC |
| INSP215-EX2 | **ATGCTGGGCACCGTGCTGCTGCTCGCCCTG*****CTCCCAGGGATCACCACC*** |
| INSP215-EX3 | GCAGGCTTCGCCACCATGCTGGGCACCGTGCTG |
| INSP215-EX4 | *TGATGGTGATGGTGCTGGGA*CCTCCTGGGCTG |
| INSP215 Mut FP | CGCCCTGAGCCTCCCGCGCACCATCACCATCACCAT |
| INSP215 Mut RP | ATGGTGATGGTGATGGTGCGCGGGAGGCTCAGGGCG |
| GCP Forward | GGGGACAAGTTTGTACAAAAAAGCAGGCTTCGCC ACC |
| GCP Reverse | GGGGACCACTTTGTACAAGAAAGCTGGGTTTCAATGGTGATGGTGATGGTG |
| pEAK12F | GCCAGCTTGGCACTTGATGT |
| pEAK12R | GATGGAGGTGGACGTGTCAG |
| 21M13 | TGTAAAACGACGGCCAGT |
| M13REV | CAGGAAACAGCTATGACC |
| T7 | TAATACGACTCACTATAGG |
| T3 | ATTAACCCTCACTAAAGG |

| | |
|---|---|
| Underlined sequence | = Kozak sequence |
| Highlighted sequence | = Stop codon |
| Italic sequence | = His tag |
| Bold sequence | = exon 1 sequence added by PCR |

The second PCR reaction (in a final volume of 50 µl) contains respectively: 5 µl of purified PCR1 product, 1.5 µl dNTPs (10 mM), 10 µl of 10X Pfx polymerase buffer, 1 µl MgSO4 (50 mM), 2X PCRₓ Enhancer solution (Invitrogen), 0.5 µl each of gene specific primer (100 µM) (INSP215-EX2 and INSP215-EX4), and 0.5 µl Platinum Pfx DNA polymerase (Invitrogen). The PCR reaction was performed using an initial denaturing step of 95 °C for 2 min, followed by 12 cycles of 94 °C for 15 sec; 55 °C for 30 sec and 68 °C for 2 min; and a holding cycle of 4 °C. The amplification product was directly purified using the Wizard SV Gel and PCR Clean-up System (Promega) and recovered in 50 µl sterile water according to the manufacturer's instructions.

The third PCR reaction (in a final volume of 50 µl) contains respectively: 5 µl of purified PCR1 product, 1.5 µl dNTPs (10 mM), 10 µl of 10X Pfx polymerase buffer, 1 µl MgSO4 (50 mM), 2X PCRₓ Enhancer solution (Invitrogen), 0.5 µl each of gene specific primer (100 µM) (INSP215-EX3 and INSP215-EX4), and 0.5 µl Platinum Pfx DNA polymerase (Invitrogen). The PCR reaction was performed using an initial denaturing step of 95 °C for 2 min, followed by 12 cycles of 94 °C for 15 sec; 55 °C for 30 sec and 68 °C for 2 min; and a holding cycle of 4 °C. The amplification product was directly purified using the Wizard SV Gel and PCR Clean-up System (Promega) and recovered in 50 µl sterile water according to the manufacturer's instructions.

The fourth PCR reaction (in a final volume of 50 µl) contained 10 µl of purified PCR2 product, 1.5 µl dNTPs (10 mM), 10 µl of 10X Pfx polymerase buffer, 1 µl MgSO₄ (50 mM), 2X PCRₓ Enhancer solution (Invitrogen), 0.5 µl of each Gateway conversion primer (100 µM) (GCP forward and GCP reverse) and 0.5 µl of Platinum Pfx DNA polymerase. The conditions for the 4th PCR reaction were: 95 °C for 1 min; 4 cycles of 94 °C, 15 sec; 50 °C, 30 sec and 68 °C for 2 min; 25 cycles of 94 °C, 15 sec; 55 °C, 30 sec and 68 °C, 2 min; followed by a holding cycle of 4 °C. A 10 µl aliquot was visualized on 0.8 % agarose gel in 1 X TAE buffer (Invitrogen) in order to verify that the product was of the expected molecular weight (876 + 70 = 946 bp). The remaining 40 µl were loaded on 0.8 % agarose gel in 1 X TAE buffer gel and the band was purified using the Wizard SV Gel and PCR Clean-up System (Promega) and recovered in 50 µl sterile water according to the manufacturer's instructions.

### 3.2 Subcloning of Gateway compatible INSP215 ORF into Gateway entry vector pDONR221 and expression vectors pEAK12d and pDEST12.2

The second stage of the Gateway cloning process involves subcloning of the Gateway modified PCR product into the Gateway entry vector pDONR221 (Invitrogen) as follows: 5 µl of purified product from PCR4 were incubated with 1.5 µl pDONR221 vector (0.1 µg/µl), 2 µl BP buffer and 1.5 µl of BP clonase enzyme mix (Invitrogen) in a final volume of 10 µl at RT for 1 h. The reaction was stopped by addition of proteinase K 1 µl (2 µg/µl) and incubated at 37 °C for a further 10 min. An aliquot of this reaction (1 µl) was used to transform *E*. *coli* DH10B cells by electroporation as follows: a 25 µl aliquot of DH10B electrocompetent cells (Invitrogen) was thawed on ice and 1 µl of the BP reaction mix was added. The mixture was transferred to a chilled 0.1 cm electroporation cuvette and the cells electroporated using a BioRad Gene-Pulser™ according to the manufacturer's recommended protocol. SOC media (0.5 ml) which had been pre-warmed to room temperature was added immediately after electroporation. The mixture was transferred to a 15 ml snap-cap tube and incubated, with shaking (220 rpm) for 1 h at 37 °C. Aliquots of the transformation mixture (10 µl and 50 µl) were then plated on L-broth (LB) plates containing kanamycin (40 µg/ml) and incubated overnight at 37 °C.

Plasmid miniprep DNA was prepared from 5 ml culture from 8 of the resultant colonies using a Qiaprep BioRobot 8000 system (Qiagen). Plasmid DNA (150-200 ng) was subjected to DNA sequencing with 21M13 and M13Rev primers as described above using the BigDyeTerminator system (Applied Biosystems cat. no. 4336919) according to the manufacturer's instructions. The primer sequences are shown in Table 3. Sequencing reactions were purified using Montage SEQ 96 cleanup plates (Millipore cat. no. LSKS09624) then analyzed on an Applied Biosystems 3700 sequencer.

Plasmid eluate (2 µl or approx. 150 ng) from one of the clones which contained the correct sequence (pENTR_INSP215-6HIS) was then used in a recombination reaction containing 1.5 µl of either pEAK12d vector or pDEST12.2 vector (0.1 µg / µl), 2 µl LR buffer and 1.5 µl of LR clonase (Invitrogen) in a final volume of 10 µl. The mixture was incubated at RT for 1 h, stopped by addition of proteinase K (2 µg) and incubated at 37 °C for a further 10 min. An aliquot of this reaction (1 µl) was used to transform *E*. *coli* DH10B cells by electroporation as follows: a 25 µl aliquot of DH10B electrocompetent cells (Invitrogen) was thawed on ice and 1 µl of the LR reaction mix was added. The mixture was transferred to a chilled 0.1 cm electroporation cuvette and the cells electroporated using a BioRad Gene-Pulser™ according to the manufacturer's recommended protocol. SOC media (0.5 ml) which had been pre-warmed to room temperature was added immediately after electroporation. The mixture was transferred to a 15 ml snap-cap tube and incubated, with shaking (220 rpm) for 1 h at 37 °C. Aliquots of the transformation mixture (10 µl and 50 µl) were then plated on L-broth (LB) plates containing ampicillin (100 µg/ml) and incubated overnight at 37 °C.

Plasmid mini-prep DNA was prepared from 5 ml cultures from 6 of the resultant colonies subcloned in each vector using a Qiaprep BioRobot 8000 system (Qiagen). Plasmid DNA (200-500 ng) in the pEAK12d vector was subjected to DNA sequencing with PEAK12F and pEAK12R primers as described above. Plasmid DNA (200-500 ng) in the pDEST12.2 vector was subjected to DNA sequencing with 21M13 and M13Rev primers as described above. Primer sequences are shown in Table 3.

CsCl gradient purified maxi-prep DNA was prepared from a 500 ml culture of the sequence verified clone (pEAK12d_INSP215-6HIS) using the method described by Sambrook J. et al., 1989 (in Molecular Cloning, a Laboratory Manual, 2nd edition, Cold Spring Harbor Laboratory Press), Plasmid DNA was resuspended at a concentration of 1 µg/µl in sterile water (or 10 mM Tris-HCl pH 8.5) and stored at -20 °C.

Endotoxin-free maxi-prep DNA was prepared from a 500 ml culture of the sequence verified clone (pDEST12.2_INSP215-6HIS) using the EndoFree Plasmid Mega kit (Qiagen) according to the manufacturer's instructions. Purified plasmid DNA was resuspended in endotoxin free TE buffer at a final concentration of at least 3 µg/µl and stored at -20 °C.

### 3.3 Generation of Gateway compatible INSP215 EC domain ORF fused to an in frame 6HIS tag sequence.

The sequence encoding the transmembrane and intracellular domains (amino acids 235-292) of the INSP215 cDNA insert in the plasmid pDONR221 were deleted using site directed mutagenesis using pDONR221_INSP215-6HIS as a template.

### 3.31 Gene specific cloning primers for site-directed mutagenesis of INSP215-6HIS ORF

The PCR primers, INSP215 Mut FP and INSP215 Mut RP (Table 3), were designed such that the primers annealed to opposite strands of the plasmid pDONR221_INSP215-6HIS sequence and each primer annealed to 15 - 25 bases on either side of the amino acid to be mutated. The PCR primers were designed following the instructions given in the Instruction manual for QuickChange^{®} II XL Site-Directed Mutagenesis Kit (Stratagene).

### 3.32 Site-direct mutagenesis of INSP215-6HIS ORF

Site-directed mutagenesis, was carried out using the QuikChange^{®} II Site-Directed Mutagenesis Kit (Stratagene) according to the manufacturer's instructions. The reaction was performed in a final volume of 50 µl containing 1X reaction buffer, 10 ng plasmid template DNA (pDONR221_INSP215-6HIS), 125 ng INSP215 Mut FP and INSP215 Mut RP, 1 µl dNTP mix, and 2.5 units *PfuUltra* HF DNA polymerase. Thermal cycling was performed using a MJ Research DNA Engine, programmed as follows: 95 °C, 1 min; 18 cycles of 95 °C, 30 sec, 55 °C, 1 min, and 68 °C, 3 min 30 sec; followed by a holding cycle at 4 °C.

*Dpn* I digestion was used to digest the methylated and hemimethylated parental DNA template (plasmid pDONR221_INSP215-6HIS in the sample reaction). 1 µl of *Dpn* I restriction enzyme (10 U/µl, Stratagene) was added to the products of the amplification reactions. The reactions were mixed gently and incubated at 37 °C for 1 hour. The reaction mixture was then transformed into XL1-Blue supercompetent cells (Stratagene) as follows. A 50 µl aliquot of XL1-Blue cells was thawed on ice and 1 µl *of Dpn* I-treated DNA was added. The mixture was incubated for 30 min on ice and then heat shocked by incubation at 42 °C for exactly 45 s. Samples were returned to ice for 2 min and 250 µl of pre-warmed (42 °C) NZY media was added. Samples were incubated with shaking (220 rpm) for 1 h at 37 °C. The transformation mixture (250 µl on each of 2 plates) was plated on L-broth (LB) plates containing kanamycin (50 µg/ml). Plates were incubated overnight at 37 °C.

### 3.33 Plasmid DNA preparation and sequencing

One transformant was selected and plasmid mini-prep DNA was prepared from 5 ml cultures using QIAprep Spin Miniprep kit (Qiagen). Plasmid DNA (150-200 ng) was subjected to DNA sequencing with 21M13 and M13Rev primers using the CEQ Dye Terminator Cycle sequencing Quick Start Kit (Beckman Coulter P/N 608120) according to the manufacturer's instructions. The primer sequences are shown in Table 3. Sequencing reactions were analyzed on CEQ 2000 XL DNA analysis system (Beckman Coulter P/N 608450). Sequence analysis identified a clone which contained the expected INSP215 insert sequence (pDONR221_INSP215-EC-6HIS).

### 3.4 Sub cloning of Gateway compatible INSP215-EC-6HIS ORF into expression vectors pEAK12d and pDEST12.2

Plasmid DNA (2 µl or approx. 150 ng) of pDONR221_INSP215-EC-6HIS was then used in a recombination reaction containing 1.5 µl of either pEAK12d vector or pDEST12.2 vector (0.1 µg / µl), 2 µl LR buffer and 1.5 µl of LR clonase (Invitrogen) in a final volume of 10 µl.

The reaction was stopped by addition of 1 µl proteinase K (2 µg/µl) and incubated at 37 °C for a further 10 min. An aliquot of this reaction (2 µl) was used to transform DH5α strain (Invitrogen) as follows: a 50 µl aliquot of DH5α cells was thawed on ice and 2 µl of reaction mixture added. The mixture was incubated for 30 min on ice and then heat shocked by incubation at 42 °C for exactly 45 s. Samples were returned to ice and 250 µl of warm SOC media (room temperature) was added. Samples were incubated with shaking (250 rpm) for 1 h at 37 °C. The transformation mixture was then plated on L-broth (LB) plates containing ampicillin (100 µg/ml) and incubated overnight at 37 °C.

Five transformants were picked and patched on LB agar plates containing ampicillin (100 µg/ml) and incubated overnight at 37 °C. A scoop of the grown culture from the patched plate was resuspended in 50 µl of water and boiled for 5 minutes to lyse the cells. The cell lysate was centrifuged to remove the cell debris and the supernatant obtained was used as a template for colony PCR screening.

The PCR mixture (in a final volume of 25 µl) contained 10 µl of the centrifuged cell lysate, 2.0 µl dNTPs (10 mM), 2.5 µl of Taq polymerase buffer, 0.5 µl of screening primers (to give a final concentration of 100 picomoles and 0.5 µl of Taq DNA polymerase. pEAK12d clones were screened using the primers pEAK12 FP and pEAK12 RP and pDEST12.2 clones were screened using the primers 21M13FP and M13Rev RP.

The conditions for the screening PCR reaction were: 95 °C for 2 min, followed by 30 cycles of 94 °C for 30 s; 60°C for 30 s and 72 °C for 1 min; and a final extension cycle of 72 °C for 5 minutes and a holding cycle of 4 °C. The PCR products were loaded onto a 1.0% agarose gel to verify the fragment size.

One positive clone was selected and plasmid mini-prep DNA was prepared from 5 ml cultures using QIAprep Spin Miniprep kit (Qiagen).

Plasmid DNA (150 - 200 ng) in the pEAK12d vector was subjected to DNA sequencing with the sequencing primers pEAK12 FP and pEAK12 RP as described above. Plasmid DNA (150 - 200 ng) in the pDEST12.2 vector was subjected to DNA sequencing with the sequencing primers 21M13 FP and M13Rev RP as described above.

Sequence confirmed clones were designated as pEAK12d-INSP215-EC-6HIS and pDEST12.2_INSP215-EC-6HIS.

### 3.5 DNA maxipreps

Maxi-prep DNA was prepared from a 500 ml culture of the sequence verified clones (pEAK12d_INSP215-EC-6HIS) using a Qiagen mega plasmid prep kit (cat no. 12183). Plasmid DNA was resuspended at a minimum concentration of 1 µg/µl in sterile water (or 10 mM Tris-HCl pH 8.5) and stored at -20 °C.

Endotoxin-free maxi-prep DNA was prepared from a 500 ml culture of the sequence verified clones (pDEST12.2_INSP215-EC-6HIS), using the EndoFree Plasmid Mega kit (Qiagen cat no. 12381) according to the manufacturer's instructions. Purified plasmid DNA was resuspended in endotoxin free TE buffer at a final concentration of at least 3 µg/µl and stored at -20 °C.

### Example 4: Cancer Assay

A soluble INSP215 is expressed either alone or as a fusion protein with Fc in HEK 293 cells. The protein is purified using either Protein A column or conventional chromatography and tested in a cell-based assay. The cell-based assay is developed on an existing animal model of breast cancer metastatic disease. Briefly, MDA-MB-231 cells are treated with TGFbeta1 (5 ng/ml) in the presence or absence of different amount of soluble INSP215 or INSP215-Fc for 48 hours. The conditioned media are collected and assayed for the amounts of IL-11 produced by these cells in response to TGF-beta. IL-11 ELISA kit is purchased from R&D Systems. TGFb-mediated production of IL-11 by metastatic breast adenocarcinoma MDA-MB-231 cells is measured. Anti-TGFb antibodies (R&D Systems) and OPG-Fc (Serono) are used as positive and negative controls, respectively.

### Example 5: Microarray studies

Custom microarrays have been manufactured using Agilent Technologies' (Agilent Technologies Inc, Palo Alto, CA) non-contact in situ synthesis process of printing 60-mer length oligonucleotide probes, base-by-base, from digital sequence files. This is achieved with an inkjet process which delivers extremely small, accurate volumes (picoliters) of the chemicals to be spotted. Standard phosphoramidite chemistry used in the reactions allows for very high coupling efficiencies to be maintained at each step in the synthesis of the full-length oligonucleotide. Precise quantities are reproducibly deposited "on the fly." This engineering feat is achieved without stopping to make contact with the slide surface and without introducing surface-contact feature anomalies, resulting in consistent spot uniformity and traceability (Hughes et al. (2001) Nat. Biotech. Apr; 19(4): 342-7. Expression profiling using microarrays fabricated by an ink-jet oligonucleotide synthesizer).

### Probe Synthesis

Methodologies were carried out according to Agilent instructions. Essentially, cDNA synthesis and subsequent T7 polymerase amplification of Cyanine 3(5)-CTP labeled cRNA probe was carried out using Agilent's low RNA input fluorescent linear amplification kit from a template of 5µg of total RNA according to the kit protocol (version 2 August 2003, Agilent, Palo Alto, CA). cRNA is then fragmented using Agilent's *In Situ* hybridization kit-plus and hybridized both according to Agilent's protocol (Agilent 60-mer oligo microarray processing protocol version 4.1 April 2004, Agilent, Palo Alto, CA).

### Microarray Chip Design

- 10,536 probes are on the array
- 5557 of the probes designed specifically to detect secreted sequences of primary interest
- 1000 probes designed as negative controls
- 500 probes designed as positive controls

Remainder of the probes were designed to public domain sequences which are known to be either secreted soluble extracellular proteins or membrane bound proteins with an extracellular domain in contact with the extracellular milieu.

### Example 6: Further studies

Functionality and use of the polypeptides of the invention may be further assessed as set out below:

This may involve the preparation of human cDNA templates, for example, from a variety of normal human tissue total RNA samples (which can be purchased from Clontech, Stratagene, Ambion, Biochain Institute and prepared in-house) using an enzyme such as Superscript II RNase H- Reverse Transcriptase (Invitrogen). Human cDNA libraries (in bacteriophage lambda (λ) vectors) can be purchased from Clontech, Invitrogen, or made in-house in λ GT10 vectors. Pairs of specific PCR primers can be designed for amplifying the complete coding sequence of the virtual cDNA using software such as the Primer Designer Software (Scientific & Educational Software, PO Box 72045, Durham, NC 27722-2045, USA). PCR primers were optimized to have a Tm close to 55 + 10 0C and a GC content of 40-60%. Primers should be selected which have high selectivity for the target sequence (INSP215) with little or no non-specific priming. PCR can then be used to amplify the gene sequence of interest. Procedures necessary for cloning of the sequence, such as sub-cloning of PCR products, colony PCR, plasmid DNA preparation and sequencing and finally construction of mammalian cell expression vectors are known in the art (see, for example, WO03/055913).

Further experiments may then be performed to determine the tissue distribution and expression levels of the INSP215 polypeptides *in vivo,* on the basis of the nucleotide and amino acid sequences disclosed herein.

For example, the presence of the transcripts for INSP215 may be investigated by PCR of cDNA from different human tissues. The INSP215 transcripts may be present at very low levels in the samples tested. Therefore, extreme care is needed in the design of experiments to establish the presence of a transcript in various human tissues as a small amount of genomic contamination in the RNA preparation will provide a false positive result. Thus, all RNA should be treated with DNAse prior to use for reverse transcription. In addition, for each tissue a control reaction may be set up in which reverse transcription was not undertaken (a -ve RT control).

For example, 1µg of total RNA from each tissue may be used to generate cDNA using Multiscript reverse transcriptase (ABI) and random hexamer primers. For each tissue, a control reaction is set up in which all the constituents are added except the reverse transcriptase (-ve RT control). PCR reactions are set up for each tissue on the reverse transcribed RNA samples and the minus RT controls. INSP215-specific primers may readily be designed on the basis of the sequence information provided herein. The presence of a product of the correct molecular weight in the reverse transcribed sample together with the absence of a product in the minus RT control may be taken as evidence for the presence of a transcript in that tissue. Any suitable cDNA libraries may be used to screen for the INSP215 transcripts, not only those generated as described above.

The tissue distribution pattern of the INSP215 polypeptides will provide further useful information in relation to the function of those polypeptides.

Furthermore, overexpression or knock-down of the expression of the polypeptides in cell lines may be used to determine the effect on transcriptional activation of the host cell genome. Dimerisation partners, co-activators and co-repressors of the INSP215 polypeptide may be identified by immunoprecipitation combined with Western blotting and immunoprecipitation combined with mass spectroscopy.

## Claims

1. A polypeptide comprising the amino acid sequence as recited in SEQ ID NO:8.

2. A polypeptide according to claim 1 which consists of the amino acid sequence as recited in SEQ ID NO:2, SEQ ID NO:4, SEQ ID NO:6, SEQ ID NO:8, SEQ ID NO:19 or SEQ ID NO:21.

3. A polypeptide comprising SEQ ID NO:10.

4. A polypeptide of claim 3 which consists of the amino acid sequence as recited in SEQ ID NO:10, SEQ ID NO:12, SEQ ID NO:14, SEQ ID NO:19 or SEQ ID NO:21.

5. A polypeptide which is a fragment or a functional equivalent as recited in any one of the preceding claims, which has greater than 80% sequence identity with the amino acid sequence recited in SEQ ID NO:8, preferably greater than 85%, 90%, 95%, 98% or 99% sequence identity, and contains a ZP/TGR3 subdomain as found in any of the amino acid sequences recited in SEQ ID NO:10, SEQ ID NO:12 or SEQ ID NO:14.

6. A fusion protein comprising the polypeptide according to any one of the preceding claims.

7. The polypeptide of claim 6, wherein said polypeptide comprises a histidine tag.

8. The polypeptide of claim 7, whose sequence is recited in SEQ ID NO:19 or SEQ ID NO:21.

9. The polypeptide of any one of the preceding claims, wherein said polypeptide comprises a signal peptide.

10. The polypeptide of claim 9, whose sequence is recited in SEQ ID NO:2, SEQ ID NO:6, SEQ ID NO:12, SEQ ID NO:19 or SEQ ID NO:21.

11. A vector comprising a nucleic acid molecule which encodes a polypeptide according to any one claims 1 to 4 or 6 to 10.

12. A vector according to claim 11, which comprises the nucleic acid sequence as recited in SEQ ID NO:1, SEQ ID NO:3, SEQ ID NO:5, SEQ ID NO:7, SEQ ID NO:11, SEQ ID NO: 13, SEQ ID NO:16, SEQ ID NO:17, SEQ ID NO:18 or SEQ ID NO:20.

13. A vector according to claim 12, wherein the nucleic acid molecule consists of the nucleic acid sequence as recited in SEQ ID NO: 1, SEQ ID NO: 3, SEQ ID NO: 5, SEQ ID NO:7, SEQ ID NO:9, SEQ ID NO:11, SEQ ID NO: 13, SEQ ID NO:16, SEQ ID NO:17, SEQ ID NO:18 or SEQ ID NO:20.

14. A host cell transformed with a vector according to any one of claims 11 to 13.

15. An antibody which binds specifically to the ZP/TGR3 subdomain as found in any one of the amino acid sequence recited in SEQ ID NO:10, SEQ ID NO:12 or SEQ ID NO:14 of the polypeptide according to any one of claims 1 to 10.

16. A polypeptide according to any one of claims 1 to 10, a vector according to any one of claims 11 to 13, a host cell according to claim 14, an antibody according to claim 15, for use in therapy or diagnosis of disease.

17. A polypeptide according to any one of claims 1 to 10 for use in binding a protein belonging to the TGF-beta protein superfamily.

## Patentansprüche

1. Polypeptid umfassend die Aminosäuresequenz, wie sie in SEQ ID NR:8 gezeigt ist.

2. Polypeptid nach Anspruch 1, das aus der Aminosäuresequenz besteht, die in SEQ ID NR:2, SEQ ID NR:4, SEQ ID NR:6, SEQ ID NR:8, SEQ ID NR:19 oder SEQ ID NR:21 gezeigt ist.

3. Polypeptid umfassend SEQ ID NR:10.

4. Polypeptid nach Anspruch 3, das aus der Aminosäuresequenz besteht, die in SEQ ID NR:10, SEQ ID NR:12, SEQ ID NR:14, SEQ ID NR:19 oder SEQ ID NR:21 gezeigt ist.

5. Polypeptid, das ein Fragment oder ein funktionelles Äquivalent nach einem der vorstehenden Ansprüche ist, welches Sequenzidentität mit der Aminosäuresequenz der SEQ ID NR:8 aufweist, die größer als 80% ist, vorzugsweise größer als 85%, 90%, 95%, 98% oder 99% Sequenzidentität, und eine ZP/TGR3 Subdomäne enthält, wie sie in einer der Aminosäuresequenzen, die in SEQ ID NR:10, SEQ ID NR:12 oder SEQ ID NR:14 gezeigt ist, gefunden wird.

6. Fusionsprotein umfassend das Polypeptid nach einem der vorstehenden Ansprüche.

7. Polypeptid nach Anspruch 6, wobei das Polypeptid eine Histidinmarkierung umfasst.

8. Polypeptid nach Anspruch 7, dessen Sequenz in SEQ ID NR:19 oder SEQ ID NR:21 gezeigt ist.

9. Polypeptid nach einem der vorstehenden Ansprüche, wobei das Polypeptid ein Signal-peptid umfasst.

10. Polypeptid nach Anspruch 9, dessen Sequenz in SEQ ID NR:2, SEQ ID NR:6, SEQ ID NR:12, SEQ ID NR:19 oder SEQ ID NR:21 gezeigt ist.

11. Vektor umfassend ein Nukleinsäuremolekül, welches ein Polypeptid nach einem der Ansprüche 1 bis 4 oder 6 bis 10 kodiert.

12. Vektor nach Anspruch 11, der die Nukleinsäuresequenz umfasst, die in SEQ ID NR:1, SEQ ID NR:3, SEQ ID NR:5, SEQ ID NR:7, SEQ ID NR:11, SEQ ID NR:13, SEQ ID NR:16, SEQ ID NR:17, SEQ ID NR:18 oder SEQ ID NR:20 gezeigt ist.

13. Vektor nach Anspruch 12, wobei das Nukleinsäuremolekül aus der Nukleinsäuresequenz besteht, die in SEQ ID NR:1, SEQ ID NR:3, SEQ ID NR:5, SEQ ID NR:7, SEQ ID NR:9, SEQ ID NR:11, SEQ ID NR:13, SEQ ID NR:16, SEQ ID NR:17, SEQ ID NR:18 oder SEQ ID NR:20 gezeigt ist.

14. Wirtszelle transformiert mit dem Vektor nach einem der Ansprüche 11 bis 13.

15. Antikörper, der an die ZP/TGR3 Subdomäne spezifisch bindet, wie sie in einer der Aminosäuresequenzen gefunden wird, die in SEQ ID NR:10, SEQ ID NR:12 oder SEQ ID NR:14 des Polypeptids nach einem der Ansprüche 1 bis 10 gezeigt ist.

16. Polypeptid nach einem der Ansprüche 1 bis 10, Vektor nach einem der Ansprüche 11 bis 13, Wirtszelle nach Anspruch 14 oder Antikörper nach Anspruch 15 zur Verwendung in der Therapie oder Diagnose einer Erkrankung.

17. Polypeptid nach einem der Ansprüche 1 bis 10, zur Verwendung bei der Bindung eines Proteins, das zur TGF-Beta-Protein-Superfamilie gehört.

## Revendications

1. Polypeptide comprenant la séquence d'acides aminés donnée en tant que Séquence N° 8.

2. Polypeptide conforme à la revendication 1, constitué de la séquence d'acides aminés donnée en tant que Séquence N° 2, Séquence N° 4, Séquence N° 6, Séquence N° 8, Séquence N° 19 ou Séquence N° 21.

3. Polypeptide comprenant la Séquence N° 10.

4. Polypeptide conforme à la revendication 3, constitué de la séquence d'acides aminés donnée en tant que Séquence N° 10, Séquence N° 12, Séquence N° 14, Séquence N° 19 ou Séquence N° 21.

5. Polypeptide qui est un fragment ou un équivalent fonctionnel d'un polypeptide conforme à l'une des revendications précédentes, dont la séquence est identique à plus de 80%, et de préférence à plus de 85%, 90%, 95%, 98% ou 99%, à la séquence d'acides aminés donnée en tant que Séquence N° 8, et qui contient un sous-domaine ZP/TGR3, tel qu'on le trouve dans n'importe laquelle des séquences d'acides aminés données en tant que Séquence N° 10, Séquence N° 12 et Séquence N° 14.

6. Protéine de fusion, comprenant un polypeptide conforme à l'une des revendications précédentes.

7. Polypeptide conforme à la revendication 6, lequel polypeptide comprend une étiquette histidine.

8. Polypeptide conforme à la revendication 7, dont la séquence est donnée en tant que Séquence N° 19 ou Séquence N° 21.

9. Polypeptide conforme à l'une des revendications précédentes, lequel polypeptide comprend un peptide signal.

10. Polypeptide conforme à la revendication 9, dont la séquence est donnée en tant que Séquence N° 2, Séquence N° 6, Séquence N° 12, Séquence N° 19 ou Séquence N° 21.

11. Vecteur comprenant une molécule d'acide nucléique qui code un polypeptide conforme à l'une des revendications 1 à 4 ou 6 à 10.

12. Vecteur conforme à la revendication 11, qui comprend la séquence d'acide nucléique donnée en tant que Séquence N° 1, Séquence N° 3, Séquence N° 5, Séquence N° 7, Séquence N° 11, Séquence N° 13, Séquence N° 16, Séquence N° 17, Séquence N° 18 ou Séquence N° 20.

13. Vecteur conforme à la revendication 12, dans lequel la molécule d'acide nucléique est constituée de la séquence d'acide nucléique donnée en tant que Séquence N° 1, Séquence N° 3, Séquence N° 5, Séquence N° 7, Séquence N° 9, Séquence N° 11, Séquence N° 13, Séquence N° 16, Séquence N° 17, Séquence N° 18 ou Séquence N° 20.

14. Cellule hôte transformée avec un vecteur conforme à l'une des revendications 11 à 13.

15. Anticorps qui se lie spécifiquement au sous-domaine ZP/TGR3, tel qu'on le trouve dans n'importe laquelle des séquences d'acides aminés données en tant que Séquence N° 10, Séquence N° 12 et Séquence N° 14, d'un polypeptide conforme à l'une des revendications 1 à 10.

16. Polypeptide conforme à l'une des revendications 1 à 10, vecteur conforme à l'une des revendications 11 à 13, cellule hôte conforme à la revendication 14, ou anticorps conforme à la revendication 15, pour leur utilisation dans la thérapie ou le diagnostic d'une maladie.

17. Polypeptide conforme à l'une des revendications 1 à 10, pour utilisation dans la fixation d'une protéine appartenant à la superfamille de protéines TGF-β.
